# EUROPEAN PATENT APPLICATION

(11) **EP 4 435 117 A1**
(43) Date of publication of application: **25.09.2024**
(21) Application number: 23382282.4
(22) Date of filing: 24.03.2023
(51) Int. Cl.: C12Q 1/6855, C12Q 1/686, C12N 9/12, C12N 15/10

(54) **NOVEL METHOD FOR ISOTHERMAL AMPLIFICATION OF PADLOCK PROBES FOR NUCLEIC ACID DETECTION AND PHI29 DNA POLYMERASE VARIANTS**

(71) Applicant: Consejo Superior de Investigaciones Cientificas, 28006 Madrid (ES)
(72) Inventor: BLANCO DÁVILA, Luis, 28049 Madrid (ES); DE VEGA JOSÉ, Miguel, 28049 Madrid (ES); MARTÍNEZ JIMÉNEZ, María Isabel, 28049 Madrid (ES); DEL PRADO DÍAZ, Alicia, 28049 Madrid (ES)
(74) Representative: Hoffmann Eitle

(57) **Abstract**

The present invention relates to a method of primer-less isothermal amplification for detecting a target nucleic acids. In this method (a cartoon is shown in Figure 14), a specific DNA padlock probe, complementary to the 3' terminal region of a target nucleic acid, is circularized and amplified by rolling circle amplification (RCA), where the 3'-end of the target nucleic acid can act as an efficient primer by an optimized phi29 DNApol variant. The padlock-directed concatemeric ssDNA produced by RCA is further amplified by the combination of a modified phi29 DNApol (Qx3, Qx5) and a specific DNA primase (*Tth*PrimPol) which continuously synthesizes suitable DNA primers on the RCA product to trigger a hyperbranched rolling circle amplification (HRCA), that can be directly analyzed by any detection method. Variants of phi29 DNApol and their use in this and other amplification methods are also provided herein.

## Description

### TECHNICAL FIELD

The present invention relates to the field of molecular biology. Particularly, the present invention relates to the field of amplification and detection methods using Hyperbranched Rolling Circle Amplification.

### BACKGROUND ART

Rolling circle amplification (RCA) is an enzymatic process where a short DNA or RNA primer initiates the synthesis of a long concatemeric single-stranded DNA using a circular single-stranded DNA template and DNA polymerases with strand-displacement ability, such as bacteriophage phi29 DNApol (phi29 DNApol; Blanco et al. JBC 1989). A variant of this method is used for detection of DNA or RNA target molecules, where specially designed single-stranded DNA padlock probes hybridize to the target nucleic acid, whereby the two ends of the probe become juxtaposed and are joined by a ligase, forming the circular single-stranded nucleic acid that will be the DNA template for RCA. The addition of a strand-displacement polymerase as phi29 DNApol and nucleotides starts the polymerisation, that can be carried out under isothermal conditions. As the circular single-stranded DNA template (the ligated padlock) is endless, the resultant product of the RCA is a long single-stranded DNA molecule composed of tandem repeats, or monomers, that are complementary to said padlock template (i.e., a concatemer).

The RCA reaction thus produces a linear amplification of DNA, as each circular template grows at a given speed for a certain amount of time. To increase yield and achieve exponential amplification, several approaches have been investigated. One of them is the Hyperbranched Rolling Circle Amplification (HRCA), where synthetic DNA primers that anneal to the RCA products are added, and also extended in a further amplification step of hyperbranched DNA synthesis, which can also be carried out under isothermal conditions due to the strand-displacement ability of phi29 DNApol. In this way the ongoing RCA linearly creates more template that can be exponentially amplified and subsequently detected. Methods to detect nucleic acid are widely known, and include the use of DNA dyes, beacons probes, electrochemical assays and Crispr/Cas based methods.

Some of the polymerases used in RCA and HRCA methods are phi29 DNApol, the large fragment of *Bacillus stearothermophilus* DNA polymerase I (Bst), or the exo-deficient *Thermococcus litorales* DNA polymerase I (Vent) for DNA amplification, and T7 RNA polymerase for RNA amplification. More recently, an upgraded version of phi29 DNApol (Qualiphi) has been described (de Vega et al. PNAS 2010), which improves amplification of very limiting amounts of input DNA. Since phi29 DNApol has the best fidelity, processivity and strand-displacement ability among all aforementioned polymerases, it has been most frequently used in RCA and HRCA reactions.

In almost all DNA replication methods, the initiation of replication requires a primer which provides a free hydroxyl group to be the acceptor of the nucleotides added by DNA polymerases. For this task, specific or randomized DNA primers are usually provided in *in vitro* amplification methods. However, a different solution was found in all cellular life forms, as well as many viruses and plasmids, which is a type of enzyme called PrimPol, as it has both primase and polymerase activities. Said PrimPol enzyme can initiate DNA replication thanks to its unique primase activity, able to start synthesis *de novo* of DNA primers, and elongate them to some extent with its polymerase activity, to create newly generated mature DNA primers. A particular interesting PrimPol enzyme is the primase/polymerase of *Thermus thermophilus* (also called herein *Tth*PrimPol), which has been reported to have unique features for its cooperation with phi29 DNApol (see Picher, Á., et al. TruePrime is a novel method for whole-genome DNA amplification from single cells based on TthPrimPol. Nat Commun 7, 13296 (2016). https://doi.org/10.1038/ncomms13296).

Since its first introduction, HRCA has been proven to be a robust DNA amplification technique for detecting pathogenic fungi and other applications, allowing rapid detection of nucleic-acid sequences with high specificity. This method has been adopted as the basis for a laboratory method for amplifying and detecting linear and circular nucleic acid molecules.

For the detection/amplification or ribonucleic acids by HRCA methods, and also by those based on thermal cycling, a previous step of reverse transcription is required to convert the RNA into a DNA template that can be amplified by DNA polymerases. That first step requires a specialized DNA polymerase, called reverse transcriptase. In the recent COVID-19 pandemics, the gold standard method to detect/amplify the SARS-CoV2 RNA viral genome is RT-PCR, a sensitive, but technically complex amplification procedure requiring reverse transcription and thermal cycling.

Despite the growth of research on point-of-care (POC) tests, most devices never leave the laboratory. This is especially relevant in the case of diagnostic tests, as the COVID-19 pandemic has shown us the importance of developing rapid testing and diagnosis for efficient epidemic control. Hence, it is desired that methods such as RCA and HRCA are modified and optimized to be moved from the bench to the field in low-cost, point of care diagnostics. However, this represents a challenge as several hurdles need to be overcome. On the one hand, the polymerases are most efficient at a usually narrow temperature range that may not be feasible in a POC setting. Further, the use of several reagents, such as primers, dNTPs, and buffers, and the requisite of a previous reverse transcription step add extra complexity to the method. Lastly, reducing the number of steps, or even integrating several steps (such as nucleic acid extraction, nucleic acid amplification, and detection methods) into a one-step approach, is also not easy to achieve nor straight forward. On the other hand, while the method should be adapted to be rapid, portable, simple, and low cost, this adaptation should not be detrimental on the specificity and accuracy of the method.

The present invention aims at overcoming the above hurdles and provides a new method for the rapid and easy (isothermal conditions), low cost, but specific and efficient, detection of target ribonucleic acids without the need of reverse transcription.

### SUMMARY OF THE INVENTION

In one aspect, the present invention provides a method for detecting the presence of a single-stranded target nucleic acid by hyperbranched rolling circle amplification (HRCA), the method comprising the steps of:
a) contacting the target nucleic acid with at least one padlock probe, wherein the at least one padlock probe comprises a 5' target-specific hybridization region that is complementary to a first region of the target nucleci acid and a 3' target-specific hybridization region that is complementary to a second region of the target nucleic acid, wherein the first and the second regions are located adjacent to each other and located in the 3' region of the target nucleic acid,
b) circularising the padlock probe whose 5' and 3' end sequences have hybridized to the target nucleic acid by ligating the ends of said padlock probe,
c) subjecting the circularized padlock probe to rolling circle amplification (RCA), wherein the RCA is carried out by a bacteriophage phi29 DNA polymerase (phi29 DNApol-type enzyme), wherein the 3' end of the target nucleic acid is used as a primer for the RCA,
d) during and/or after step c) amplifying the product of c) using the DNA primers made by the primase/polymerase of *Thermus thermophilus* (TthPrimPol), and
e) detecting the amplified product.

Preferably, the phi29 DNApol-type enzyme comprises an amino acid sequence that is at least 70% identical to SEQ ID NO: 1, wherein the DNA polymerase comprises 3'-5' exonuclease and processive strand-displacement DNA synthesis. Also preferably, the phi29 DNApol-type enzyme comprises at least the amino acid substitutions E415X, E416X and E417X, as defined using the amino acid numbering of SEQ ID NO: 1, wherein X is a positively charged amino acid, preferably lysine (K). Preferably, the phi29 DNApol-type further comprises the amino acid substitutions E218M and V267L, as defined using the amino acid numbering of SEQ ID NO: 1.

Preferably, the primase/polymerase of *Thermus thermophilus* (T*th*PrimPol) is at least 70% identical to SEQ ID NO: 2.

Preferably, the at least one padlock probe is characterized by not having in its nucleotide sequence the priming sites recognised by the primase/polymerase protein of *Thermus thermophilus* (T*th*PrimPol), and/or the at least one padlock probe is characterized by having the complementary sequence of said priming sites.

Preferably, the thermostable primase/polymerase of *Thermus thermophilus* (T*th*PrimPol) is at least 90% identical to SEQ ID NO: 2, and the at least one padlock probe is characterized by:
i) not having in its nucleotide sequence, preferably in its backbone sequence, the sequences "5'-CCTC-3"' and/or "5'-CTC-3"', and/or
ii) having in its nucleotide sequence, preferably in its backbone sequence, one or more sequences "5'-GAGG-3"' and/or "5'-GAG-3"'.

Preferably, a specific DNA primer for the strand-displaced complementary copy of the RCP is added.

Preferably, the padlock comprises SEQ ID NO: 7-14, or a sequence with at least 90% sequence identity to SEQ ID Nos: 7-14.

Preferably, the ligation in step b) is performed with the DNA Ligase from *Paramecium bursaria* chlorella virus-1.

Preferably the single-stranded target nucleic acid is the genome of an infectious agent, preferably the genome of a virus.

Preferably, the single-stranded target is a ribonucleic acid. Preferably the single-stranded target ribonucleic acid comprises a polyA tail, and the first and second regions of the target ribonucleic acid are not located in said the polyA tail.

Preferably the single-stranded target nucleic acid is comprised in a biological sample, preferably saliva.

Preferably, the method is performed in a one-pot reaction.

Preferably, the method is performed at alkaline pH, preferably at pH ranging from 8.5-9.

Preferably, the method is performed isothermally in the 30-37°C range.

In a further aspect, the present invention provides a padlock probe comprising SEQ ID NO: 7-14, or a sequence with at least 90% sequence identity to SEQ ID NOs: 7-14.

In a further aspect, the present invention provides a baccteriophage phi29 DNA polymerase comprising an amino acid sequence that is at least 90% identical to SEQ ID NO: 1, wherein the bacteriophage phi29 DNA polymerase comprises at least the amino acid substitutions E415K, E416K and E417K, as defined using the amino acid numbering of SEQ ID NO: 1. Preferably, the bacteriophage phi29 DNA polymerase further comprises the amino acid substitutions E218M and V267L, as defined using the amino acid numbering of SEQ ID NO: 1.

### BRIEF DESCRIPTION OF DRAWINGS

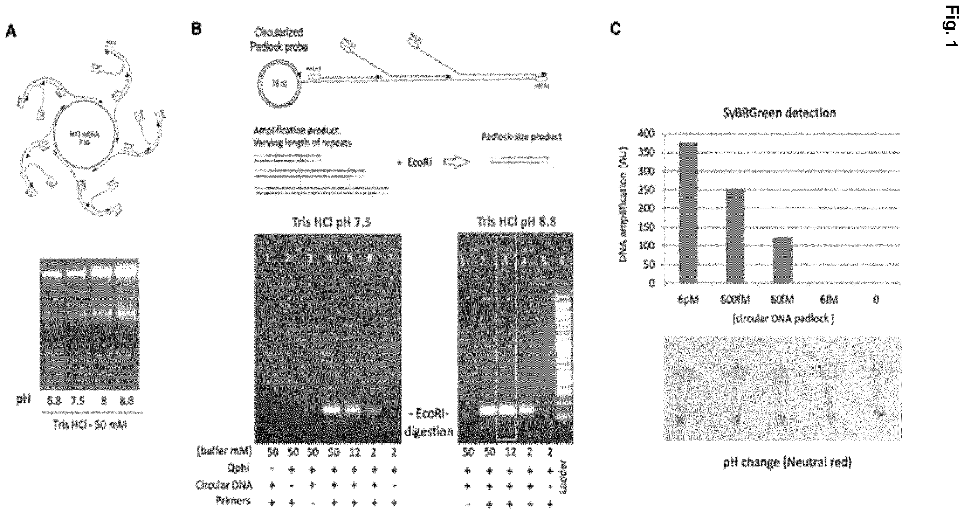

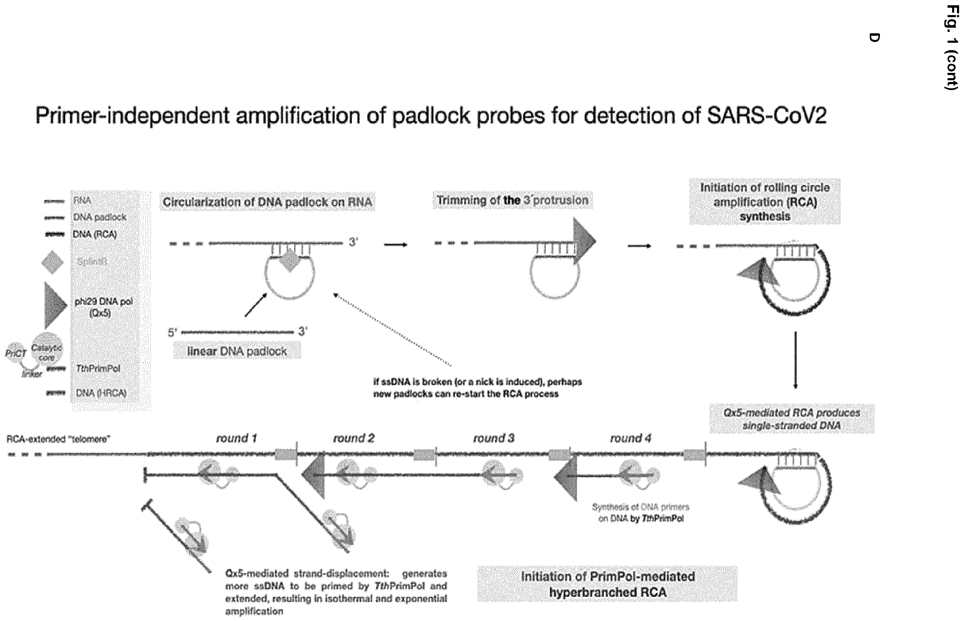

**Fig. 1****: Starting alkaline conditions favour amplification detection by Qualiphi. A)** Hyperbranched rolling circle amplification (HRCA) of M13mp18 ssDNA (2 nM) by Qualiphi (75 nM) using random hexamers (10 µM). Reaction conditions (20 µL) were: 50 mM Tris-HCl at the indicated pH (6.8 to 8.8), 10 mM MgCl₂, 50 mM KCI, 45 mM (NH₄)₂SO₄, 0.025% Tween-20, 1.5 % (vol/vol) glycerol and 0.5 mM dNTPs. Reactions were incubated at 30 °C during 1 h. The gel shows that an increase in the pH up to 8.8 sustains M13 amplification by Qualiphi, and even improves the yield of amplified DNA. B) HRCA on a gene (actine)-specific padlock probe by Qualiphi. The incubation mixture contained, in a total volume of 20 µL, the indicated concentration of Tris-HCl at the specified pH (7.5 or 8.8), 10 mM MgCl₂, 50 mM KCI, 45 mM (NH₄)₂SO₄, 0.025% Tween20, 1.5 % (vol/vol) glycerol, 0.5 mM dNTPs, 1 µM oligos (HRCA1: 5' TGTAAAACGACGGCC 3' and oligo HRCA2: 5' CAGGAAACAGCTATG 3'), 6.5 nM of a of 76 nt long circular ssDNA and 75 nM Qualiphi. To obtain a small circular ssDNA, 25 µM of a gene(actine)-specific linear padlock (5'-**TGCGGTGGACGATGG**AAAAATGTAAAACGACGGCCGAATTCATAGCTGTTTCCTG AAAAA**CCGCCTAGAAGCATT**-3'; in bold, regions that hybridize to the target) was circularized by hybridization with 25 µM of its corresponding target (actine) DNA (5'-TCCGGCCCCT**CCATCGTCCACCGCAAATGCTTCTAGGCGG**ACTATGACTT-3'; in bold, region that hybridize to the padlock) in the presence of 50 mM Tris-HCl pH 7.5, 10 mM MgCl₂, 1 mM ATP and 10 mM DTT during 10 min at 90 °C and further cooled to room temperature. After that 400 U of ligase T4 (New England Biolabs, Ipswich, USA) was added during 2 h at room temperature. Amplification reactions were incubated at 30 °C during 2 h and inactivated during 10 min at 65 °C. Then, 2 µL of the amplification reaction were digested with EcoRI (40 U) during 2 h at 37 °C and loaded in a 0.8 % agarose gel containing ethidium bromide (0.5 µg/mL). **C)** Quantification, measured by SYBRGreen, of the amplified DNA produced using decreasing concentrations of the circularized padlock used as input DNA template for HRCA (6 pM, 600 fM, 60 fM, 6 fM and 0). Reaction conditions were as follow: 75 nM Qualiphi, 12 mM Tris HCl pH 8.8, 10 mM MgCl₂, 50 mM KCI, 45 mM (NH₄)₂SO₄,, 0.025% Tween-20, 1.5 % (vol/vol) glycerol, 0.5 mM dNTPs, 1 µM HRCA1 and HRCA2 oligos, and 100 µM Neutral Red. Reactions were incubated at 30 °C during 2 h and inactivated by heating 10 min at 65 °C. DNA amplification was also evidenced by a pH-dependent color change using Neutral Red.

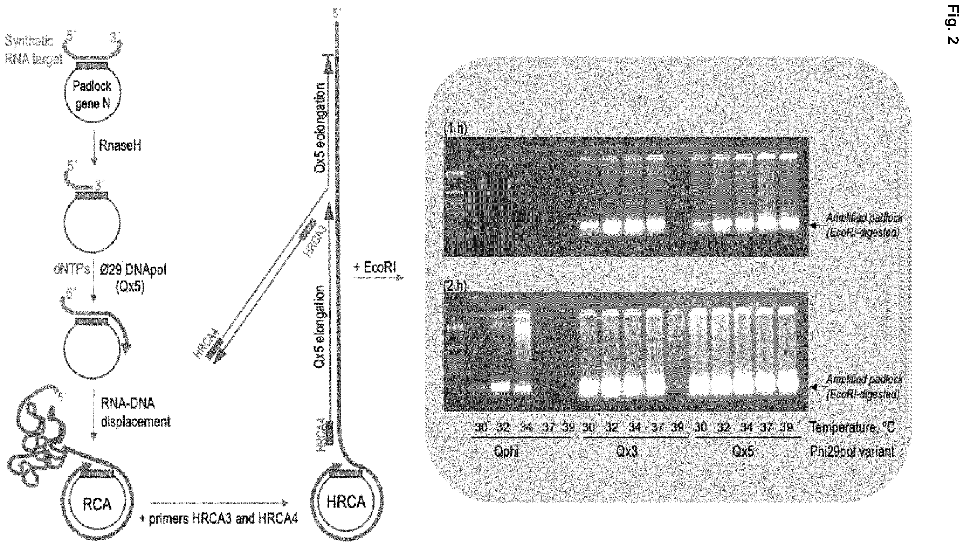

**Fig. 2****: HRCA by thermo-stabilized versions (Qx3 and Qx5) is more efficient than that by Qualiphi, even at a low temperature.** The incubation mixture contained in a final volume of 20 µL, 4.5 mM Tris-HCl pH 8.3, 40 mM NaCl, 45 mM (NH₄)₂SO₄, 7.5 % (vol/vol) glycerol, 10 µM EDTA, 0.5 mM ATP, 5 mM DTT, 5 mM MgCl₂, 6 U SplintR ligase (New England Biolabs, Ipswich, USA), 2.5 U RNaseH, 50 pM of the SARS-CoV2 (gene N)-specific padlock (5' **TTCTTGAACTGTTGC**GAATTCGGTAAAACGACCATAGCTGTTGTAAAACGACCATA GCTGTTGTAAAACGACCATAGCTGTTGTAAAACGACCTCAGCCGACCATAGCTGT TGTAAAACGACCATAGCTGTTGTAAAACGACCATAGCTGTTCGATATC**TGCCTGGA GTTG*A*A*T**; in bold, the regions that hybridize to the target), 5 pM of a synthetic RNA target (geneN) oligonucleotide (5'-*ACGUAGUC**GCAACAGUUCAAGAAAUUCAACUCCAGGCA**AGCAGUAG,* in bold, the region that hybridizes to the padlock), 0.25 µM HRCA3 (5'-GTAAAAC*G*A*C) and HRCA4 (5'-AACAGCT*A*T*G) oligos, 2.5 % thermo-inactivated non-infected saliva (saliva was mixed with TE 1:1, then incubated 95 °C during 30 min), 320 nM of the indicated Phi29 DNApol variant (Qphi, Qx3 or Qx5), and 0.5 mM dNTPs. After incubation for 1 or 2 h at the indicated temperature (30, 32, 34, 37, 39°C) the samples were inactivated by heating 10 min at 65 °C. Afterwards, 1 µL of the sample was digested with EcoRI 40 U (2 h at 37 °C) and the reaction products were analyzed by electrophoresis in 0.8% agarose gels containing ethidium bromide (0.5 µg/mL). Asterisks in the sequences indicate degradation resistent phosphorothioate bond linking the last four 3' nucleotides.

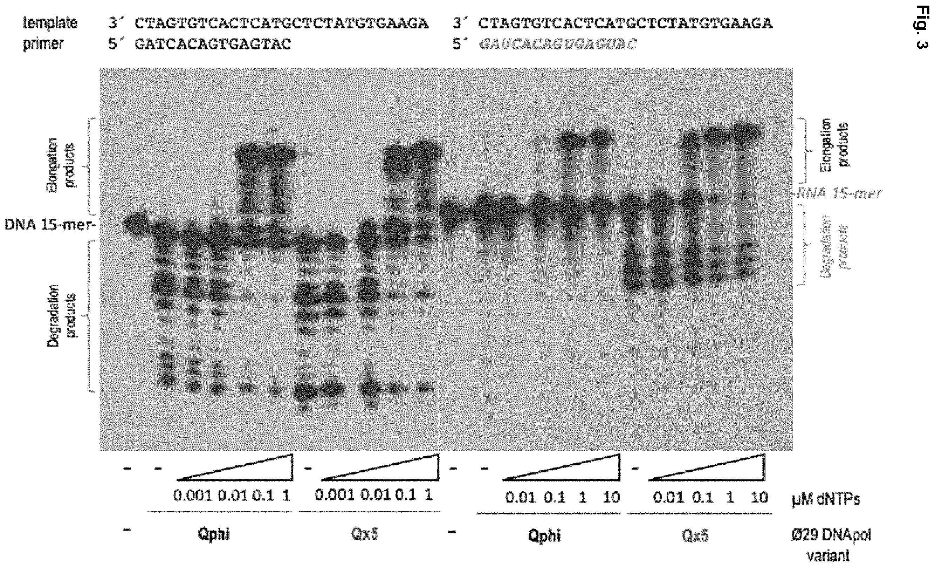

**Fig. 3****: The thermo-stabilized version of Qualiphi (Qx5) shows a more profficient exonucleolysis on RNA primers, and an optimal RNA primer extension when** dNTPs **are provided.** The functional coupling between DNA synthesis and DNA versus RNA degradation was assayed using as substrates the 28/15mer template/primer duplexes formed by 5'AGAAGTGTATCTCGTACTCACTGTGATC hybridized to 5'-labeled 2'-deoxyoligonucleotide 5' GATCACAGTGAGTAC (left panel), or hybridized to the 5'-labeled oligoribonucleotide 5' *GAUCACAGUGAGUAC* (right panel). The incubation mixture contained, in a final volume of 12.5 µL, 50 mM Tris-HCl pH 7.5, 1 mM DTT, 4 % (vol/vol) glycerol, 0.1 mg/mL BSA, 10 mM MgCl₂, 50 nM of the Phi29 DNApol variants Qphi or Qx5, 4 nM of the indicated substrate, and the specified concentration of dNTPs (0-10 µM). After incubation for 5 min at 30 °C, reactions were stopped by adding EDTA up to a final concentration of 10 mM and analyzed by 7 M urea/20% polyacrylamide gel electrophoresis and autoradiography. Polymerization or 3'-5' exonucleolysis is detected as an increase or decrease, respectively, in the size (15 mer) of the 5'-labeled primer.

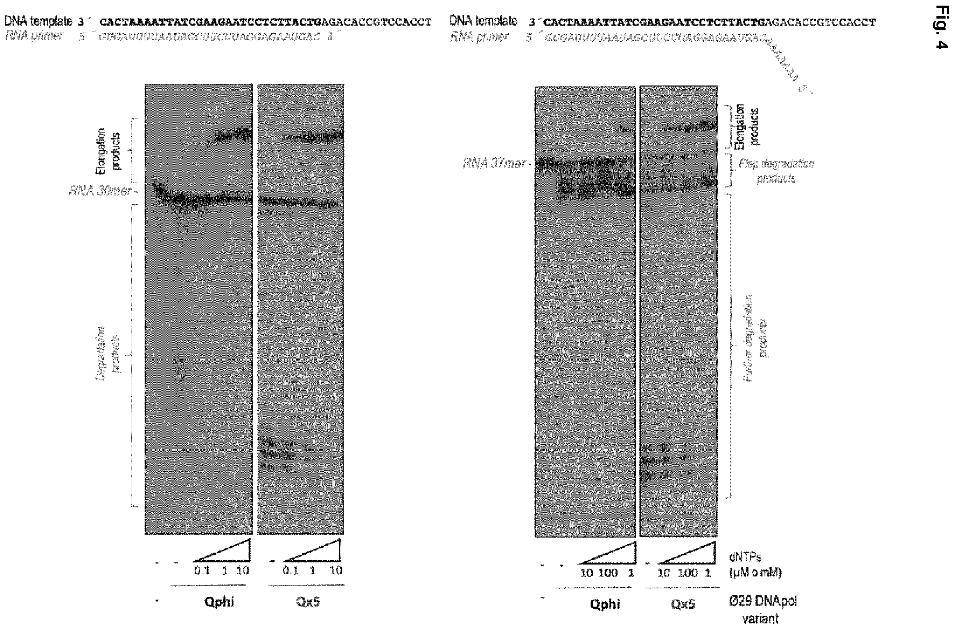

**Fig. 4****: The thermo-stabilized version of Qualiphi (Qx5) shows a more profficient polymerization from a RNA primer with a 3'terminal tail.** The coupling between synthesis and degradation was assayed as a function of the concentration of dNTPs as described above. A DNA oligonucleotide, containing the padlock sequence used for its circularization (5'-TCCACCTGCCACAGAGTCATTCTCCTAAGAAGCTATTAAAATCAC) was hybridized to both, an RNA oligo containing the SARS-CoV2 viral sequence proximal to the 3'-end polyA tail (*5'-GUGAUUUUAAUAGCUUCUUAGGAGAAUGAC*; left panel), or an RNA oligo containing the same viral sequence and the polyA tail (*GUGAUUUUAAUAGCUUCUUAGGAGAAUGACAAAAAAA*, right panel), obtaining the substrates 30/45 mer or 37/45mer, respectively. The incubation mixture contained in a final volume of 20 µL, 5 mM Tris-HCl pH 8.8, 1 mM Tris-HCl pH 7.4 (from the enzymes), 27.5 nM NaCl, 3.75 % (vol/vol) glycerol, 0.5 mM ATP, 5 mM DTT, 25 mM MgCl₂, 5 mM MgSO₄, 90 nM of the Phi29 DNApol variants Qphi or Qx5, 2.5 nM 5'-labeled hybrid indicated and the specified concentration of dNTPs (0, 0.1, 1, 10, 100 y 1000 µM). After incubation for 5 min at 30 °C, reactions were stopped by adding EDTA up to a final concentration of 10 mM and analyzed by 7 M urea/15% polyacrylamide gel electrophoresis and autoradiography. Polymerization or 3'-5' exonucleolysis is detected as an increase or decrease, respectively, in the size of the 5'-labeled primer.

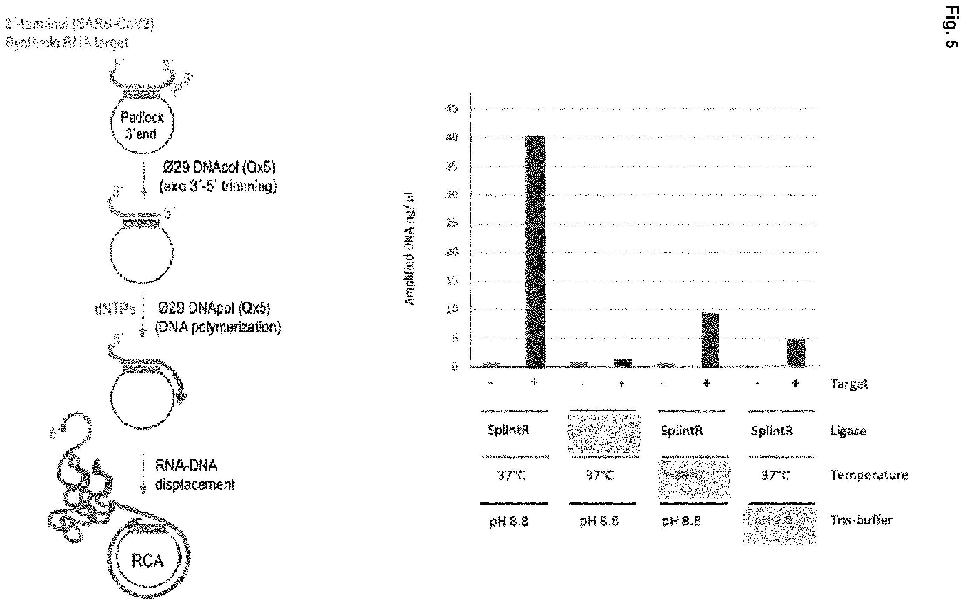

**Fig. 5****: The thermo-stabilized version of Qualiphi (Qx5) shows an optimal RCA yied at 37 °C and pH 8.8, in the presence of saliva.** As shown in the scheme, 10 nM of the SARS-CoV2-specific padlock 3' (**5'P-AAGAAGCTATTAAAATCAC**CGCAACTGAACTACTTGTCGCCTCAGCGCATCTCCT GATGAGGTTCCACCTGCCTCAGC**GTCATTCTCCT**, in bold, regions that hybridize to the target) was hybridized with 5 nM of the synthetic RNA target (*5'AUCCCCAU**GUGAUUUUAAUAGCUUCUUAGGAGAAUGAC**AAAAAAA,* in bold, region that hybridizes to the padlock) mimicking the 3'-end of SARS CoV-2 RNA_during the ligation step with SplintR ligase 12 U (New England Biolabs, Ipswich, USA) in the presence of 4 mM Tris pH 8.8, 1 mM Tris-HCl pH 7.4 (from the enzymes), 30 mM NaCl, 5% (vol/vol) glycerol, 1 mM ATP, 10 mM DTT and 10 mM MgCl₂ and 2.5 % thermo-inactivated non-infected saliva (saliva was mixed with TE 1:1, then incubated 95 °C during 30 min). Ligation reaction (10 µL) was incubated 30 min at 30°C. Then, amplification (total volume 20 µL) was performed in the presence of 2 mM Tris HCl 8.8, 1 mM Tris pH7.4 (from the enzymes), 40 mM NaCl, 7.5% (vol/vol) glycerol, 0.5 mM ATP, 5 mM DTT, 5 mM MgCl₂, 5 mM MgSO₄, 0.012% Tween-20, Qx5 (90 nM), and 2.5 mM dNTPs. The reaction was incubated for 1 h at 37°C. The amplified DNA (ng/µL) obtained by RCA was measured using a Quantus device from Promega. Evaluated variables are indicated in a grey box.

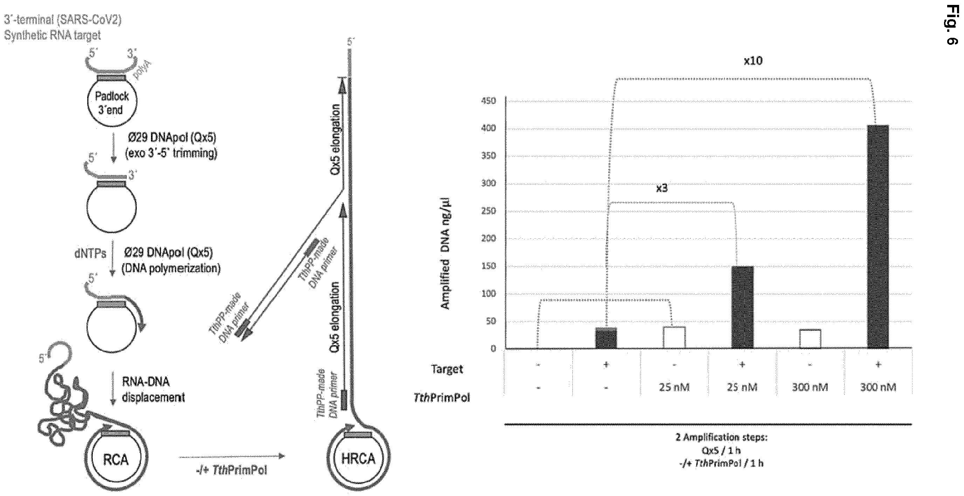

**Fig. 6****: Addition of *Tth*PrimPol (after the RCA step) significantly increases amplification.** As shown in the scheme, 10 nM of the SARS-CoV2-specific padlock 3'-end (**5'P-AAGAAGCTATTAAAATCAC**CGCAACTGAACTACTTGTCGCCTCAGCGCATCTCCT GATGAGGTTCCACCTGCCTCAGC**GTCATTCTCCT**; in bold, regions that hybridize to the target) was hybridized with 5 nM of the synthetic RNA target (*5'AUCCCCAU**GUGAUUUUAAUAGCUUCUUAGGAGAAUGAC**AAAAAAA*; in bold region that hybridizes to the padlock) mimicking the 3'-end of SARS CoV-2 RNA. Afterwards, intramolecular ligation of the padlock ends was performed in a final volume of 10 µL in the presence of 12 U SplintR ligase (New England Biolabs, Ipswich, USA), 4 mM Tris-HCl pH 8.8, 1 mM Tris-HCl pH 7.4 (from the enzymes), 30 mM NaCl, 5% glycerol , 10 µM EDTA, 1 mM ATP, 10 mM DTT, 10 mM MgCl₂ and 2.5% thermo-inactivated non-infected saliva (saliva was mixed with TE 1:1, then incubated 95 °C during 30 min). The reaction was incubated 30 min at 37 °C. The ligated product was further amplified in a 20 µL reaction volume in the presence of 2 mM Tris-HCl pH 8.8, 1 mM Tris-HCl pH7.4 (from the enzymes), 40 mM NaCl, 5% (vol/vol) glycerol, 0.5 mM ATP, 5 mM DTT, 5 mM MgCl₂, 5 mM MgSO₄, 90 nM Qx5 and 2.5 mM dNTPs during 1 h at 37 °C. Afterwards, *Tth*PrimPol was added to the rolling circle amplification at the indicated concentration (0, 25, 300 nM) and the reaction was allowed to continue for 1 h at 37 °C. DNA amplification (ng/µL) was measured using the fluorescent DNA binding dye QuantiFluor system and Quantus fluorometer from Promega (Madison, USA).

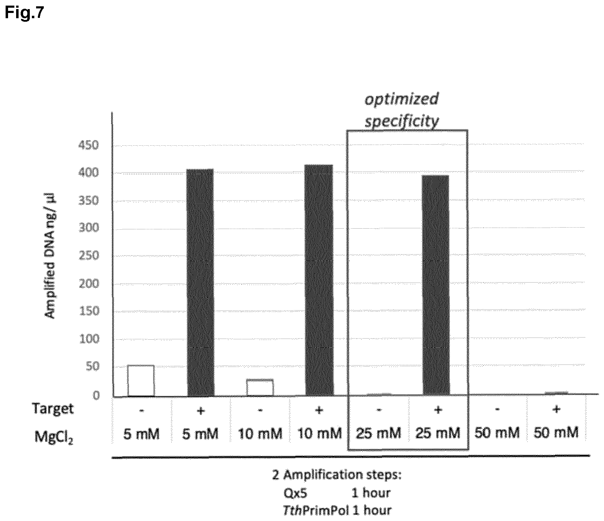

**Fig. 7****: 25 mM MgCl₂ reduces the background (off-target) amplification by *Tth*PrimPol.** 10 nM of the SARS-CoV2-specific padlock 3'end (**5'P-AAGAAGCTATTAAAATCAC**CGCAACTGAACTACTTGTCGCCTCAGCGCATCTCCT GATGAGGTTCCACCTGCCTCAGC**GTCATTCTCCT**; in bold regions that hybridize to the target) was hybridized with 5 nM of the synthetic RNA target (*5'AUCCCCAU**GUGAUUUUAAUAGCUUCUUAGGAGAAUGAC**AAAAAAA*; in bold region that hybridizes to the padlock), which mimicks the 3'-end of SARS-CoV2 RNA. Ligation reaction (10 µL) was carried out in the presence of 12 U SplintR (New England Biolabs, Ipswich, USA) 4 mM Tris-HCl pH 8.8, 1 mM Tris-HCl pH 7.4 (from the enzymes), 30 mM NaCl, 5% (vol/vol) glycerol, 10 µM EDTA, 1 mM ATP, 10 mM DTT, 2.5% thermo-inactivated non-infected saliva (saliva was mixed with TE 1:1, then incubated 95 °C during 30 min), various MgCl₂ concentrations (10, 20, 50 and 100 mM), and incubated for 30 min at 37 °C. Afterwards, the amplification of the ligated products (total volume 20 µL) was performed in the presence of 5 mM Tris-HCl pH 8.8, 1 mM Tris-HCl pH7.4 (from the enzymes), 40 mM NaCl, 7.5% glycerol, 0.5 mM ATP, 5 mM DTT, 5 mM MgSO₄ , 90 nM Qx5, 2.5 mM dNTPs, and the indicated concentration of MgCl₂ (5, 10, 25 and 50 mM) during 1 h at 37 °C. Then, *Tth*PrimPol (300 mM) was added and the reaction was allowed to continue for 1 h at 37 °C. DNA amplification (ng/µL) was measured using the fluorescent DNA binding dye QuantiFluor^{®} system and Quantus fluorometer from Promega (Madison, USA).

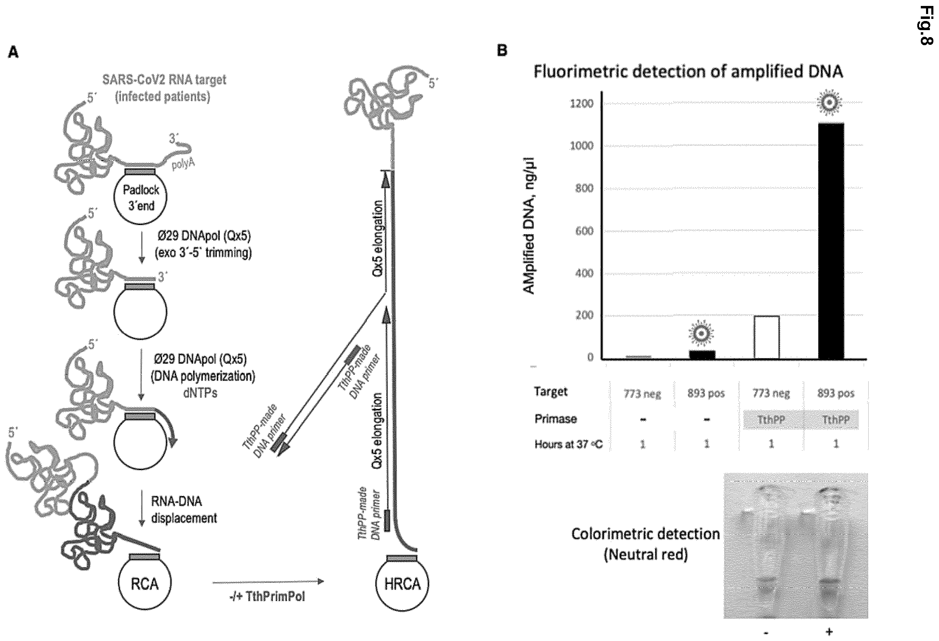

**Fig. 8****: Purified viral RNA from SARS-CoV2 infected patients can be detected by using the TthPrimPol-assisted padlock amplification method.** The ligation step was performed in a final volume of 10 µL in the presence of 4 mM Tris-HCl pH 8.8, 1 mM Tris-HCl pH 7.4 (from the enzymes), 30 mM NaCl, 5% glycerol, 10 µM EDTA, 1 mM ATP, 10 mM DTT, 10 mM MgCl₂, 10 nM padlock 3' end (5'P-**AAGAAGCTATTAAAATCAC**CGCAACTGAACTACTTGTCGCCTCAGCGCATCTCCT GATGAGGTTCCACCTGCCTCAGC**GTCATTCTCCT**-3'; in bold, the regions that hybridize to the target), 4 µL of RNA extracted from human nasopharyngeal swabs (provided by Begoña Aguado, CBMSO, Madrid), and 12 U SplintR ligase (New England Biolabs, Ipswich, USA). Samples were incubated for 30 min at 37 °C. Afterwards, amplification of the ligated products (20 µL) was performed in the presence of 5 mM Tris-HCl pH 8.8, 1 mM Tris-HCl pH 7.4 (from the enzymes), 40 mM NaCl, 5% (vol/vol) glycerol, 0.5 mM ATP, 5 mM DTT, 5 mM MgCl₂, 5 mM MgSO₄, 90 nM Qx5, 300 nM *Tth*PrimPol and 2.5 mM dNTPs. Reactions were incubated for 1 h at 37 °C. Samples were inactivated 5 min at 90 °C and the amplification product was quantified with the QuantiFluor^{®} system and Quantus fluorometer from Promega (Madison, USA), or alternatively by quick colour change detection with Neutral Red.

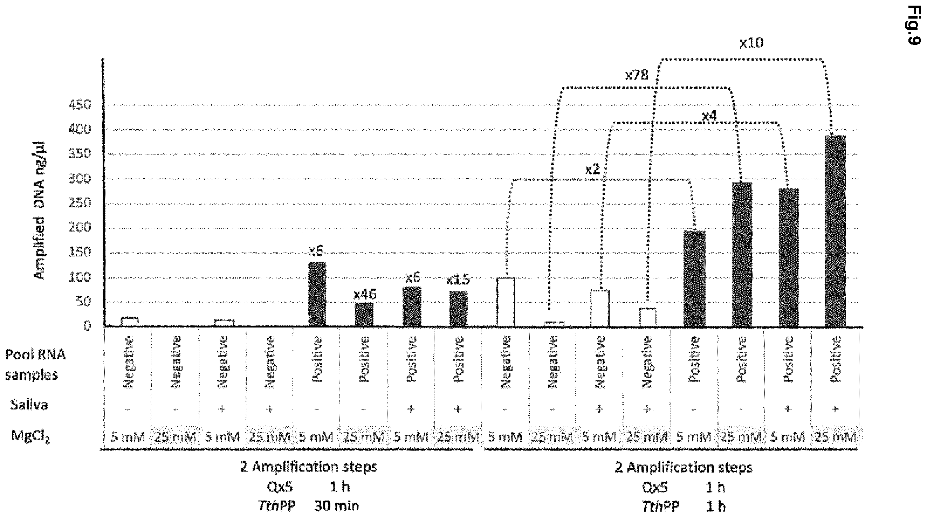

**Fig. 9****: 25 mM MgCl₂ improves the specificity of the TthPrimPol-assisted padlock amplification method, also for diagnosis of SARS-CoV2 infected patients.** The ligation step was performed in a final volume of 10 µL in the presence of 4 mM Tris-HCl pH 8.8, 1 mM Tris-HCl pH 7.4 (from the enzymes)30 nM NaCl, 5% glycerol, 10 µM EDTA, 1 mM ATP, 10 mM DTT, 10 mM MgCl₂, 10 nM padlock 3'end (5'P-**AAGAAGCTATTAAAATCAC**CGCAACTGAACTACTTGTCGCCTCAGCGCATCTCCT GATGAGGTTCCACCTGCCTCAGC**GTCATTCTCCT**- 3'; in bold regions that hybridize to the target), 1 µL of an extracted RNA pool from human nasopharyngeal swabs (provided by Begoña Aguado, CBMSO, Madrid), 12 U SplintR ligase (New England Biolabs, Ipswich, USA) and, when indicated 2.5% thermo-inactivated non-infected saliva (saliva was mixed with TE 1:1, then incubated 95 °C during 30 min). The samples were incubated 30 min at 37 °C. After that, the amplification reaction (20 µL) was performed in the presence of 5 mM Tris-HCl pH 8.8, 1 mM Tris pH7.4 (from the enzymes), 40 mM NaCl, 5% (vol/vol) glycerol, 0.5 mM ATP, 5 mM DTT, 5-25 mM MgCl₂, 5 mM MgSO₄, 90 nM Qx5 and 2.5 mM dNTPs, and incubated 1 h at 37 °C. Then, 300 nM *Tth*PrimPol was added and samples were incubated for additional 30 minutes or 1 h at 37 °C. The reaction was inactivated by heating during 5 min at 90 °C. The reaction products were analyzed using the QuantiFluor^{®} system and Quantus fluorometer from Promega (Madison, USA).

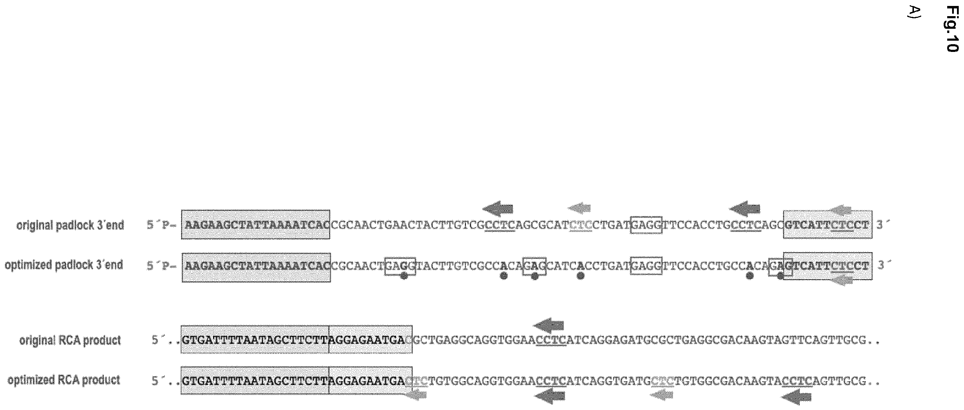

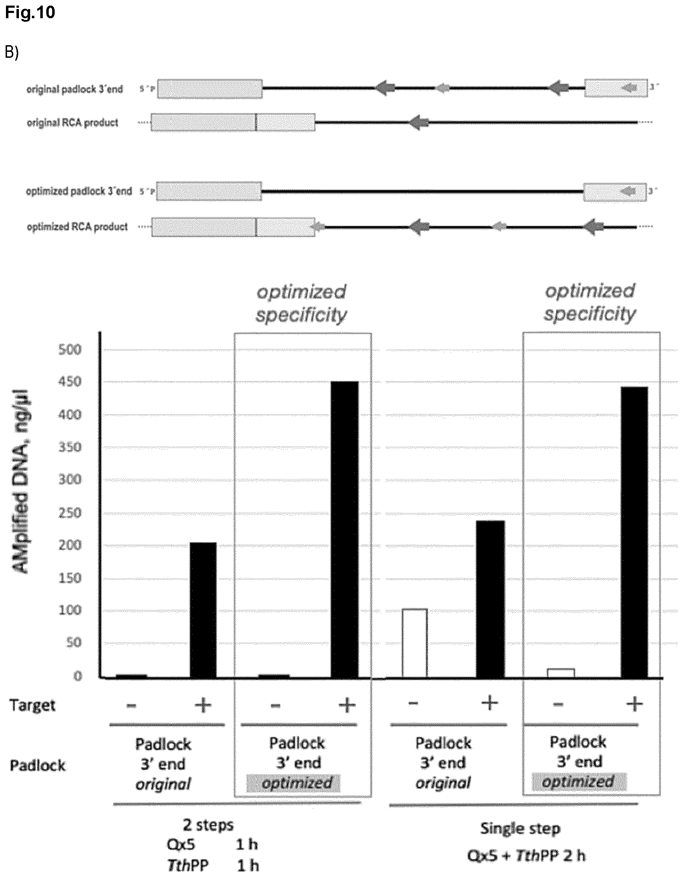

**Fig. 10****: A) Padlock sequence optimization, to preclude *Tth*PrimPol priming on the linear padlock, and improved priming on the RCA product.** Padlock 3'end and Optimized padlock 3'end (sequences at the top)): complementary sequences to SARS-CoV2 (bold, and boxed in yellow and orange); sequence that will give rise to an optimal *Tth*PrimPol priming site in the RCA product is boxed. Optimized padlock 3'end: nucleotides that have been changed from padlock 3'end are outlined with a dot. RCA products derived from the two padlocks (sequences at the bottom): direct DNA sequences corresponding to SARS-CoV2 (bold, and boxed in yellow and orange). Most preferred priming site for *Tth*PrimPol (bold/underlined and with a dark arrow); low affinity priming site for *Tth*PrimPol (underlined and with a light arrow)._**B) The use of an optimized padlock increases the amplification yield and reduces the background.** Upper panel shows a scheme of the original and optimized 3'end padlock described in more detail in Figure 10A. Lower panel shows the amplification yield obtained with each padlock and where ligation of the padlocks was performed in a final volume of 10 µL in the presence of 4 mM Tris-HCl pH 8.8, 1 mM Tris-HCl pH 7.4 (from the enzymes), 30 nM NaCl, 5% glycerol, 10 µM EDTA, 1 mM ATP, 10 mM DTT, 10 mM MgCl₂, 10 nM padlock 3'end (5'P-**AAGAAGCTATTAAAATCAC**CGCAACTGAACTACTTGTCGCCTCAGCGCATCTCCT GATGAGGTTCCACCTGCCTCAGC**GTCATTCTCCT**-3'; in bold, the regions that hybridize to the target) or the optimized padlock 3' end (5'P-**AAGAAGCTATTAAAATCAC**CGCAACTGAGGTACTTGTCGCCACAGAGCATCACCT GATGAGGTTCCACCTGCCACAGA**GTCATTCTCCT**-3'; in bold, the regions that hybridize to the target), 5 nM synthetic RNA target oligo corresponding to the 3' end of SARSCoV2 (*5'-AUCCCCAU**GUGAUUUUAAUAGCUUCUUAGGAGAAUGAC**AAAAAAA-3';* in bold region that hybridizes to the padlock), 12 U SplintR ligase (New England Biolabs, Ipswich, USA) and 2.5% thermo-inactivated non-infected saliva (saliva was mixed with TE 1:1, then incubated 95 °C during 30 min). Samples were incubated 30 min at 37 °C. After that, the amplification reaction was carried out in 20 µL in the presence of 5 mM Tris-HCl pH 8.8, 1 mM Tris-HCl pH7.4 (from the enzymes), 40 mM NaCl, 5% (vol/vol) glycerol, 0.5 mM ATP, 5 mM DTT, 25 mM MgCl₂, 5 mM MgSO₄, 90 nM Qx5, and 2.5 mM dNTPs. In the 2-steps amplification protocol, the amplification reaction with Qx5 was incubated for 1 h at 37 °C and afterwards 300 nM *Tth*PrimPol was added, allowing the reaction to proceed for 1 h at 37 °C. In the 1-step amplification protocol, the samples contained also 300 nM *Tth*PrimPol at the beginning of the amplification, and the reactions were incubated for 2 h at 37 °C. The reactions were inactivated by heating 5 min at 90 °C and the amplification products analyzed using the QuantiFluor^{®} system and Quantus fluorometer from Promega (Madison, USA).

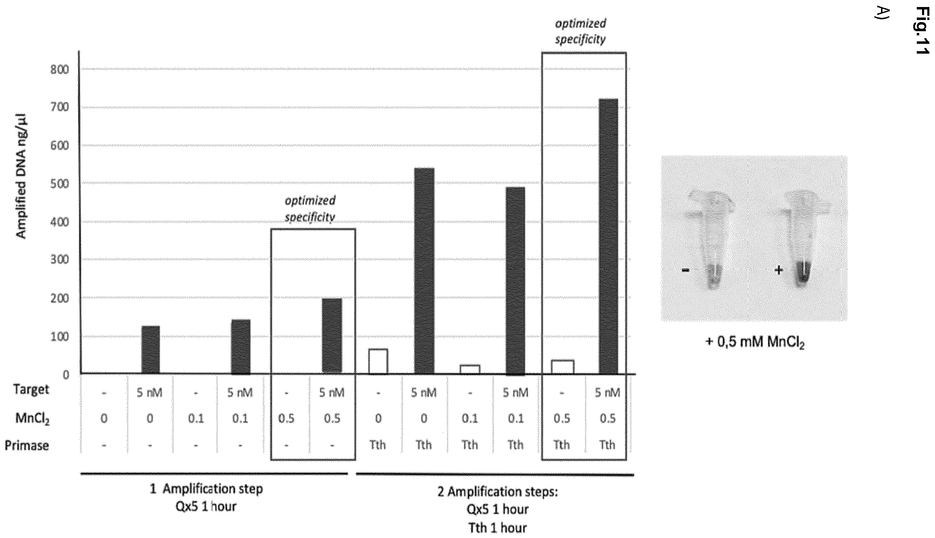

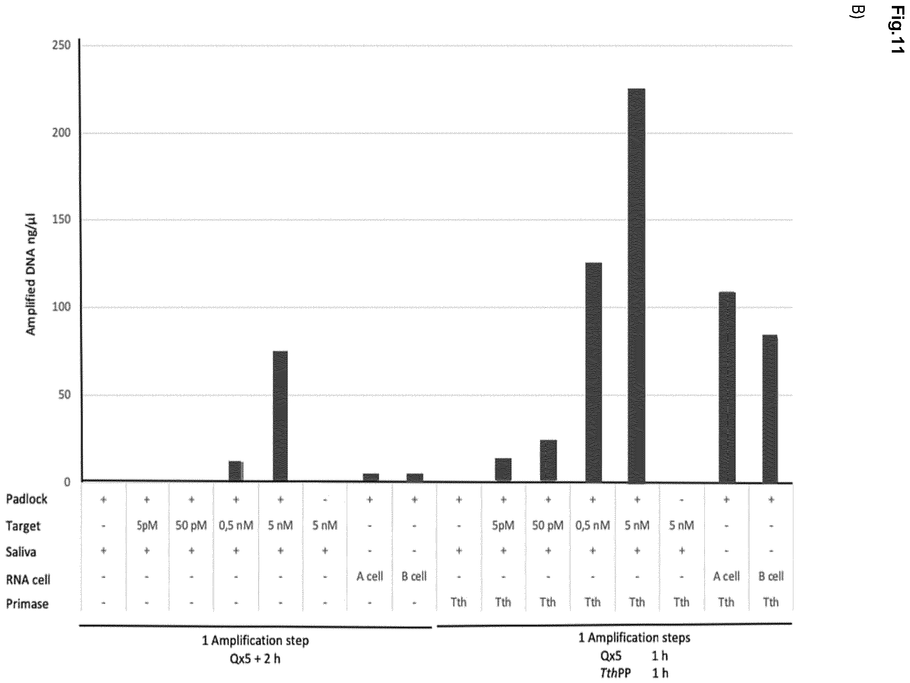

**Fig. 11****: A) Mn²⁺ ions used to boost *Tth*PrimPol synthesis of primers also improve RCA by Qx5.** Optimized Padlock 3' end (10 nM) (5' P-**AAGAAGCTATTAAAATCAC**CGCAACTGAGGTACTTGTCGCCACAGAGCATCACCT GATGAGGTTCCACCTGCCACAGA**GTCATTCTCCT** 3'; in bold region that hybridize to the target) was ligated with 12 U SplintR ligase (New England Biolabs, Ipswich, USA) using 5 nM of the synthetic RNA target (*5'AUCCCCAU**GUGAUUUUAAUAGCUUCUUAGGAGAAUGAC**AAAAAAA*; in bold region that hybridizes to the padlock). Ligation (10 µL) was performed in the presence of 4 mM Tris-HCl pH 8.8, 1 mM Tris pH7.4 (from the enzymes), 30 mM NaCl, glycerol 5% (vol./vol.), 10 µM EDTA, 1 mM ATP, 10 mM DTT, 25 mM MgCl₂ and 2.5% thermo-inactivated non-infected saliva (saliva was mixed with TE 1:1, then incubated 95 °C during 30 min). Then, amplification (total volume 20 µL) of the ligated products was carried out in the presence of 5 mM Tris HCl pH 8.8, 1 mM Tris pH7.4 (from the enzymes), 40 mM NaCl, 2.5 mM KCI, 7.5% (vol/vol) glycerol, 0.5 mM ATP, 25 mM MgCl₂, 5 mM DTT, 5 mM MgSO₄, 90 nM Qx5 and 2.5 mM dNTPs, and the indicated concentrations of MnCl₂ (0, 0.1, 0.5 mM). Half of the samples were inactivated for 5 min at 90 °C. 300 nM *Tth*PrimPol was added to the other half, and samples were further incubated for 1h at 37 °C. DNA amplification (ng/µL) was measured using the fluorescent DNA binding dye QuantiFluor^{®} system and Quantus fluorometer from Promega (Madison, USA). **B) PrimPol-assisted amplification of padlock probes, boosted with Mn²⁺ ions, decreases the detection limit of synthetic RNA targets, and improves detection of SARS-CoV2 RNA from infected cells.** 10 nM of the optimized Padlock 3' end (5'P-**AAGAAGCTATTAAAATCAC**CGCAACTGAGGTACTTGTCGCCACAGAGCATCACCT GATGAGGTTCCACCTGCCACAGA**GTCATTCTCCT**-3'; in bold, regions that hybridize to the target) was hybridized either to increasing concentrations (5 pM, 50 pM, 0.5 nM and 5 nM) of the synthetic RNA target (*5'AUCCCCAU**GUGAUUUUAAUAGCUUCUUAGGAGAAUGAC**AAAAAAA*; in bold, region that hybridizes to the padlock), or to total RNA extracted from SARS-CoV2 infected cells (A and B pools). The ligation reaction (10 µL) was performed in the presence of SplintR ligase (12 U, New England Biolabs, Ipswich, USA), 4 mM Tris pH 8.8, 1 mM Tris-HCl pH 7.4 (from the enzymes), 30 mM NaCl, glycerol 5% (vol./vol.), 1 mM ATP, 10 mM MgCl₂, 10 mM DTT and 2.5% thermo-inactivated non-infected saliva (saliva was mixed with TE 1:1, then incubated 95 °C during 30 min), when indicated. The reaction was incubated 30 min at 37 °C. Then, amplification reaction (total volume 20 µL) was carried out in the presence of 2 mM Tris-HCl pH 8.8, 1 mM Tris pH 7.4 (from the enzymes), 40 mM NaCl, 2.5 mM KCI, 7.5% (vol/vol) glycerol, 0.5 mM ATP, 5 mM DTT, 5 mM MgSO₄, 25 mM MgCl₂, 0.5 mM MnCl₂ and Qx5 (90 nM) during 1 h at 37 °C. Afterwards, half of the samples were incubated by 1 h, and *Tth*PrimPol (300 mM) was added to the other half to continue the reaction for 1 h at 37 °C. DNA amplification (ng/µL) was measured using the fluorescent DNA binding dye QuantiFluor^{®} system and Quantus fluorometer from Promega (Madison, USA).

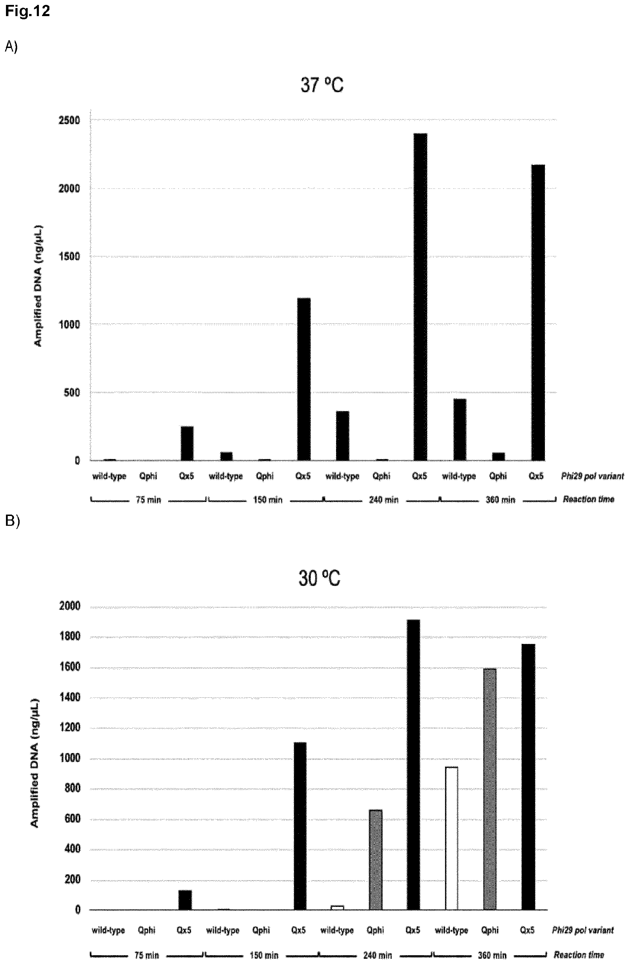

**Fig.12****: Qx5 elongates DNA primers generated by *Tth*PrimPol more efficiently than Qualiphi.** The Figure shows the result of Hyperbranched Rolling Circle Amplification (HRCA) of M13mp18 ssDNA (2 nM) by 100 nM of the indicated phi29 DNApol variant combined with 300 nM *Tth*PrimPol. Reaction conditions (20 µL) were as described in the TruePrime amplification kit [4BB^{™}TruePrime^{®} Whole Genome Amplification (WGA) kit)]. Reactions were incubated at either 37 °C or 30 °C, during the indicated time. DNA amplification (ng/µL) was measured using the fluorescent DNA binding dye QuantiFluor^{®} system and Quantus fluorometer from Promega (Madison, USA).

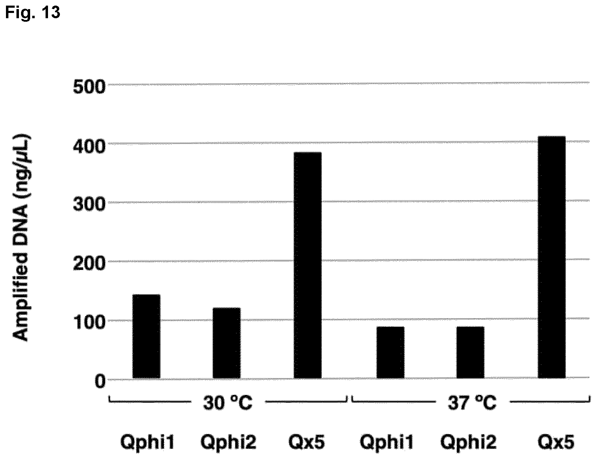

**Fig.13****: Qx5 elongates random hexameric DNA primers more efficiently than Qualiphi.** The Figure shows the result of Hyperbranched Rolling Circle Amplification (HRCA) of M13mp18 ssDNA (2 nM) by 100 nM of the indicated phi29 DNApol variant (two different preparations of Qphi (1 & 2) were assayed) using DNA random hexamers (10 µM). Reaction conditions (20 µL) were as described in the TruePrime amplification kit [4BB^{™}TruePrime^{®} Whole Genome Amplification (WGA) kit)]. Reactions were incubated during 75 min at either 37 °C or 30 °C, as indicated. DNA amplification (ng/µL) was measured using the fluorescent DNA binding dye QuantiFluor^{®} system and Quantus fluorometer from Promega (Madison, USA).

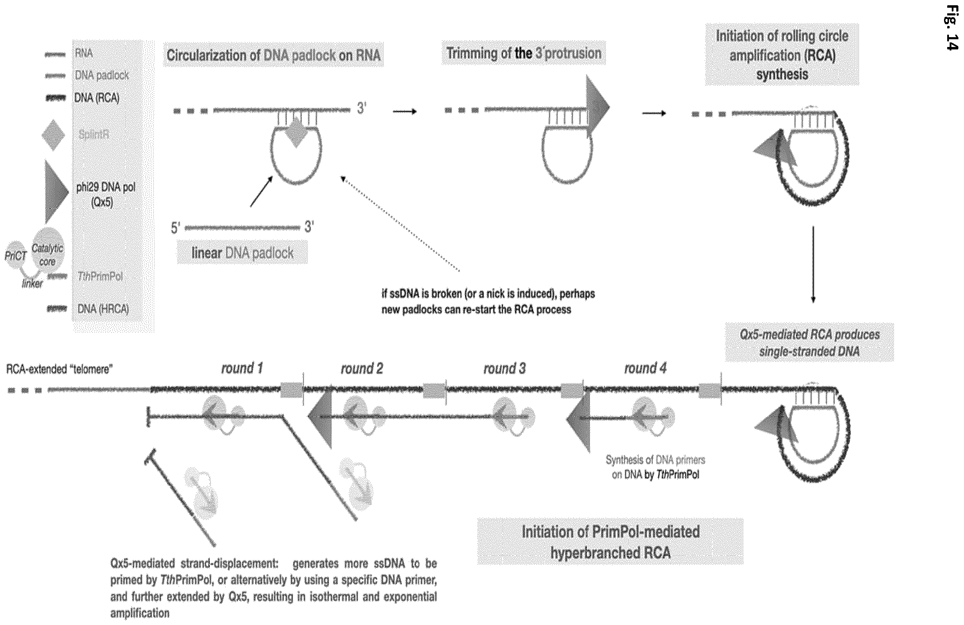

**Fig. 14****:** Preferred embodiment of the novel method for isothermal amplification of padlock probes for RNA detection, as described in the present invention, based on primer-less HRCA and in the use of an improved version (Qx5) of phi29 DNApol.

### GENERAL DEFINITIONS

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

It is noted that the term "about", as used herein, refers to +/- 30%, +/- 20%, preferably +/- 15%, more preferably +/- 10%, of the indicated referred value.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of does not exclude materials or steps that do not materially affect the basic and novel characteristics of the claim.

"Percent (%) amino acid sequence identity" with respect to proteins or polypeptides described herein is defined as the percentage of amino acid residues in a candidate sequence that are identical with the amino acid residues in the reference sequence (i.e., the protein or polypeptide from which it is derived), after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for example, using publicly available computer software such as BLAST. Those skilled in the art can determine appropriate parameters for measuring alignment, including any algorithms needed to achieve maximum alignment over the full-length of the sequences being compared.

Preferably, the "percentage of identity" as used herein is decided in the context of a local alignment, i.e., it is based on the alignment of regions of local similarity between nucleobase sequences, contrary to a global alignment, which aims to align two sequences across their entire span. Thus, in the context of the present invention, percentage identity is calculated preferably only based on the local alignment comparison algorithm.

The term "complementary" and "complementarity" are interchangeable and refer to the ability of polynucleotides to form base pairs with one another. Base pairs are typically formed by hydrogen bonds between nucleotide units in antiparallel polynucleotide strands or regions. Complementary polynucleotide strands or regions can base pair in the Watson-Crick manner (e.g., A to T, A to U, C to G). 100% (or total) complementary refers to the situation in which each nucleotide unit of one polynucleotide strand or region can hydrogen bond with each nucleotide unit of a second polynucleotide strand or region. Less than perfect (or partial) complementarity refers to the situation in which some, but not all, nucleotide units of two strands or two regions can hydrogen bond with each other and can be expressed as a percentage. Please note that the term "complementary" as used in the present invention also encompasses the term "substantially complementary". A region is "substantially complementary" to a target region when the percentage of complementarity between both regions is of at least 60%, at least 70%, at least 80%, at least 85%, at least 90%, at least 93%, at least 95%, at least 96%, at least 97%, at least 98%, at least 99% or at least 100%. A region is "substantially complementary" to another region if they hybridize under low stringency conditions, preferably medium stringency conditions, most preferably high stringency conditions. Similarly, when the term "non-complementary" is used herein, it should be understood that said term also encompasses the term "substantially non-complementary". A region is "substantially non-complementary" to another region when the complementarity between both regions is less than 40%, less than 30%, less than 20%, preferably less than 10%, most preferably less than 5%, or even most preferably 0% complementarity. A region is "substantially non-complementary" to another region if they do not hybridize under high stringency conditions, preferably medium stringency conditions, most preferably low stringency conditions.

By "functional equivalents" is referred herein to other nucleotide sequences or amino acid sequences that differ in their nucleobase or amino acid sequences, respectively, from that of SEQ ID NO referred to in the specific embodiment, but which perform the same function and provide the same utility or technical effect as the SEQ ID NO: referred to in the specific embodiment.

The term "hybridization" is used to refer to the structure formed by 2 independent strands of nucleic acids that form a double stranded structure via base pairings from one strand to the other. These base pairs are considered to be G-C, A-U/T and G-U. (A - Adenine, C -Cytosine, G- Guanine, U - Uracil, T - thymine). As in the case of the complementarity, the hybridization can be total or partial. In the context of the present invention, each uracil and thymine base can be optionally replaced, respectively, by a thymine or uracil base. In general, whether such hybridization takes place is influenced by, among other things, the length of the polynucleotides and the complementary, the pH, the temperature, the presence of mono- and divalent cations, the proportion of G and C nucleotides in the hybridizing region, the viscosity of the medium, and the presence of denaturants. Such variables influence the time required for hybridization. Thus, the preferred annealing conditions will depend upon the particular application. Such conditions, however, can be routinely determined by the person of ordinary skill in the art without undue experimentation.

The term "rolling-circle amplification" or "RCA" refers to an isothermal enzymatic process where a short DNA or RNA primer triggers continuous amplification of a circular DNA template (i.e a circular padlock probe) to form a long single-stranded DNA by using a strand-displacement competent DNA polymerase, as phi29 DNA pol. Thus, the RCA product is a concatemer containing tens to hundreds of tandem repeats that are complementary to the padlock probe. In the method of the present invention, the 3'end of the RNA target is the only primer used for RCA.

The term "hyperbranched rolling-circle amplification" or "HRCA" refers to an RCA reaction coupled to a MDA reaction.

The expression "3' region", as used herein, refers to a region of a nucleotide strand that includes the 3' end of said strand. As used herein, the "3' region" and the "3' terminal region" refer to the same region of a nucleotide strand and are used interchangeably. The term "3' end", as used herein, designates the end of a nucleotide strand that has the hydroxyl group of the third carbon in the sugar-ring of the ribose or deoxyribose at its terminus. The expression "5' region", as used herein, refers to a region of a nucleotide strand that includes the 5' end of said strand. As used herein, the "5' region" and the "5' terminal region" refers to the same region of a nucleotide strand and may be used interchangeably. The term "5' end", as used herein, designates the end of a nucleotide strand that has the fifth carbon in the sugar-ring of the deoxyribose at its terminus.

As used herein, the term "portion" when referred to a nucleotide sequence means a nucleotide region or fragment of said sequence. Preferably, in the context of the present invention, a portion is a region or fragment of about 50, 40, 30, 20, or 10 nucleotides.

As used herein, the term nucleotides or nucleotide analogues is not limited in particular, and is meant to include deoxynucleoside triphosphates (dNTPs) such as for example, but not limited to dATP, dCTP, dGTP, dTTp, dITP, dUTP or derivatives thereof. As non limiting examples of such derivatives reference is made to dideoxynucleotides such as ddATP, ddCTP, ddGTP, ddTTp, ddITP, ddUTP, or oxidized derivatives like 8oxoA, 8oxoG, 5OHC, 5OHU, or labelled derivatives like fluorescent labelled derivatives or even more complex labels like the labelles used for third generation sequencing.

### DESCRIPTION OF THE EMBODIMENTS

The present invention relates to an isothermal amplification method, referred from herein after as to "the method of the present invention". For the method of the present invention (see Fig. 1D), only 3 enzymes are needed: SplintR (to ligate the padlock to its circular form, when viral RNA is present), bacteriophage Phi29 DNApol (phi29 DNApol) or any variants thereof, and the unique DNA primase *Tth*PrimPol. This novel HRCA method relies on the use of a specific ssDNA padlock.

A first bottle neck of standard HRCA method relates to the efficiency of padlock circularization, thus implying the use of 37 °C to optimize both the efficiency and specificity of the ligation reaction by SplintR ligase. Consequently, we needed to improve the thermoresistance of phi29 DNApol, which is routinely used at 30°C due to its thermolability. For this method, the variant of phi29 DNApol named Qualiphi (Qphi), was selected, as it has an improved DNA binding which allows amplification from limited amounts of template (de Vega et al., PNAS 2010). The sequence of Qualiphi was modified to include 3 (Qx3) or 5 (Qx5) point mutations. As shown in Figure 2, whereas the optimal reaction temperature for the Qualiphi wild-type version was 32-34 °C (see lower panel/2 h in Figure 2), the amplification capacity of Qx3 and Qx5 remained constant over the range 30-37 °C and 30-39 °C, respectively, the latest showing the highest thermo-resistance. On top of that, and even at the lowest reaction temperature assayed (30 °C), the thermo-stabilized versions Qx3 and Qx5 yielded a much higher amount of amplification product than the wild-type Qualiphi enzyme, resulting in large amplification of the padlock even at 1 h of reaction (Fig 2, upper panel). Thus, the mutations included in the Qualiphi variant resulted in an improved polymerase.

The superior activity of the thermo-stabilized variants Qx3 and Qx5 in the RNA-targeted amplification of the circularized padlock was found to be due to a more efficient recognition of the DNA/RNA junction (as the one produced after RNAse H nicking) required to start the initial RCA process, as shown in Fig. 3. It was found that on the DNA template/RNA primer substrate (mimicking that generated during RNA-primed amplification of the DNA padlock), Qx5 showed a much higher exoribonucleolytic and polymerization activities than Qualiphi. Surprisingly, as shown in Figure 4, the exonuclease activity exhibited by Qx5 was much more efficient than that by Qualiphi, providing a more efficient removal of the 3'-end polyA tail, allowing the enzyme to get a net elongation of the RNA, which acts as a primer after the exhaustive exonucleolytic removal of the polyA tail. Additionally, the efficiency of Qx5 was also shown to be superior to that of Qualiphi when random DNA primers were added, see Fig. 13. These results support the convenient use of Qx3 and Qx5 variants not only in the detection method described in the present invention, but also in more general methods to amplify nucleic acids.

Experimental conditions of standard RCA methods were also tested and improved. Figure 5 summarizes the optimal conditions in which the highest specificity and efficiency can be obtained: 1) presence of target RNA; 2) Specific (target-assisted) ligation of the padlock ends by SplintR; 3) Amplification temperature of 37 °C and alkaline pH 8.8.

To improve the sensitivity of the method one step further, the primer-less RCA step was coupled to a second isothermal amplification step without requiring the addition of primers thanks to the use of *Tth*PrimPol. Figure 6 shows the efficiency and specificity of padlock amplification in the presence of saliva, wherein the addition of *Tth*PrimPol increased the ssDNA amplification product 3-fold and 10-fold, respectively, improving the sensitivity of the detection procedure. Further method optimizations included the use of 25 mM Mg²⁺ (see Fig. 9).

Altogether, these results indicate that using 25 mM MgCl₂ in combination with *Tth*PrimPol extremely improved both, the sensitivity and the specificity of detection of the viral RNA extracted from patient samples, and the effect was even better when using a biological sample such as saliva.

In order to decrease the off-target amplification of the linear padlock, but to improve *Tth*PrimPol priming on the target-specific RCA product, the sequence of the original padlock 3'-end was optimized (Fig. 10A, as explained in the Examples section). As shown in Fig. 10B (lower panel), the amount of target-dependent amplification obtained with the optimized padlock was higher than that produced with the non-optimized padlock. Importantly, the optimized padlock did not produce a significant background, even when using the single step protocol, which is very convenient to simplify the procedure. Altogether, these results indicate that using 25 mM MgCl₂ in combination with *Tth*PrimPol and the optimized padlock probe extremely improved both, the sensitivity and the specificity of the detection procedure.

To explore the effect of Mn²⁺ ions on the *Tth*PrimPol-assisted amplification, two experimental conditions were compared. Fig 11A shows the higher production of amplified DNA observed during the RCA reaction, which indicates the stimulation of the amplification capacity of Qx5 by Mn²⁺ ions. Further, as shown in Figure 11B, the sequential use of an RCA step by Qx5, followed by a *Tth*PrimPol-assisted HRCA step, using 25 mM MgCl₂, 0.5 mM MnCl₂, pH 8.8 and 37 °C allowed the sensitive and specific detection of a low concentrations (5 pM) of the synthetic RNA oligo, and SARS-CoV2 RNA obtained from infected cells.

To further evaluate whether the mutations introduced in Qualiphi also provided an advantage to extend the PrimPol-generated DNA primers, when amplifying the ssDNA generated during the RCA, the authors of the present invention also tested the effect of said polymerase using different type of primers. The results, shown in Fig. 12, demonstrate that the mutations introduced make Qx5 a more robust enzyme to elongate both, an RNA primer (as needed *ab initio* of the first RCA step of the method) and DNA primers (produced during the second PrimPol-mediated MDA step of the method). Unexpectedly, the superior amplification by Qx5 was maintained when the reaction temperature was 30 °C (see Figure 12B), supporting the benefit of using Qualiphi variants in other DNA amplification methods performed at low DNA temperature.

In view of the above, **a first aspect** of the present invention relates to an improved method (from herein after referred to as the "method of the present invention") to carry out RCA, preferably HRCA, method. In an embodiment, the method of the present invention in a RCA method that comprises the steps of:
a) contacting a target nucleic acid with at least one padlock probe (from herein after, the at least one padlock probe is also referred herein as the padlock of the present invention),
b) circularising the padlock probe whose 5' and 3' end sequences have hybridized to the target nucleic acid by ligating the ends of said padlock probe,
c) subjecting the circularized padlock probe to rolling circle amplification (RCA), wherein the RCA is carried out by a bacteriophage phi29 DNApol and detecting the amplified product.

In a preferred embodiment, the method of the present invention is a HRCA method, comprising the steps of:
a) contacting a target nucleic acid with at least one padlock probe of the present invention,
b) circularising the padlock probe whose 5' and 3' end sequences have hybridized to the target nucleic acid by ligating the ends of said padlock probe,
c) subjecting the circularized padlock probe to rolling circle amplification (RCA), wherein the RCA is carried out by a bacteriophage phi29 DNApol,
d) during and/or after step c), amplifying the product of c) with the primase/polymerase of *Thermus thermophilus,* and
e) detecting the amplified product.

Preferably, the method is for detecting a single-stranded target nucleic acid, preferably single-stranded RNA, and preferably in the absence of exogenous primers or in the absence of further oligonucleotides that prime the amplification, as will be explained below. Also preferably, the method is to measure the levels of a single-stranded target nucleic acid present in a sample. Each of the steps of the method of the present invention are further explained below:
Step a) of the method of the present invention comprises or consists of contacting the target nucleic acid with at least one padlock of the present invention.

Step a) comprises a hybridization reaction between the at least one padlock of the present invention and the target nucleic acid, in which the at least one padlock forms a complex with a target nucleic acid. Preferably, the target nucleic acid is a ribonucleotide and the padlock is a DNA molecule, so the hybridization reaction of step a) preferably refers to the ability of complementary single-stranded DNA (the padlock) to form a heteroduplex (DNA:RNA hybrid) with the a single-stranded RNA (the target).

Preferably, the padlock of the present invention is a single stranded DNA nucleotide sequence whose 3' and 5' ends are complementary to a region of a target nucleic acid. Thus, the padlock of the present invention comprises a nucleotide sequence located at the 5' end of the padlock probe that is complementary (including substantially complementary, see definitions above) to a first region of the target nucleic acid. The nucleotide sequence located at the 5' end of the padlock and that is complementary (including substantially complementary) to a first region of the target nucleic acid is named herein the "5' target-specific hybridization region of the padlock".

The padlock of the present invention further comprises a nucleotide sequence located at the 3' end of the padlock probe that is complementary (including substantially complementary, see definitions above) to a second region of the target nucleic acid. The nucleotide sequence located at the 3' end of the padlock and that is complementary (including substantially complementary) to a second region of the target nucleic acid is named herein the "3' target-specific hybridization region of the padlock".

In an embodiment, the "5' target-specific hybridization region of the padlock" and the "3' target-specific hybridization region of the padlock" are located exactly at the 5' and 3' ends of the padlock probe, and are capable of hybridizing, under suitable hybridizing conditions, with the first and second regions, respectively, of the target nucleic acid, resulting in a circularizable padlock probe that is ready for ligation. A padlock in a circularizable form, also called herein "circularizable padlock", is thus a target-specific, ligation-dependent circularizable probe. Suitable hybridization conditions, which are within the knowledge of those skilled in the art.

In an embodiment, the 5' and 3' target-specific hybridization regions of the padlock of the present invention comprises a nucleotide sequence that is at least 70%, 75%, 80%, 85%, preferably 90%, 92%, 94%, 96%, 98% or 100% complementary to a first and a second region, respectively, of the target nucleic acid. Percentages of complementarity lower than 100% are possible as long as the 5' and 3' target-specific hybridization regions of the padlock probes are capable of hybridizing, under suitable hybridizing conditions, with the first and second regions, respectively, of the target nucleic acid, resulting in a circularizable padlock probe.

The 5' and 3' regions of the padlock of the present invention are thus characterized by each having a "target-specific hybridization region", which are the 5' target-specific hybridization region and the 3' target-specific hybridization region, respectively. The 5' and 3' target-specific hybridization regions of the padlock probe may be of any suitable length as long as they allow the padlock probe to stably hybridise to the target nucleic acid sequence resulting in a circularizable probe, as will be explained in detail below. For example, each target-specific hybridization region may be 6-30 nucleotides in length. A minimum size of 6 nucleotides is selected to ensure hybridization specificity.

Thus, preferably the size range of each of the target-specific hybridization regions may be 6-30, 6-25, 6-20, 6-19, 6-18, 6-17, 6-16, 6-15, 6-14, 6-13, 6-12, 6-11, 10-25, preferably 10-20 or 6-10 nucleotides in length. The length of each target-specific hybridization regions of a probe may be the same or different, and thus for example a probe may have target-complementary regions of 6+6, 6+7, 7+7, 6+8, 7+8, 8+8, 8+9, 7+9, 9+9, 10+9, 10+10, 10+11, 11+11, 11+12, 12+12 etc., i.e., any combination of any integers within the above-noted ranges. In a preferred embodiment, the 3' target-specific hybridization region is 11 nucleotides in length and the 5' target-specific hybridization region is 19 nucleotides in length, or a length with a variation of ± 1, 2, 3, 4, 5, 6, 7, 8, 9, or 10 nucleotides.

The total length of the hybridized region (i.e., the sum of the 5' and 3' target-specific hybridization regions) may be 12-60, 12-50, more particularly 12-40 nucleotides, more particularly 12-36, 12-32 or 12-30, 12-28, 12-26, 12-24, 12-23, 12-22, 12-21 , 12-20, 12-19, 12-18, 12-17, 12-16, 10 13-30, 13-28, 13-26, 13- 24, 13-23, 13-22, 13-21 , 13-20, 13-19, 13-18, 13-17, 13-16, 14-30, 14-28, 14-26, 14-24, 12-23, 14-22, 14-21 , 14-20, 12-19, 14-18, 14-17, 14-16, 15-30, 15-28, 15-26, 15-24, 15-23, 15-22, 15-21 , 15-20, 15-19, 15-18, 15-17, or 15-16 nucleotides. Preferably, the hybridization region comprises 30 nucleotides, or a length with a variation of 30 ± 1, 2, 3, 4, 5, 6, 7, 8, 9, or 10 nucleotides.

The padlock of the present invention further comprises a backbone sequence. This backbone sequence is located between the two target-specific hybridization regions, preferably immediately adjacent to them, and is not complementary (including not substantially complementary) to the target nucleic acid. The length of the backbone sequence is not particularly limited, but it may be long enough to allow the padlock to have sufficient flexibility to hybridize to the target nucleic acid and being circularized. In a preferred embodiment, the backbone sequence has a total length that is equal or longer than the sum of the 5' and 3' target-specific hybridization regions. Preferably, the backbone sequence is at least 30 nucleotides in length, preferably at least 40, 45, 50, 60, 70, or 80, nucleotides in length, preferably between 45-50, 45-80, 45-90, 45-100, 45-120, 45-140, or 45-160, nucleotides in length.

The first and second region of the target nucleic acid are complementary to the 5' and 3' target-specific hybridization regions of the padlock probe, respectively. This is essential because the padlock probe will adopt a circularizable form upon hybridization to the target, which will bring together the 5' and 3' ends of the padlock. Hence, the target-specific domain located at the 5' and 3' ends of the padlock probe are complementary to and capable of hybridising to adjacent sequences in a target nucleic acid, such that the hybridisation of the padlock probe to the target results in the ends of the padlock probe being brought into juxtaposition, which results in the padlock form to adopt a circularizable form which is then ready for ligation. Use of the term "adjacent" is herein intended to mean that there are no nucleotides (i.e., there are 0 nucleotides) of the target sequence left without base-pairing between the first and second regions of the target nucleic acid sequence which are base paired to the target-specific hybridization regions of the padlock probe. This proximity between the first and second regions of the target nucleic acid enables the 5' and 3' ends of the padlock probes to be close to each other, and be ligated in step b) to provide a circular, covalently-closed padlock.

Some RCA methods make use of a RNAseH to eliminate the regions of a target ribonucleic acid that are not hybridized to the padlock probe, in order to prepare the hybrid ribonucleic acid-padlock for the RCA reaction which is primed by the 3'end of the RNAseH-digested RNA. Alternatively, an exogenous DNA oligonucleotide, complementary to the backbone of the padlock can be used as a primer. It was found herein that neither the RNAseH nor added DNA primers are necessary if the padlock probe is designed to hybridize in the 3' region of the target nucleic acid (see Fig. 4). This is because the phi29 DNApol used in the RCA reaction is capable of efficiently using its 3'-5' exonuclease activity to cleave the 3' end of the nucleic acid that is not hybridized to the padlock probe, and subsequently use its polymerase activity to trigger the amplification using the 3' end of the target nucleic acid as a primer.

In view of the above findings, the padlock of the present invention is further characterized in that the 5' and 3' target-specific hybridization regions may be designed to hybridize in the 3' region of the target nucleic acid. In other words, the first and the second (adjacent) regions of the target are preferably located at the 3' region of target nucleic acid. By "at the 3' region of the target nucleic acid" is referred herein that first and second regions of the target nucleic acid are located towards or in the vicinity of the 3' end of said molecule, i.e., in the terminal 3' region of the target nucleic acid. In an embodiment, the first and second regions of the target nucleic acid are located about 80, 70, 60, 50, 40, 30, 25, 20, 15, 10, 9, 8, 7, 6, 5, 4, 3, 2, or 1 nucleotides from the 3' end of the target nucleic acid.

In an embodiment, the second region of the target nucleic acid is located 100, 90, 80, 70, 60, 50, 40, or 30 nucleotides of distance from the 3' end of the target nucleic acid. In an embodiment, the second region of the target nucleic acid is located 30, 20, 10, 9, 8, 7, 6, 5, 4, 3, 2 or 1 nucleotides of distance from the 3' end of the target nucleic acid. Preferably, the second region of the target nucleic acid (to which the 3' target-specific domain of the padlock probe hybridizes) is located at least 100, 90, 80, 70, 60, preferably 50, 40, 30, 20, 10, or less than 10 nucleotides of distance from the 3' end of the target nucleic acid, so that, when the padlock probe is hybridized, there is a nucleotide region of the target nucleic acid of about 100, 90, 80, 70, 70, 60, preferably 50, 40, 30, 20, 10, or less than 10 nucleotides between the second region of the target nucleic acid and the 3' end of the target nucleic acid. The region of the target nucleic acid between the second region of the target nucleic acid and the 3' end of the target nucleic acid will be preferably exonucleolytically cleaved by action of the 3'-5' exonuclease activity of phi29 DNA pol in step c) of the method, as will be explained below.

In an embodiment, the padlock of the present invention hybridizes in a region of the target nucleic acid that is located in the 3' region of the target, preferably in a region that is at least 80, 70, 60, 50, 40, 30, 20 or 10 nucleotides of distance from the 3' end of the target nucleic acid.

In a most preferred embodiment, the padlock of the present invention is characterized by comprising:
- a 5' target-specific hybridization region that comprises a nucleotide sequence that is at least 85%, 90%, 95%, or 100% complementary to a first region of the target nucleic acid and is capable of hybridizing thereto,
- a 3' target-specific hybridization region that comprises a nucleotide sequence that is at least 85%, 90%, 95%, or 100% complementary to a second region of the target nucleic acid and is capable of hybridizing thereto,
- a backbone nucleotide region located between the 5' target-specific hybridization region and the 3' target-specific hybridization region and that is not complementary to the target, and thus not capable of hybridizing thereto,

wherein each of the 5' and 3' target-specific hybridization region comprises between 10-25, preferably 10-20 nucleotides in length, and
wherein there are no nucleotides (i.e., there are 0 nucleotides) of the target sequence left without base-pairing between the first and second regions of the target nucleic acid sequence which are base paired to the target-specific hybridization regions of the padlock probe, and wherein the target nucleic acid is a single stranded molecule.

In a most preferred embodiment, the padlock of the present invention is characterized by comprising:
- a 5' target-specific hybridization region that is complementary to a first region of the target nucleic acid and is capable of hybridizing thereto,
- a 3' target-specific hybridization region that is complementary to a second region of the target nucleic acid and is capable of hybridizing thereto, and
- a backbone nucleotide region located between the 5' target-specific region and the 3' target-specific region, and that is not complementary to the target nucleic acid and thus not capable of hybridizing thereto,

wherein each of the 5' and 3' target-specific hybridization region comprises between 10-25, preferably 10-20 nucleotides in length,
wherein the first and second regions of the target nucleic acid are separated by 0 nucleotides, and
wherein the first and second regions of the target nucleic acid are located in the 3' region of the target nucleic acid, most preferably located 80, 70, 60, 50, or 40 nucleotides of distance from the 3' end of the target nucleic acid, and wherein the target nucleic acid is a single stranded molecule.

In a most preferred embodiment, the padlock of the present invention is characterized by comprising:
- a 5' target-specific hybridization region that is complementary to a first region of the target nucleic acid and is capable of hybridizing thereto,
- a 3' target-specific hybridization region that is complementary to a second region of the target nucleic acid and is capable of hybridizing thereto, and
- a backbone nucleotide region located between the 5' target-specific region and the 3' target-specific region, and that is not complementary to the target nucleic acid and thus not capable of hybridizing thereto,

wherein each of the 5' and 3' target-specific hybridization region comprises between 10-25, preferably 10-20 nucleotides in length,
wherein the first and second regions of the target nucleic acid are separated by 0 nucleotides,
wherein the padlock hybridizes in the target nucleic acid in a region that is located in the 3' region of the target nucleic acid, preferably in a region that is 80, 70, 60, 50, or 40 nucleotides of distance from the 3' end of the target nucleic acid, and wherein the target nucleic acid is a single stranded molecule.

In an embodiment, the padlock of the present invention comprises, consists, or consists essentially of SEQ ID NO: 7-14, or a sequence with at least 85%, 86%, 87%, 88%, 89%, 90%, 91%, 92%, 93%, 94%, 95%, 96%, 97%, 98%, 99% or 100% sequence identity to SEQ ID NO: 7-14. More preferably, the padlock of the present invention comprises, consists, or consists essentially of SEQ ID NO: 8, 10, 12, or 14 or a sequence with at least 85%, 86%, 87%, 88%, 89%, 90%, 91%, 92%, 93%, 94%, 95%, 96%, 97%, 98%, 99% or 100% sequence identity to SEQ ID NO: 8, 10, 12, or 14, wherein the backbone region, indicated with "N", preferably comprises between 20-160, preferably between 40-150 nucleotides that are not complementary to the target nucleic acid.

In an embodiment, the at least one padlock is a population of padlocks that are identical in sequence, or a population of padlocks that differ in their sequence.

As already mentioned, step a) is a hybridization step and is thus performed at suitable hybridization conditions. Hybridization conditions resulting in particular degrees of stringency will vary depending upon the nature of the hybridization and the composition and length of the hybridizing nucleic acid sequences. The hybridization temperature of the methods described herein can be determined based on various factors, for example, the length of the complementary regions between the capture/detection probe and the target nucleic acid, the composition of the complementary regions (e.g., G/C content), and the stringency needed, which are within the knowledge of those skilled in the art. The hybridization may be performed under a suitable temperature, e.g., a temperature under which the padlock probe and the target nucleic acid hybridise with high specificity, but not with other nucleic acids, even those that share sequence homology with the target nucleic acid. Such a suitable hybridization temperature can be determined based on various factors as known to those skilled in the art.

The method of the present invention has been developed and validated for the detection or ribonucleic acids, such as the genome of a RNA virus as SARS-CoV2. The target nucleic acid may also be the mRNA transcribed from a DNA genome. In any case, the amplification of the padlock is indicative of the presence of the nucleotide sequence of interest. Therefore, preferred targets for this method may include different types of single-stranded RNAs (e.g. mRNA, microRNA, rRNA, snRNA, viral RNA etc.), but the method can be also adapted for detecting single stranded DNA targets, such as the genomes of some animal and plant viruses of the Parvoviridae, Bidnaviridae and Nanoviridae families. Other possible targets are synthetic and/or modified single-stranded nucleic acid molecules, (e.g. including nucleic acid domains comprising or consisting of synthetic or modified nucleotides such as LNA, PNA, morpholino etc.), or fragments thereof. Preferably, the target comprises more than 20, 30, 40, 50, or 60 nucleotides.

As noted above, the preferred target nucleic acid or analyte may be a single-stranded target RNA molecule. The target nucleic acid or analyte may, for example, be an RNA molecule in a pool of RNA or other nucleic acid molecules or nucleotide sequences, for example genomic nucleic acids, whether human or from any source, from a transcriptome, or any other nucleic acid (e.g. organelle nucleic acids, i.e. mitochondrial or plastid nucleic acids), whether naturally occurring or synthetic. The target RNA or analyte may thus be or may be derived from coding (i.e. pre-mRNA or mRNA) or noncoding RNA sequences (such as tRNA, rRNA, snoRNA, miRNA, siRNA, snRNA, exRNA, piRNA and long ncRNA). The target RNA or analyte may typically be an RNA molecule it is desired to detect in a sample, in the sense of being the target of the assay, i.e. an analyte. In one preferred embodiment, the target nucleic acid or analyte is a micro RNA (miRNA). In another preferred embodiment, the target RNA or analyte is 16S RNA, preferably wherein the 16S RNA is from and identificatory of a microorganism (e.g. a pathogenic microorganism) in a sample.

Alternatively, the target RNA or analyte may be genomic RNA, e.g. ssRNA of a virus having RNA as its genetic material. Such viruses include Ebola, HIV, SARS, influenza, hepatitis C, West Nile fever, polio and measles. Accordingly, the target RNA or analyte may be positive sense RNA, negative sense RNA, or positive-sense RNA from a retroviral RNA genome.

The target nucleic acid may be comprised in a sample. The sample may be any sample which contains the nucleotide sequence of interest, and includes both natural and synthetic samples, that is materials which occur naturally or preparations which have been made. All biological and clinical samples are included, e.g. any cell or tissue sample of an organism, or any body fluid or preparation derived therefrom, as well as samples such as cell cultures, cell preparations, cell lysates etc. The samples may be freshly prepared, or they may be prior-treated in any convenient way e.g. for storage.

The sample may be a biological sample, which may contain any viral or cellular material, including all prokaryotic or eukaryotic cells, viruses, bacteriophages, mycoplasmas, protoplasts and organelles. Such biological material may thus comprise all types of mammalian and non-mammalian animal cells. Exemplary biological samples include tissue samples (such as tissue sections and needle biopsies of a tissue); cell samples (e.g., cytological smears (such as Pap or blood smears) or samples of cells obtained by microdissection); samples of whole organisms (such as samples of yeasts or bacteria); or cell fractions, fragments or organelles (such as obtained by lysing cells and separating the components thereof by centrifugation or otherwise). Other examples of biological samples include blood, serum, urine, semen, fecal matter, cerebrospinal fluid, interstitial fluid, mucous, tears, sweat, pus, biopsied tissue (e.g., obtained by a surgical biopsy or needle biopsy), nipple aspirates, milk, vaginal fluid, saliva, swabs (such as buccal swabs), or any material containing biomolecules that is derived from a said biological sample. In some embodiments, the biological sample can be a body fluid, which can be fluid isolated from the body of an individual. For example, "body fluid" may include blood, plasma, serum, mucus, bile, saliva, nasopharyngeal swabs, urine, tears, perspiration, or washings from bodily cavities. Preferably, the sample is a biological sample, preferably saliva. In one particular embodiment the sample comprises microbial cells or viruses which have been isolated from a food or water sample or from a culture of a food or water sample.

The biological sample may be obtained from a subject in need of the analysis. A "subject" may be a human (i.e., male or female of any age group, for example, pediatric subject (e.g., infant, child, or adolescent) or adult subject (e.g., young adult, middle-aged adult, or senior adult). Alternatively, the subject may be a non-human animal. In certain embodiments, the non-human animal is a mammal (e.g., primate, for example, cynomolgus monkey or rhesus monkey), commercially relevant mammal (e.g., cattle, pig, horse, sheep, goat, cat, or dog), or bird (e.g., commercially relevant bird, such as chicken, duck, goose, or turkey). In other examples, the non-human animal is a fish, reptile, or amphibian. The non-human animal may be a transgenic animal or genetically engineered animal. In some examples, the subject may also be a plant.

In one particular embodiment the sample comprises microbial cells or viruses which have been isolated from a clinical sample or from a culture of a clinical sample, such as saliva. In such a sample the target nucleic acid may be a nucleotide sequence present in a microbial cell or virus, e.g. a nucleotide sequence which is characteristic for, or discriminatory or identificatory of a microbial cell or virus, at any level, e.g. at type, group, class, genus, species or strain level. In an embodiment the at least one target nucleic acid is the genome of an infectious agent. By "infectious agent" is meant herein a pathogen that can produce a disease in the subject in need of the analysis, preferably in humans. Preferably, the target nucleic acid is a viral genome, preferably a single-stranded viral genome, preferably RNA single-stranded viral genome. In an embodiment according to the first aspect or any of its embodiments, the viral genome is SARS-CoV2 virus genome.

Preferably, the target nucleic acid is a single-stranded RNA molecule comprising a polyA tail. The polyA tail is a long chain of adenine nucleotides that is added to the 3' end of a RNA molecule to increase its stability. A polyA tail may comprise between between 10 and 250 residues long. Preferably, the target nucleic acid is a single-stranded RNA viral genome comprising a polyA tail, wherein said polyA tail comprises between 10-70 residues, preferably between 20-50 residues. In an embodiment, the target nucleic acid comprises a polyA tail and the first and second regions of said target nucleic acid are located outside said polyA tail, so that the padlock probe does not hybridize within the polyA tail of the target nucleic acid, which will then be exonucleolytically degraded in step c), as explained below. In an embodiment, the single stranded target nucleic acid comprises a polyA tail and the at least one padlock according to the present invention hybridizes adjacent to said polyA tail, preferably at least 0, 1, 2, 3, 4, 5, 6, 7, 8, 9, 10, 15, 20, 25, or 30 nucleotides of distance, in the 5' direction, from the polyA tail.

Step a) of the present method thus comprises or consist of providing at least one padlock of the present invention and contacting a test sample with the at least one padlock under suitable conditions to allow the padlock to hybridise to the first and second regions of the target nucleic acid, if present in said test sample. Hybridization of the padlock to the target nucleic acid results in juxtaposition of the padlock probe 5' and 3' ends of the present invention. However, the circularization of the padlock probe immediately after its hybridization to the target nucleic acid is not complete (there is a nick in the hybridized padlock probe), as the 3' and 5' ends of said padlock are brought together to juxtaposition but need to be ligated in order for the padlock probe to be a completely closed circular molecule. Step b) of the method of the present invention comprises circularising any padlock probe which has hybridised to its target nucleic acid sequence by ligation of its 3' and 5' ends to form a rolling circle amplification template.

Step b) of the method of the present invention comprises or consists of circularising the padlock probe whose 5' and 3' end sequences have hybridized to the target nucleic acid by ligating the ends of said padlock probe.

Step b) comprises at least one ligation reaction. The 5' and 3' ends of the padlock probe may hybridise to a sequence in a target nucleic acid sequence directly adjacent to each other such that they may be directly ligated, thereby to provide a circular, covalently-closed padlock.

In an embodiment, the ligation of the 5' and 3' end of the padlock of the present invention is carried out by action of a ligase. As is known in the art, in template-directed ligation ligases catalyse the formation of a phosphodiester bond between juxtaposed 3'-hydroxyl and 5'- phosphate termini of two immediately adjacent nucleic acids when they are annealed or hybridized to a third nucleic acid sequence to which they are complementary (i.e. a ligation template). Any convenient ligase may be employed, where representative ligases of interest include, but are not limited to: temperature sensitive and thermostable ligases. Temperature sensitive ligases include, but are not limited to, bacteriophage T4 DNA ligase, bacteriophage T7 ligase, and *E. coli* ligase. Thermostable ligases include, but are not limited to, *Taq* ligase, *Tth* ligase, Ampligase^{®} and *Pfu* ligase. Thermostable ligase may be obtained from thermophilic or hyperthermophilic organisms, including but not limited to, prokaryotic, eukaryotic, or archael organisms. Certain RNA ligases may also be employed in the methods of the invention. In a preferred embodiment, the ligase is the DNA Ligase from *Paramecium bursaria* chlorella virus-1 (also known as SplintR ligase), represented by SEQ ID NO: 6. In an embodiment, the ligase is the DNA Ligase from *Paramecium bursaria* chlorella virus-1 comprising or consisting of SEQ ID NO: 6, or a sequence with at least 75%, 80%, 85%, 89%, 90%, 91%, 92%, 95%, 97%, 98%, 99%, or 100% identity to the nucleotide sequence as defined in SEQ ID NOs: 6 and is capable of ligating the 5' and 3' end of the padlock probe once it has hybridized to the target nucleic acid.

A suitable ligase and any reagents that are necessary and/or desirable may be combined with the reaction mixture and maintained under conditions sufficient for ligation of the hybridized oligonucleotides to occur. Ligation reaction conditions are well known to those of skill in the art. During ligation, the reaction mixture may be maintained at a temperature ranging from about 30°C to about 40°C, such as from about 30°C to about 27°C, e.g., at or about 30°C, 35°C, 36°C or 37 °C, for a period of time ranging from about 5 seconds to about 16 hours, such as from about 1 minute to about 1 hour, including from about 2 minutes to about 8 hours. Temperature ranges as discussed immediately above are generally suitable for mesophilic ligases (e.g., T4 ligase or *E. coli* ligase). For more thermostable ligases (e.g. *Tth* ligase, ampligase, *Taq* ligase etc.) higher temperatures may be used, in accordance with protocols and procedures well known in the art. RNase inhibitor may additionally be included if RNA is the target.

It will be evident that the ligation conditions may depend on the ligase enzyme used in the methods of the invention. Hence, the above-described ligation conditions are merely a representative example and the parameters may be varied according to well-known protocols

Only the padlock probes whose 5' and 3' ends have hybridized to the target nucleic acid in step a), and have been ligated in step b) by action of a ligase, preferably SplintR ligase, will become a template for RCA in step c).

Step c) of the method of the present invention comprises or consists of subjecting the circularized padlock probe to rolling circle amplification (RCA), wherein the RCA is carried out by a phi29 DNApol-type enzyme.

Step c) comprises a RCA reaction. Once the ligated circular padlock is formed, it is amplified in order to increase the copy number of the ligation product in a sample, which increases the sensitivity of the detection method of the present invention.

The RCA reaction of step c) is carried out by a bacteriophage phi29 DNA polymerase (also named herein phi29 DNApol-type enzyme or phi29 DNApol). Bacteriophage phi29 DNApol shows unique properties that enable its application in numerous DNA amplification and DNA sequencing technologies and platforms: highly processive DNA synthesis; exceptional strand-displacement, which allows polymerization coupled to the unwinding of double-stranded DNA, in the absence of helicase-type enzymes; high fidelity of synthesis, with very low error insertion rates and efficient proofreading of inserted errors, which collectively enhance fidelity. For the method of the present invention, it is thus required that the phi29 DNApol-type enzyme has at least 3'-5' exonuclease and processive strand-displacement DNA polymerase activity.

By "phi29 DNApol-type" or "DNApol-type enzyme" is understood herein to include the wild-type Phi29 DNApolymerase, represented by SEQ ID NO: 4, but also any variant, chimera, functional equivalent, or truncated version of said polymerase, as long as said variant, chimera, functional equivalent, or truncated version of the phi29 DNApol-type enzyme that maintains its 3'-5' exonuclease, processivity and strand-displacement during DNA polymerization. 3'-5' exonuclease activity is defined as the ability to break the phosphodiester bond at the 3' end and is used for proofreading mispairs (errors). In this method, the 3'-5' exonuclease activity acts first to trim the unpaired 3'-terminal region of the target nucleic acid after hybridization with the padlock, thus creating a valid 3' end (primer) to initiate RCA. Processivity is defined as the ability of DNA polymerase to carry out continuous DNA synthesis on a template DNA without frequent dissociation. The term strand-displacement refers to efficiently performing a 3' end elongation reaction while melting a double-stranded template DNA encountered ahead. Thus, the term "a phi29 DNApol-type" also includes polymerases which have been genetically modified, as well as those which are substantially identical to a naturally-occurring phi29 DNApol-type or a modified polymerase thereof, or to the equivalent enzymes. By substantially identical is meant that the enzyme may contain amino acid substitutions (including one or multiple amino acid exchanges, and/or insertions of one or multiple consecutive amino acids, and/or inversions of consecutive amino acids, and/or deletions of amino acids) which do not affect the above mentioned properties of the enzyme. Depending on the polymerase used, the skilled in the art would know that the reaction conditions for a 3' end elongation reaction may be adequately set.

In a preferred embodiment, the phi29 DNApol-type enzyme is an improved chimeric phi29 DNApol that comprises the entire amino acid sequence of phi29 wildtype of SEQ ID NO: 4, but it further comprises a *Methanopyrus kandleri* Topo V (HhH)₂ domain H (residues 696-751) or H and I (residues 696-802) fused to the C-terminus of phi29 DNApol. This chimeric phi29 DNApol, also named Qualiphi polymerase, presents enhanced DNA binding and thus is a preferred phi29 DNApol to be used in the method of the present invention. The Qualiphi polymerase is represented by SEQ ID NO: 1.

In a further embodiment, the Qualiphi polymerase is further improved by several amino acid substitutions that, as shown in Examples below, make it not only more thermostable but also more efficient. Said amino acid substitutions comprise at least the amino acid substitutions E415X, E416X and E417X, as defined using the amino acid numbering of SEQ ID NO: 1. In the context of the amino acid substitutions disclosed in the present invention, "X" means a positively charged amino acid, i.e., histidine (His, H), lysine (Lys, K), and arginine (Arg, R). Preferably, the amino acid is Lys, so the amino acid substitutions with respect to SEQ ID NO: 1 are E415K, E416K and E417K. The Qualiphi polymerase comprising SEQ ID NO: 1 and at least the amino acid substitutions E415K, E416K and E417K is also called herein Qx3, represented by SEQ ID NO: 3

In a further embodiment, two more amino acid substitutions are introduced over SEQ ID NO: 1, which are E218M and V267L. The Qualiphi polymerase comprising SEQ ID NO: 1 and at least the amino acid substitutions E415K, E416K, E417K, E218M and V267L is also called herein Qx5, represented by SEQ ID NO: 5.

Thus, in an embodiment, the phi29 DNApol used in step c) of the method of the present invention is a phi29 DNApol-type enzyme comprising or consisting of SEQ ID NOs: 1, 3, 4 or 5, or a sequence with at least 75%, 80%, 85%, 89%, 90%, 91%, 92%, 95%, 97%, 98%, 99%, or 100% identity to the nucleotide sequence as defined in SEQ ID NOs: 1, 3, 4, or 5, respectively. Preferably, the phi29 DNApol used in step c) of the method of the present invention is a phi29 DNApol-type enzyme comprising or consisting of SEQ ID NO: 3 or 5. Of note, a certain % of variation over SEQ ID NOs: 1, 3, 4 or 5 are included and allowed in the method of the present invention provided that the variant of the phi29 DNApol maintains or improves the 3'-5' exonuclease, processivity and strand-displacement DNA polymerase activity of the corresponding phi29 of SEQ ID NOs 1, 3, 4 or 5.

In an embodiment, the phi29 DNApol used in step c) of the method of the present invention is a phi29 DNApol-type enzyme comprising or consisting of SEQ ID NO: 1, or a sequence with at least 75%, 80%, 85%, 89%, 90%, 91%, 92%, 95%, 97%, 98%, 99%, or 100% identity to the nucleotide sequence as defined in SEQ ID NOs: 1, wherein said sequence comprises at least the amino acid substitutions E415K, E416K, E417K (Qx3). In an embodiment, the phi29 DNApol used in step c) of the method of the present invention is a phi29 DNApol-type enzyme comprising or consisting of SEQ ID NO: 1, or a sequence with at least 75%, 80%, 85%, 89%, 90%, 91%, 92%, 95%, 97%, 98%, 99%, or 100% identity to the nucleotide sequence as defined in SEQ ID NOs: 1, wherein said sequence further comprises at least the amino acid substitutions E415K, E416K, E417K, E218M and V267L (Qx5). It is noted that, when referred to the amino acid substitutions in the context of a sequence with certain % of identity to SEQ ID NO: 1, it is to be understood that said % of identity allows for modifications of the SEQ ID NO: 1, as long as the specific amino acid substitutions mentioned herein are maintained. As a mode of example, in a preferred embodiment, the phi29 DNApol-type enzyme comprises or consists of a sequence with at least 90% identity to SEQ ID NO 1, with the proviso that the amino acid residues in position number 415, 416, 417, 218 and 267 in SEQ ID NO: 1, correspond to K, K, K, M and L, respectively. It is also noted that here and throughout the whole document, the positions or location of the amino acid residues of a protein or a polypeptide sequence are numbered sequentially starting from the first amino acid residue at the N-terminal end which would then be located at position 1. For example, a protein of 137 amino acids will have those residues numbered 1 (first amino acid residue located in the N-terminal end of the protein) until 137 (the last amino acid residue, located in the C-terminal end of the protein). Thus, the numbering preferably starts from the first amino acid residue at the N-terminal end of the protein or polypeptide and ends at the C-terminal end of the protein or polypeptide. Preferably, the positions of the amino acid residues are numbered using the amino acid sequence of the translated mature protein.

Step c) comprises or consist of performing at least one RCA reaction using as a template the circularized padlock probe and using a bacteriophage phi29 DNApol-type enzyme as defined above. In an embodiment, at least one RCA reaction comprises a first step (c.1) comprising the exonucleolytic cleavage of the 3' end of the target nucleic acid that is not hybridized to the padlock, and a second step (c.2) comprising amplifying the padlock probe to generate the RCP product using the 3' end of the target nucleic acid as a primer, wherein both steps are preferably carried out by a bacteriophage phi29 DNApol-type enzyme. The exonucleolytic step is especially useful when the padlock probe of the present invention has hybridized in the 3' region of the target nucleic acid but there is a portion of the target nucleic acid between the hybridization region of the padlock probe and the 3' end of the target nucleic acid. Said portion of the target nucleic acid, which may be a polyA tail, needs to be removed so that the 3' end of the target nucleic acid is located immediately after the complex formed by the hybridised padlock and the nucleic acid and thus can serve as a primer for the subsequent DNA polymerase activity that carries out the amplification step. Thus, in a preferred embodiment of the method of the present invention, the target nucleic acid contains, or give rise, to the RCA primer. The RCA primer is generated or ("released") by 3' exonucleolytic cleavage of the free 3' end of the target nucleic acid. This may be provided by a phi29 DNApol having 3'-5' exonuclease activity. As shown in the Examples, this step is efficient and allows the resulting 3' end of the target nucleic acid to prime the amplification step of the RCA reaction without the need of primers or RNAse.

In an embodiment, the target nucleic acid is a single-stranded nucleic acid, and step c.1) comprises or consists of the 3' exonucleolytic cleavage by action of a bacteriophage phi29 DNApol-type enzyme of the single-stranded 3' end of the target nucleic acid that is not hybridized to the padlock probe. In other words, step c.1) comprises or consists of the 3' exonucleolytic cleavage by action of a bacteriophage phi29 DNApol-type enzyme of the single-stranded 3' end of the target nucleic acid in the direction to the second region of the target nucleic acid, which is hybridized to the 3' target-specific hybridization region of the padlock. Preferably, the target nucleic acid is a single stranded RNA molecule comprising a polyA tail, wherein the at least one padlock probe hybridizes in the 3' region of the target nucleic acid, preferably adjacent to said polyA tail, and wherein step c.1) comprises the 3' exonucleolytic cleavage of the polyA tail.

In an embodiment, step c.2) comprises or consists of using as a primer the new 3' end of the target nucleic acid created after the exonucleolytic cleavage of step c.1) to amplify via strand-displacement synthesis/RCA the hybridized padlock probe using a phi29 DNApol-type enzyme.

The RCA amplification of step c) comprises at least a linear RCA reaction using the RCA template(s) formed in step (b) to form first rolling circle amplification product(s) (RCP), wherein a first RCP is a concatemer comprising monomers which are complementary to the circularised padlock probe which templated its formation. Suitable conditions to carry out said RCA reaction are known in the art. The RCA product (RCP) may be the product of a primary (i.e. initial) RCA reaction, or it may be the product of a further or later RCA reaction. The second RCA reaction may be a secondary or further, or later, RCA reaction. It will thus be understood that the method of the invention may involve multiple, successive rounds of RCA, e.g. two, three, four or more, wherein in each round a probe is used which is hybridised, directly or indirectly, to the reaction product of a previous round of RCA. Expressed in other words, the method of the invention may comprise repeating steps (a), (b) and (c) one or more times.

The resulting product of step c) is a linear RCP product that is made of concatemers of the complementary sequence of the padlock of the present invention. In some embodiments, the amplification product resulting from step c) is detected in step e). However, in other embodiments, the amplification product of step c) may be used as a template for a further amplification reaction, as it will be explained in step d).

Step d) of the method of the present invention is an optional but preferrable step and comprises or consists of amplifying the product of c) with the primase/polymerase protein (PrimPol) of *Thermus thermophilus* (from herein after also referred to as *Tth*PrimPol).

Step d) comprises a multiple displacement amplification (MDA) reaction, where the product of step c) is exponentially amplified. This further amplification step may also be performed in the absence of exogenous primers or further oligonucleotides that prime the amplification, as the MDA reaction can be primed by the primase/polymerase activity of *Tth*PrimPol. The term "primase/polymerase activity" refers to a DNA-directed primase/polymerase enzyme. The combination of both RCA and MDA steps in the absence of exogenous DNA primers represents a novel hyperbranched RCA reaction (HRCA, also known as strand-displacement cascade reaction) and may be performed subsequent to, or more preferably, simultaneously with step c).

In conventional HRCA, further amplification of the first RCP that is produced in step c) would require the addition of new (complementary) primers for both the RCP strand and its complementary strand. However, the method of the present invention has been optimized as to not to require the addition of any primers (the target itself is the primer for the RCA step), and this is why it is important that the enzyme carrying out step d) is *Tth*PrimPol or a functional equivalent. This protein has primase/polymerase activity, and thus is capable of creating the necessary DNA primers to feed the MDA second step that increases the yield of this HRCA method. The *Tth*PrimPol is defined in detail in EP2971080B1. As shown in Fig 6 the efficiency and specificity of the padlock amplification was highly increased when step d) of the method of the present invention was introduced, i.e., when a MDA reaction was primed by *Tth*PrimPol, thus avoiding the need to add specific (or unspecific) primers. Thus, the HRCA method of the present invention may be preferably carried out in a primer-free environment.

The MDA reaction of step d) may be further subdivided in two steps, namely, d.1) priming on the linear RCP template by *Tth*PrimPol and elongation of these resulting primers by a phi29 DNApol-type polymerase, and d.2) further events of DNA priming and elongation occurring on the multi-branched displaced strands resulting from d.1). As used herein, the term "DNA priming" refers to the generation of oligonucleotide DNA primers on a polynucleotide template by a PrimPol-type enzyme.

The DNA primase activity of *Tth*PrimPol can be enhanced on templates with "optimal priming sequences" which are recognized by *Tth*PrimPol and that are used to generate DNA primers. By "optimal priming sequences" or "optimal priming sites" are referred herein as the sequences used by the *Tth*PrimPol to efficiently create an oligonucleotide primer. Preferably, optimal priming sequences are "5'-CCTC-3"' and/or "5'-CTC-3"' sequences, although other sequences may be less efficiently used. For step d) to be carried in the absence of additional primers, it is advantageous that the *Tth*PrimPol finds at least one, preferably several, optimal priming sequences found in a ssDNA template (the RCP) so that the primase activity of *Tth*PrimPol is activated and the HRCA reaction of step d) starts.

The fact that the *Tth*PrimPol has a preference for particular priming sequences in the template to carry out its primase activity led the authors of the present invention to introduce a further optimization of the method. This further optimization comprises specifically designing the sequence of the padlocks of the present invention, so that they comprise the complementary sequence of the "optimal priming sequences" that will appear on the RCP. This would lead to the presence of optimal priming sequences for *Tth*PrimPol in the RCP product generated during step c) and in the multi-branched products of step d.2. Thus, by simply introducing, preferably in the backbone sequence (i.e, the sequence that does not hybridize to the target) of the padlock of the present invention, one or more sequences "'5'-GAGG-3'" and/or "5'-GAG-3"', the efficiency of the amplification is improved.

Alternatively, or additionally to introducing in the sequence of the padlock one or more complementary sequences of the optimal priming sequences used by *Tth*PrimPol, a further optimization of the method consisted in minimizing the presence of optimal priming sites or sequences in the sequence of the padlock probe. By "minimizing the presence of optimal priming sites in the sequence of the padlock" is preferably referred herein to reducing as much as much as possible, preferably reducing to zero, the number of optimal priming sites present in the padlock. This would avoid spurious priming during the RCA step, likely reducing untargeted amplification of the padlock. This off-target amplification was likely occurring in the original padlock, as it contained the optimal priming sequences 5'-CCTC-3‴ and/or "5'-CTC-3"' in its nucleotide sequence. Thus, the initial results were further optimized by removing from the nucleotide sequence of the original padlock probe the optimal priming sites for *Tth*PrimPol. This further optimization step can be performed by designing a padlock probe that has less than 5, 4, 3, 2, 1, or preferably totally lacks, the optimal priming sequences "5'-CCTC-3"' and/or "5'-CTC-3"'. Figure 10B (lower panel) shows the results of the above mentioned optimization methods to decrease the off-target amplification of the linear padlock by *Tth*PrimPol, and to promote that the primase activity of *Tth*PrimPol that preferably works on the concatemer product (RCP) of step c). Preferably, the optimization of the padlock sequence to lack optimal priming sites for *Tth*PrimPol or to comprise one or more sequences "'5'-GAGG-3"' and/or "5'-GAG-3' is performed in the backbone sequence of the padlock (i.e., outside the target-specific hybridization regions of the padlock). This is because it may happen that the target-specific hybridization regions, due to the fact that they need to be complementary to the target nucleic acid, may have a specific sequence that cannot be edited without comprosiming the ability of the padlock to hybridize to the target nucleic acid..

Thus, in an embodiment, the at least one padlock of the present invention is further characterized by not having in its nucleotide sequence, preferably by not having in the backbone sequence of the padlock, any of the optimal priming sites recognized by *Tth*PrimPol. In a further embodiment, the at least one padlock of the present invention is also characterized by having in its sequence, preferably in the backbone sequence of the padlock, one or more of the complementary sequences of said optimal priming sites. In a preferred embodiment, at least one padlock of the present invention is characterized by:
i. not having in its nucleotide sequence, preferably in the backbone sequence of the padlock, the sequences "5'-CCTC-3"' and/or "5'-CTC-3"', and/or
ii. having in its nucleotide sequence, preferably in the backbone sequence of the padlock, one or more sequences "5'-GAGG-3"' and/or "5'-GAG-3"'.

In view of the above, in a most preferred embodiment, the padlock of the present invention is characterized by comprising:
- a 5' target-specific hybridization region that is complementary to a first region of the target nucleic acid and is capable of hybridizing thereto,
- a 3' target-specific hybridization region that is complementary to a second region of the target nucleic acid and is capable of hybridizing thereto, and
- a backbone nucleotide region located between the 5' target-specific region and the 3' target-specific region, and that is not complementary to the target nucleic acid and thus not capable of hybridizing thereto,

wherein the first and second regions of the target nucleic acid are separated by 0 nucleotides,
wherein the nucleotide sequence of the at least one padlock probe, preferably the backbone region of the padlock, is characterized by not presenting any of the optimal priming sites recognised by *Tth*PrimPol and/or wherein the nucleotide sequence of the at least one padlock probe, preferably the backbone region of the padlock, is characterized by having one or more of the complementary sequence of said optimal priming sites, wherein the optimal priming sites are "5'-CCTC-3"' and/or "5'-CTC-3"' sequences, and wherein the padlock probe hybridizes at the 3' region of the target nucleic acid, preferably 80, 70, 60, 50, or 40 nucleotides of distance from the 3' end of the target nucleic acid.

In a preferred embodiment, the optimization of the sequence of the padlock probe is carried out in the backbone region of the padlock probe.

In a preferred embodiment, the method of the present invention is a primer-free or primerless amplification and detection method, comprising cooperative synthesis of DNA by both of the two polymerases provided for the amplification reaction (a bacteriophage phi29 DNApol-type enzyme in step c) and *Tth*PrimPol in step d)). As used herein, a "primer-free method" or a "primerless method" is referred to a preferred embodiment of the method of the present invention in which it is carried out in the absence of any added primers, wherein "primer" is understood as a nucleic acid sequence that is used as a starting point for DNA synthesis. This means that the RCA/HRCA method can be carried out without the addition of oligonucleotides that prime the amplification. Preferably, the HRCA method defined herein is carried out in the absence of exogenous primers or further oligonucleotides that prime the amplification, wherein the target nucleic acid, preferably target RNA, is used as a primer for the RCA reaction of step c), and wherein the *Tth*PrimPol creates new primers for the MDA reaction of step d).

In an embodiment, the method of the present invention, especially when using padlocks with optimal priming sites on the RCP strand, comprises adding at least a specific primer for the RCP complementary strand, thus boosting priming on the secondary branches produced during step d) (MDA reaction). Other primers complementary to the RCP or padlock may also be added.

In a preferred embodiment, the primase/polymerase of *Thermus thermophilus* (*Tth*PrimPol) is the primase/polymerase of the thermophile bacterial strain *Thermus thermophilus* HB27, preferably the protein represented by SEQ ID NO: 2. In an embodiment, the primase/polymerase protein of the *Thermus thermophilus* bacterial strain used in step d) of the method of the present invention is a protein comprising or consisting of SEQ ID NO: 2, or a sequence with at least 75%, 80%, 85%, 89%, 90%, 91%, 92%, 95%, 97%, 98%, 99%, or 100% identity to the nucleotide sequence as defined in SEQ ID NOs: 2, wherein said protein comprises polymerase and primase activity.

Thus, in the methods of the present invention, the RCA reaction of step c) may be followed by the MDA reaction of step d), thus becoming a HRCA reaction which takes place prior to detection step e).

Step e) of the method of the present invention comprises or consists of detecting the amplified product.

Step e) comprises the detection of the amplified product. It is noted that the molecule that is detected is the amplified DNA product of steps c) or of step d), and not the target nucleic acid (e.g. RNA). However, the detection of the amplified DNA products indicates that the target nucleic acid of interest is present in the sample. This step may be performed subsequently to steps c) or d), or at the same time, as the amplified products are synthesized. Steps a) to e) are preferably carried out for a suitable amount of time until a detectable signal is obtained.

The term "detecting" is used broadly herein to include any means of determining the presence or absence of the target nucleic acid (i.e. if it is present or not) or any form of measurement. Methods for detection of nucleic acids are not limited and known in the art. In an embodiment, the detection may include the use of intercalating agents that label the amplified product and release a detectable signal. Detectable said signal may be a fluorogenic or colorimetric signal. In a further embodiment, the detecting step uses a sequence-specific detection method, such as a labelled probe that binds to the amplified product and emits a detectable signal, or the use of Crispr/Cas system.

In some embodiments, the label is a directly detectable label, where directly detectable labels of interest include, but are not limited to: fluorescent labels, radioisotopic labels, chemiluminescent labels, and the like. In many embodiments, the label is a fluorescent label, where the labelling reagent employed in such embodiments is a fluorescently tagged nucleotide(s), e.g. fluorescently tagged CTP (such as Cy3-CTP, Cy5-CTP) etc. Fluorescent moieties which may be used to tag nucleotides for producing labelled probe nucleic acids (i.e. detection probes) include, but are not limited to: fluorescein, the cyanine dyes, such as Cy3, Cy5, Alexa 555, Bodipy 630/650, and the like. Other labels, such as those described above, may also be employed as are known in the art.

In an embodiment, the detection step uses a labelled nucleic acid that bind to the complementary sequence of the detectable sequence and emits a detectable signal. In certain embodiments, the specifically labelled nucleic acids (detection probes) are labelled with "energy transfer" labels. As used herein, "energy transfer" refers to the process by which the fluorescence emission of a fluorescent group is altered by a fluorescence-modifying group. Energy transfer labels are well known in the art, and such labelled oligonucleotide probes include the TaqMan^{®} type probes, Scorpion probes, Sunrise probes, and conformationally assisted probes, among others.

The detection step may include the measurement of the signal released from the detection of the amplified padlock and inferring the presence of the target nucleic acid in the sample based on the intensity of the signal. The detection step may include a quantification step in which the levels (concentration) of the target nucleic acid are obtained. To determine the concentration of a target nucleic acid in a sample from the signal of the amplified padlock, a calibration curve may be developed using HRCA reactions with known concentrations of the target nucleic acid molecule. The concentration of the target nucleic acid in a sample may be determined by comparison of a measured parameter to a calibration standard. In some cases, a calibration curve may be prepared, wherein the total measured signal is determined for a plurality of samples comprising the target nucleic acid at a known concentration using a substantially similar assay format. For example, the total intensity of the array may be compared to a calibration curve to determine a measure of the concentration of the target nucleic acid in the sample. The calibration curve may be produced by completing the method with a plurality of standardized samples of known concentration under similar conditions used to analyse test samples with unknown concentrations. A calibration curve may be used to relate the detected signal of the amplified padlock with a known concentration of the target nucleic acid. The assay may then be completed on a sample containing the target nucleic acid or fragment in an unknown concentration, and the signals detected may be compared to the calibration curve, (or a mathematical equation fitting the same) to determine a measure of the concentration of the target nucleic acid in the sample.

The method described herein can also be used to detect several targets present in a sample or measure the levels of two or more targets (multiplex assay) of a sample. In this case, the two or more target nucleic acids may then be detected using different padlock probes. In an embodiment, two or more padlocks are used, wherein each of the padlock used is capable of hybridising to a different nucleic acid of interest. In other words, each of the padlock probes have different target-specific hybridization regions at its 5' and 3' regions which direct it to hybridise (bind) to a different target nucleic acid sequence present in the sample (thus to indicate the presence thereof). The use of two or more padlock probes in a multiplexed detection method of the present invention accordingly allows the detection of two or more different target nucleic acid in a sample. As each target nucleic acid is different, each padlock probe may thereby be considered to be 'for' detecting that target nucleic acid, i.e. the analyte for which the target nucleic acid is representative.

In other embodiments, a single nucleic acid of interest may be represented by two or more different target nucleic acid sequences, such that detection of any one or more of the target nucleic acid sequences that are representative of that target nucleic acid will indicate the presence of that nucleic acid of interest in the sample.

In a multiplexed reaction, for example, in a sample suspected of containing three different target nucleic acids, the detection step may comprise (i) measuring a first signal released from a first amplification product derived of the amplification of a first padlock probe and determining the presence or a level of a first target nucleic acid in the sample based on the intensity of the first signal; (ii) measuring a second signal released from a second amplification product derived from the amplification of a second padlock probe and determining the presence or a level of the second target nucleic acid in the sample based on the intensity of the second signal; and (iii) measuring a third signal released from a third amplification product derived from the amplification of a third padlock probe and determining the presence or a level of the third target nucleic acid in the sample based on the intensity of the second signal.

### Other Improvements

The method of the present invention as defined above already provides a highly specific, sensitive method that has been optimized to detect single-stranded ribonucleic acid targets while avoiding amplification of double-stranded nucleic acids that could be present in crude samples in addition to the target, and that also require less steps and reagents, and whose great specificity relies in not adding primers. As it will be obvious to the skilled person in the art, the method of the present invention is carried out providing nucleotides and/or nucleotide analogues for incorporation in a complementary strand of nucleic acid, providing a suitable buffer to carry out the HRCA and the detection steps, and to keep the stability of the above mentioned materials for a suitable period of time.

The author of the present invention further investigated the optimum conditions in which the reagents of the present invention work at their best. Said optimum conditions are explained below.

It was found herein that starting alkaline conditions favour amplification detection, as shown in Fig. 1. Thus, in a preferred embodiment, the method is performed at alkaline pH, preferably at pH ranging from 8-9, most preferably of 8.8.

In an embodiment, the method of the invention is an isothermal method, so that all the reactions described herein can potentially be achieved at a single temperature. Figure 2 shows that the Qx3 and Qx5 polymerases were able to work at a temperature range of 30-39°C. Thus, in a preferred embodiment, the isothermal method is performed isothermally at about between 30°C and 45°C, more preferably between 30°C and 40°C, most preferably between 30°C and 37°C. Preferably, the temperature is 30°C or 37°C. Preferably, the method of the present invention is carried out at temperature is 37°C and at a pH of 8.8.

In an attempt to improve the specificity of the amplification reaction when using the additional MDA step with *Tth*PrimPol, the effect of increasing the concentration of Mg²⁺ ions on the amplification outcome was studied. Fig 7 shows that using 25 mM Mg²⁺ ions, preferably MgCl₂ in combination with *Tth*PrimPol extremely improved both, the sensitivity and the specificity of the detection procedure. Thus, in a preferred embodiment, the method is carried out in the presence of at least 15, 20, preferably 25, 30, or 35 mM Mg²⁺, preferably MgCl₂. The presence of these amounts of MgCl₂ also had a positive effect on the specific detection of the virus in crude patient samples, see Fig. 7.

The authors also investigated the effect of Mn²⁺ ions in the priming function of the *Tth*PrimPol. As shown in Figure 11a, under both experimental conditions, the extra addition of 0.5 mM MnCl₂, on the one hand allowed for a 25% increase in the yield of the amplification product, increasing the sensitivity of the detection, and on the other hand reduced the off-target amplification, increasing the specificity of the detection method. Thus, in a preferred embodiment, the method is carried out in the presence of at least 0.1, 0.25, preferably 0.5, 0.75, 1, 1.5, or 2 mM Mn²⁺, preferably MnCl₂.

In a most preferred embodiment, the method of the present invention includes steps a) to e) as defined above, wherein the media conditions are (see Fig. 11b):
- 0.1, 0.25, preferably 0.5, 0.75, 1, 1.5, or 2 mM Mn²⁺, preferably MnCl₂,
- 15, 20, preferably 25, 30, or 35 mM Mg²⁺, preferably MgCl₂
- alkaline pH, preferably between 8-9, most preferably 8.8,
- isothermal temperature of between 35-40°C, preferably 37 °C.

In an embodiment, the method is performed in a one-pot reaction. By "one-pot reaction" is meant that all the required reagents and buffers are mixed in a vessel at the beginning and then allowed to react, preferably under isothermal conditions. Consequently, one-pot reactions do not require the isolation of intermediates, which saves material, time, and energy.

Each embodiment disclosed herein is contemplated as being applicable to each of the other disclosed embodiments. Thus, all combinations of the various elements described herein are within the scope of the invention. It should also be understood that, unless clearly indicated to the contrary, in any methods claimed herein that include more than one step or act, the order of the steps or acts of the method is not necessarily limited to the order in which the steps or acts of the method are recited. Optionally, further steps may be included in between, before, or after the steps described above. Such further steps may be, for instance, one or more washing or incubating steps. Further, preferably, some of the reagents and buffers used in each of the step, for instance, the deoxynucleotide triphosphates in the RCA and HRCA amplification, may also used in the hybridization step of the method, thereby simplifying the method without compromising the efficiency while saving reagents and time.

Preferred embodiments of the method of the present invention include:
In an embodiment, the method of the present invention comprises the steps of:
a) contacting a target nucleic acid with at least one padlock probe, wherein the at least one padlock probe comprises a 5' target-specific hybridization region that is complementary to a first region of the target nucleic acid and a 3' target-specific hybridization region that is complementary to a second region of the target nucleic acid,
   wherein the first and the second regions are located adjacent to each other and located in the 3' region of the target nucleic acid,
b) circularising the padlock probe whose 5' and 3' end sequences have hybridized to the target nucleic acid by ligating the ends of said padlock probe,
c) subjecting the circularized padlock probe to rolling circle amplification (RCA), wherein the RCA is carried out by a bacteriophage phi29 DNApol-type enzyme,
d) during and/or after step c) amplifying the product of c) with the primase/polymerase protein of *Thermus thermophilus* (*Tth*PrimPol), and
e) detecting the amplified product,
wherein the target nucleic acid is a single stranded target nucleic acid, preferably a single-stranded RNA molecule.

Preferably, the method of the present invention comprises the steps of:
a) contacting a target nucleic acid with at least one padlock probe, wherein the padlock probe comprises:
   - a 5' target-specific hybridization region that is complementary to a first region of the target nucleic acid and is capable of hybridizing thereto,
   - a 3' target-specific hybridization region that is complementary to a second region of the target nucleic acid and is capable of hybridizing thereto, and
   - a backbone nucleotide region located between the 5' target-specific region and the 3' target-specific region, and that is not complementary to the target nucleic acid and thus not capable of hybridizing thereto,

   wherein the first and second regions of the target nucleic acid are separated by 0 nucleotides, and
   wherein the first and second regions are located in the 3' region of the target nucleic acid,
b) circularising the padlock probe whose 5' and 3' end sequences have hybridized to the 3' region of the target nucleic acid by ligating the ends of said padlock probe, preferably using the SplintR ligase,
c) subjecting the circularized padlock probe to rolling circle amplification (RCA), wherein the RCA is carried out by a phi29 DNApol-type enzyme, wherein said phi29 DNApol-type enzyme has 3'-5' exonuclease and strand-displacement DNA polymerase activity,
d) during and/or after step c) amplifying the product of c) with the primase/polymerase protein of *Thermus thermophilus* (*Tth*PrimPol), and
e) detecting the amplified product,
wherein the target nucleic acid is a single stranded target nucleic acid, preferably a single-stranded RNA molecule.

Preferably, the method of the present invention comprises the steps of:
a) contacting a target nucleic acid with at least one padlock probe, wherein the padlock probe comprises:
   - a 5' target-specific hybridization region that is complementary to a first region of the target nucleic acid and is capable of hybridizing thereto,
   - a 3' target-specific hybridization region that is complementary to a second region of the target nucleic acid and is capable of hybridizing thereto, and
   - a backbone nucleotide region located between the 5' target-specific region and the 3' target-specific region, and that is not complementary to the target nucleic acid and thus not capable of hybridizing thereto,
   wherein the first and second regions of the target nucleic acid are separated by 0 nucleotides, and wherein the first and second regions of the target nucleic acid are located in the 3' region of the target nucleic, preferably located 80, 70, 60, 50, or 40 nucleotides of distance from the 3' end of the target nucleic acid,
b) circularising the padlock probe whose 5' and 3' end sequences have hybridized to the 3' region of the target nucleic acid by ligating the ends of said padlock probe, preferably using the SplintR ligase
c) subjecting the circularized padlock probe to rolling circle amplification (RCA), wherein the RCA is carried out by a bacteriophage phi29 DNApol-type enzyme, wherein said bacteriophage phi29 DNApol-type enzyme has 3'-5' exonuclease and strand-displacement DNA polymerase activity, and wherein step c) comprises the steps of:
   c.1) the 3' exonucleolytic cleavage by action of a phi29 DNApol-type enzyme of the 3' end of the target nucleic acid that is not hybridized to the padlock probe, and
   c.2) using as a primer the new 3' end of the target nucleic acid created after c.1) to amplify the hybridized padlock probe using the strand-displacement DNA polymerase activity of a phi29 DNApol-type enzyme,
d) during and/or after step c) amplifying the product of c) with the primase/polymerase protein of *Thermus thermophilus* (*Tth*PrimPol), and
e) detecting the amplified product,
wherein the target nucleic acid is a single stranded target nucleic acid, preferably a single-stranded RNA molecule.

Preferably, the method of the present invention comprises the steps of:
a) contacting a target nucleic acid with at least one padlock probe, wherein the padlock probe comprises:
   - a 5' target-specific hybridization region that is complementary to a first region of the target nucleic acid and is capable of hybridizing thereto,
   - a 3' target-specific hybridization region that is complementary to a second region of the target nucleic acid and is capable of hybridizing thereto, and
   - a backbone nucleotide region located between the 5' target-specific region and the 3' target-specific region, and that is not complementary to the target nucleic acid and thus not capable of hybridizing thereto,

   wherein the first and second regions of the target nucleic acid are separated by 0 nucleotides, and
   wherein the first and second regions of the target nucleic acid are located in the 3' region of the target nucleic acid, preferably located 80, 70, 60, 50, or 40 nucleotides of distance from the 3' end of the target nucleic acid,
b) circularising the padlock probe whose 5' and 3' end sequences have hybridized to the target nucleic acid by ligating the ends of said padlock probe, preferably using the SplintR ligase,
c) subjecting the circularized padlock probe to rolling circle amplification (RCA), wherein the RCA is carried out by a phi29 DNApol-type enzyme, wherein said phi29 DNApol-type enzyme has 3'-5' exonuclease and strand-displacement DNA polymerase activity, and wherein step c) comprises the steps of:
   c.1) the 3' exonucleolytic cleavage by action of a phi29 DNApol-type enzyme of the 3' end of the target nucleic acid in the direction to the second region of the target nucleic acid, and
   c.2) using as a primer the new 3' end of the target nucleic acid created after c.1) to amplify the hybridized padlock probe using the strand-displacement DNA polymerase activity of a phi29 DNApol-type enzyme,
   wherein the phi29 DNApol-type enzyme comprises or consists of SEQ ID NO: 1, or a sequence with at least 75%, 80%, 85%, 89%, 90%, 91%, 92%, 95%, 97%, 98%, 99%, or 100% identity to the nucleotide sequence as defined in SEQ ID NOs: 1, wherein said sequence comprises at least the amino acid substitutions E415K, E416K, E417K,
d) during and/or after step c) amplifying the product of c) with the primase/polymerase protein of *Thermus thermophilus* (*Tth*PrimPol), and
e) detecting the amplified product,
wherein the target nucleic acid is a single stranded target nucleic acid, preferably a single-stranded RNA molecule.

Preferably, the method of the present invention is a primer-free method, and it comprises the steps of:
a) contacting a target nucleic acid with at least one padlock probe, wherein the padlock probe comprises:
   - a 5' target-specific hybridization region that is complementary to a first region of the target nucleic acid and is capable of hybridizing thereto,
   - a 3' target-specific hybridization region that is complementary to a second region of the target nucleic acid and is capable of hybridizing thereto, and
   - a backbone nucleotide region located between the 5' target-specific region and the 3' target-specific region, and that is not complementary to the target nucleic acid and thus not capable of hybridizing thereto,

   wherein each of the 5' and 3' target-specific hybridization region comprises between 10-25, preferably 10-20 nucleotides in length,
   wherein the first and second regions are separated by 0 nucleotides, and are located in the 3' region of the target nucleic, preferably located 80, 70, 60, 50, or 40 nucleotides of distance from its 3' end,
b) circularising the padlock probe whose 5' and 3' end sequences have hybridized to the target nucleic acid by ligating the ends of said padlock probe, preferably using the SplintR ligase,
c) subjecting the circularized padlock probe to rolling circle amplification (RCA), wherein the RCA is carried out by a phi29 DNApol-type enzyme, wherein said phi29 DNApol-type enzyme has 3'-5' exonuclease and strand-displacement DNA polymerase activity, and wherein step c) comprises the steps of:
   c.1) the 3' exonucleolytic cleavage by action of a phi29 DNApol-type enzyme of the 3' end of the target nucleic acid in the direction to the second region of the target nucleic acid, and
   c.2) using as a primer the new 3' end of the target nucleic acid created after c.1) to amplify the hybridized padlock probe using the strand-displacement DNA polymerase activity of a phi29 DNApol-type enzyme,
   wherein a phi29 DNApol-type enzyme comprises or consists of SEQ ID NO: 1, or a sequence with at least 75%, 80%, 85%, 89%, 90%, 91%, 92%, 95%, 97%, 98%, 99%, or 100% identity to the nucleotide sequence as defined in SEQ ID NOs: 1, wherein said sequence comprises at least the amino acid substitutions E415K, E416K, E417K,
d) during and/or after step c) amplifying the product of c) with the primase/polymerase of *Thermus thermophilus* (TthPrimPol), wherein the primase/polymerase protein *Tth*PrimPol comprises or consists of SEQ ID NO: 2, or a sequence with at least 75%, 80%, 85%, 89%, 90%, 91%, 92%, 95%, 97%, 98%, 99%, or 100% identity to the nucleotide sequence as defined in SEQ ID NOs: 2, and
e) detecting the amplified product,
wherein the target nucleic acid is a single stranded target nucleic acid, preferably a single-stranded RNA molecule.

Preferably, the method of the present invention is a primer-free method, and it comprises the steps of:
a) contacting a target nucleic acid with at least one padlock probe, wherein the padlock probe comprises:
   - a 5' target-specific hybridization region that is complementary to a first region of the target nucleic acid and is capable of hybridizing thereto,
   - a 3' target-specific hybridization region that is complementary to a second region of the target nucleic acid and is capable of hybridizing thereto, and
   - a backbone nucleotide region located between the 5' target-specific region and the 3' target-specific region, and that is not complementary to the target nucleic acid and thus not capable of hybridizing thereto,

   wherein each of the 5' and 3' target-specific hybridization region comprise between 10-25, preferably 10-20 nucleotides in length,
   wherein the first and second regions of the target nucleic acid are separated by 0 nucleotides,
   wherein the first and second regions of the target nucleic acid are located 80, 70, 60, 50, or 40 nucleotides of distance from the 3' end of the target nucleic acid,
   wherein the nucleotide sequence of the at least one padlock probe is characterized by not presenting any of the optimal priming sites recognised by the primase/polymerase protein of *Thermus thermophilus* (*Tth*PrimPol), and/or wherein the nucleotide sequence of the at least one padlock probe is characterized by having one or more of the complementary sequence of said priming sites, wherein the priming sites are "5'-CCTC-3"' and/or "5'-CTC-3"' sequences, and minimizing the presence of these priming sites in the sequence of the padlock,
b) circularising the padlock probe whose 5' and 3' end sequences have hybridized to the target nucleic acid by ligating the ends of said padlock probe, preferably using the SplintR ligase,
c) subjecting the circularized padlock probe to rolling circle amplification (RCA), wherein the RCA is carried out by a phi29 DNApol-type enzyme, wherein said phi29 DNApol-type enzyme has 3'-5' exonuclease and strand-displacement DNA polymerase activity, and wherein step c) comprises the steps of:
   c.1) the 3' exonucleolytic cleavage by action of a phi29 DNApol-type enzyme of the 3' end of the target nucleic acid in the direction to the second region of the target nucleic acid, and
   c.2) using as a primer the new 3' end of the target nucleic acid created after c.1) to amplify the hybridized padlock probe using the strand-displacement DNA polymerase activity of a phi29 DNApol-type enzyme,
   wherein the bacteriophage phi29 DNApol-type enzyme comprises or consists of SEQ ID NO: 1, or a sequence with at least 75%, 80%, 85%, 89%, 90%, 91%, 92%, 95%, 97%, 98%, 99%, or 100% identity to the nucleotide sequence as defined in SEQ ID NOs: 1, wherein said sequence comprises at least the amino acid substitutions E415K, E416K, E417K, E218M and V267L (Qx5),
d) during and/or after step c) amplifying the product of c) with the primase/polymerase of *Thermus thermophilus* (*Tth*PrimPol), wherein the *Tth*PrimPol protein comprises or consists of SEQ ID NO: 2, or a sequence with at least 75%, 80%, 85%, 89%, 90%, 91%, 92%, 95%, 97%, 98%, 99%, or 100% identity to the nucleotide sequence as defined in SEQ ID NOs: 2, and
e) detecting the amplified product,
wherein the target nucleic acid is a single stranded target nucleic acid, preferably a single-stranded RNA molecule.

Preferably, the method of the present invention comprises the steps of:
a) contacting a target nucleic acid with at least one padlock probe, wherein the padlock probe comprises:
   - a 5' target-specific hybridization region that is complementary to a first region of the target nucleic acid and is capable of hybridizing thereto,
   - a 3' target-specific hybridization region that is complementary to a second region of the target nucleic acid and is capable of hybridizing thereto, and
   - a backbone nucleotide region located between the 5' target-specific region and the 3' target-specific region, and that is not complementary to the target nucleic acid and thus not capable of hybridizing thereto,

   wherein each of the 5' and 3' target-specific hybridization region comprises between 10-25, preferably 10-20 nucleotides in length,
   wherein the first and second regions of the target nucleic acid are separated by 0 nucleotides,
   wherein the first and second regions of the target nucleic acid are located 80, 70, 60, 50, or 40 nucleotides of distance from the 3' end of the target nucleic acid,
   wherein the nucleotide sequence of the at least one padlock probe, preferably the backbone region of said padlock probe, is characterized by not presenting any of the optimal priming sites recognised by the primase/polymerase of *Thermus thermophilus* (*Tth*PrimPol), and/or wherein the nucleotide sequence of the at least one padlock probe, preferably the backbone region of said padlock probe, is characterized by having one or more complementary sequence of said priming sites, wherein the priming sites are "5'-CCTC-3"' and/or "5'-CTC-3"' sequences,
b) circularising the padlock probe whose 5' and 3' end sequences have hybridized to the target nucleic acid by ligating the ends of said padlock probe, preferably using the SplintR ligase,
c) subjecting the circularized padlock probe to rolling circle amplification (RCA), wherein the RCA is carried out by a phi29 DNApol-type enzyme, wherein said phi29 DNApol-type enzyme has 3'-5' exonuclease and strand-displacement DNA polymerase activity, and wherein step c) comprises the steps of:
   c.1) the 3' exonucleolytic cleavage by action of a phi29 DNApol-type enzyme of the 3' end of the target nucleic acid in the direction to the second region of the target nucleic acid, and
   c.2) using as a primer the new 3' end of the target nucleic acid created after c.1) to amplify the hybridized padlock probe using the strand-displacement DNA polymerase activity of a phi29 DNApol-type enzyme ,
   wherein a phi29 DNApol-type enzyme comprises or consists of SEQ ID NO: 1, or a sequence with at least 75%, 80%, 85%, 89%, 90%, 91%, 92%, 95%, 97%, 98%, 99%, or 100% identity to the nucleotide sequence as defined in SEQ ID NOs: 1, wherein said sequence comprises at least the amino acid substitutions E415K, E416K, E417K, E218M and V267L,
d) during and/or after step c) amplifying the product of c) with the primase/polymerase protein of *Thermus thermophilus* (*Tth*PrimPol), wherein the *Tth*PrimPol protein comprises or consists of SEQ ID NO: 2, or a sequence with at least 75%, 80%, 85%, 89%, 90%, 91%, 92%, 95%, 97%, 98%, 99%, or 100% identity to the nucleotide sequence as defined in SEQ ID NOs: 2, and
e) detecting the amplified product,
wherein the target nucleic acid is a single stranded target nucleic acid, preferably a single-stranded RNA molecule.

Preferably, the method of the present invention comprises the steps of:
a) contacting a target nucleic acid with at least one padlock probe, wherein the padlock probe comprises:
   - a 5' target-specific hybridization region that is complementary to a first region of the target nucleic acid and is capable of hybridizing thereto,
   - a 3' target-specific hybridization region that is complementary to a second region of the target nucleic acid and is capable of hybridizing thereto, and
   - a backbone nucleotide region located between the 5' target-specific region and the 3' target-specific region, and that is not complementary to the target nucleic acid and thus not capable of hybridizing thereto,

   wherein each of the 5' and 3' target-specific hybridization region comprises between 10-25, preferably 10-20 nucleotides in length,
   wherein the first and second regions of the target nucleic acid are separated by 0 nucleotides,
   wherein the first and second regions of the target nucleic acid are located 80, 70, 60, 50, or 40 nucleotides of distance from its 3' end,
   wherein the nucleotide sequence of the at least one padlock probe, preferably the backbone region of said padlock probe, is characterized by not presenting any of the optimal priming sites recognised by the primase/polymerase of *Thermus thermophilus* (*Tth*PrimPol), and/or wherein the nucleotide sequence of the at least one padlock, preferably the backbone region of said padlock probe, probe is optimized by having one or more complementary sequence of said priming sites, wherein the priming sites are "5'-CCTC-3"' and/or "5'-CTC-3"' sequences, but lacking these sites in the sequence of the padlock,
b) circularising the padlock probe whose 5' and 3' end sequences have hybridized to the target nucleic acid by ligating the ends of said padlock probe, preferably using the SplintR ligase,
c) subjecting the circularized padlock probe to rolling circle amplification (RCA), wherein the RCA is carried out by a phi29 DNApol-type enzyme, wherein said phi29 DNApol-type enzyme has 3'-5' exonuclease and strand-displacement DNA polymerase activity, and wherein step c) comprises the steps of:
   c.1) the 3' exonucleolytic cleavage by action of a phi29 DNApol-type enzyme of the 3' end of the target nucleic acid in the direction to the second region of the target nucleic acid, and
   c.2) using as a primer the new 3' end of the target nucleic acid created after c.1),
   wherein a phi29 DNApol-type enzyme comprises or consists of SEQ ID NO: 1, or a sequence with at least 75%, 80%, 85%, 89%, 90%, 91%, 92%, 95%, 97%, 98%, 99%, or 100% identity to the nucleotide sequence as defined in SEQ ID NOs: 1, wherein said sequence comprises at least the amino acid substitutions E415K, E416K, E417K, E218M and V267L,
d) during and/or after step c) amplifying the product of c) with the primase/polymerase protein of *Thermus thermophilus* (*Tth*PrimPol), wherein the *Tth*PrimPol protein comprises or consists of SEQ ID NO: 2, or a sequence with at least 75%, 80%, 85%, 89%, 90%, 91%, 92%, 95%, 97%, 98%, 99%, or 100% identity to the nucleotide sequence as defined in SEQ ID NOs: 2, and the optional addition of one or more specific primer(s) for the RCP complementary strand to boost priming on the secondary branches produced during the MDA step,
e) detecting the amplified product,
wherein the target nucleic acid is a single stranded target nucleic acid, preferably a single-stranded RNA molecule.

In a second aspect, the present invention relates to the use of the method of the present invention for diagnostic and/or prognostic purposes. The second aspect further comprise the use of the method in food industry, to detect the present of infectious agents, preferably infectious agents that cause disease in humans. The applications of the method of the present invention include detecting the presence and/or measuring the level of a target nucleic acid of interest in a suitable sample. In other embodiments, the assay methods described herein can be used in research settings for detecting presence or measuring the level of a target nucleic acid in a sample. The method may be applied in a diagnostic/prognostic setting to detect the presence or measure the level of a nucleic acid biomarker that is associated with a target disease. For example, the methods may be used to detect/measure the presence of an infectious agent, which may be associated with a specific disease, e.g., covid or flu. The methods can be used in detecting such a nucleic acid biomarker in subjects that are absent of any symptom of the disease for early stage diagnosis. The assay methods can also be used to detect nucleic acids of microorganisms for determining whether a subject has been infected by such microorganisms, for example, viruses (e.g., HBV, HCV, HPV, HIV, Influenza, coronavirus). Preferably, the method of the present invention is used for detecting the presence of a virus, preferably a coronavirus, most preferably SARS-CoV2 virus, in a sample.

In a **third aspect**, the present invention relates to a variant of phi29 DNApol as defined in the first aspect of the invention, step c). In a preferred embodiment, the phi29 DNApol variant is an improved chimeric phi29 DNApol that preferably comprises the entire amino acid sequence of phi29 wildtype of SEQ ID NO: 4, but it further comprises a *M. kandleri* Topo V (HhH)2 domain H (residues 696-751) or H and I (residues 696-802) fused to the C-terminus of phi29 DNApol. This chimeric phi29 DNApol variant, also named Qualiphi polymerase, presents enhanced DNA binding and thus is a preferred phi29 DNApol-type enzyme to be used in the method of the present invention. The Qualiphi polymerase is represented by SEQ ID NO: 1.

In a further embodiment, the variant of a phi29 DNApol comprises at least three amino acid substitutions performed over SEQ ID NO: 1, which are E415K, E416K, E417K. In a further embodiment, the variant of a phi29 DNApol comprises at least five amino acid substitutions performed over SEQ ID NO: 1, which are E415K, E416K, E417K, E218M and V267L. It has been shown herein that these variants perform better than Qualiphi wild-type, both in HRCA primer-free methods (Fig. 12), and classic HRCA methods using primers (Fig. 13).

Thus, in an embodiment, the phi29 DNApol-type enzyme of the third aspect comprises or consists of SEQ ID NO: 1, or a sequence with at least 75%, 80%, 85%, 89%, 90%, 91%, 92%, 95%, 97%, 98%, 99%, or 100% identity to the nucleotide sequence as defined in SEQ ID NOs: 1, wherein said phi29 DNApol-type enzyme comprises in its sequence at least the amino acid substitutions E415K, E416K, E417K (Qx3) or at least the amino acid substitutions E415K, E416K, E417K, E218M and V267L (Qx5). In a preferred embodiment, the phi29 DNApol-type enzyme of the third aspect comprises or consists of SEQ ID NO: 1, or a sequence with at least 75%, 80%, 85%, 89%, 90%, 91%, 92%, 95%, 97%, 98%, 99%, or 100% identity to the nucleotide sequence as defined in SEQ ID NOs: 1, wherein said phi29 DNApol-type enzyme comprises in its sequence at least the amino acid substitutions E415K, E416K, E417K (Qx3) or at least at least the amino acid substitutions E415K, E416K, E417K, E218M and V267L (Qx5), and wherein the polymerase maintains the 3'-5' exonuclease, DNA polymerase, and strand-displacement activity of the DNA polymerase represented in SEQ ID NO: 1, 3 or 5. In an embodiment, the phi29 DNApol-type enzyme according to the third aspect comprises or consists of SEQ ID NO: 3 or 5.

In a **fourth aspect**, the present invention relates to the use of a phi29 DNApol-type enzyme as defined in the third aspect, to carry out the method of the first aspect.

In a **fifth aspect**, the present invention also relates to the use of a phi29 DNApol-type enzyme as defined in the third aspect, to carry out an amplification method, preferably *in vitro* amplification method. Non limiting examples for *in vitro* amplification methods include PCR (Polymerase Chain Reaction), LAMP (Loop mediated Isothermal Amplification), RDC (Reaction déplacement chimeric), NASBA or isothermal amplification with phi29 DNApol, which are mainly performed as strand-displacement amplifications like rolling circle amplification (RCA) in the case of covalently closed DNA, or whole genome amplification (WGA) of linear genomic DNA, or HRCA.

In a sixth aspect, the present invention relates to a padlock probe, wherein the padlock probe comprises, consists, or consists essentially of SEQ ID NO: 7-14, or a sequence with at least 85%, 86%, 87%, 88%, 89%, 90%, 91%, 92%, 93%, 94%, 95%, 96%, 97%, 98%, 99% or 100% sequence identity to SEQ ID NO: 7-14. More preferably, the padlock of the present invention comprises, consists, or consists essentially of SEQ ID NO: 8, 10, 12, or 14 or a sequence with at least 85%, 86%, 87%, 88%, 89%, 90%, 91%, 92%, 93%, 94%, 95%, 96%, 97%, 98%, 99% or 100% sequence identity to SEQ ID NO: 8, 10, 12, or 14 wherein the backbone region, indicated with "N", preferably comprises between 20-160, preferably between 40-150 nucleotides that are not complementary to the target nucleic acid. Uses of the padlock probe in the method of the present invention or in any RCA or HRCA methos are also included herein.

In **a seventh aspect**, the present invention relates to a system or device to implement the method of the present invention. Several known systems or devices may be used for said purpose. They may include, but are not limited to, HRCA-based point-of-care (POC) tests for the rapid detection of the at least one target polynucleotide. The POC tests are usually designed as easy-to-use membrane-based test strips, often enclosed by a plastic test cassette. An example of a POC test is a test based on immunochromatographic assays, particularly Lateral flow tests (LFTs). In essence, these the LFTs run the test sample along the surface of a pad with reactive molecules that show a positive or negative result. The result may be displayed visually or upon stimulation with, for instance, a laser. Lateral flow-based biosensors usually comprise a backing card, a sample pad, nitrocellulose membrane, and absorbent pad.

In the context of the present invention "lateral flow system" or "lateral flow assay" is understood as a system comprising a lateral flow device implementing a methodology to detect, in a specific manner, the presence or absence of a target nucleic acid in a given matrix sample. This type of assay eliminates the need for specialized and costly equipment used in conventional laboratories. Lateral flow technology is used as point of care tool (fast and *in situ*)*,* in a large range of applications, from the widely used home pregnancy test to more specialized tests for clinical, food safety, environmental and industrial applications.

In the context of the present invention "a backing card" is understood as the material where all the membranes (sample, conjugate, membrane and wicking pads) are mounted and connected to each other. At the same time, the backing card provides better stability and handling to the lateral flow device.

In the context of the present invention "a sample pad" is understood as the element of the device where the test sample is introduced or deposited and acts as a sponge that holds the sample fluid. Subsequently, the sample fluid is transported to the membrane by capillary action.

In the context of the present invention "a membrane" is understood as the material (e.g. nitrocellulose) that transports the test sample and provides support to the capture molecules (antibodies, aptamers etc.) and allows or enhances their binding capabilities. Two different capture molecules areas are defined on the membrane, test line area which provides the lateral flow assay result (positive or negative) and control line area which probes the correct performance of the lateral flow assay.

In the context of the present invention "a wicking pad" is understood as element in the device that retains all the assay material acting as a waste container.

Further, the method disclosed herein can also be implemented in other devices or systems, such as microfluidic devices, microarrays or electrochemical biosensors. The term "microfluidic chip or device" refers to a set of micro-channels etched or molded into a material (such as glass, silicon, a thermoplastic material or a polymer such as PDMS, for PolyDimethylSiloxane). The micro-channels forming the microfluidic chip are connected together in order to achieve the desired features (mix, pump, sort, control bio-chemical environment). This network of micro-channels trapped into the microfluidic chip is connected to the outside by inputs and outputs pierced through the chip, as an interface between the macro- and micro-world. It is through these holes that the liquids are injected and removed from the microfluidic chip (through tubing, syringe adapters or even simple holes in the chip) with external active systems (pressure controller, push-syringe or peristatic pump) or passive ways (e.g. hydrostatic pressure).

A microfluidic chip or device comprises a support or substrate, wherein said support or substrate comprises at least one channel in the substrate, the channel comprising an inlet, an outlet, and a flow-path connecting the inlet and outlet, wherein the inlet and outlet together define a midplane; and a portion of the flowpath travels transversely across the midplane, wherein the portion of the flowpath that travels transversely across the midplane includes a recognition site or sensing area for detecting, for instance, a target nucleic acid.

In the context of the present invention "DNA microarray chip system" is understood as a system comprising a DNA microarray chip or device that in turn comprises a solid surface onto which DNA molecules, such as the padlocks, have been chemically bonded. Complementary base pairing between the target nucleci acid present in a test sample, preferably an isolated biological sample, and the DNA molecules attached on the chip produces light that is measured. In this particular system, the detection is produced when a hybridization complex comprising the at least one padlock and the target nucleic acid is formed, and is further amplified. Areas on the chip producing light identify the target nucleotide that is present in the test sample. The microarray technology is a platform for the simultaneous analysis of different target sequences in complex fluids, in a high throughput manner. The microarray technology also allows multiplexing (detection of different target polynucleotides at the same time).

In the context of the present invention "electrochemical system" is understood as a system comprising an electrochemical device that provides an analytical signal in the form of an electrical current, which is directly proportional to the concentration of an analyte or, in this case, a target nucleic acid. Thus, the electrochemical system or device may act as biosensor. Detection of the at least one target polynucleotide is achieved due to production of a faradaic current produced due to the binding of the target nucleic acid to the padlock, which triggers the method of the present invention. The appearance of the RCP products produces an electroactive chemical compound. The redox process of the produced electroactive compound taking place on the electrochemical device produces a current upon application of a voltage that is directly proportional to the concentration of the target nucleic acid.

In an eight aspect, the present invention provides a kit comprising the necessary reagents to carry out the method of the present invention. Preferably, the kit also comprises instructions for such method. In an embodiment, the kit comprises:
a) at least one bacteriophage phi29 polymerase, preferably a polymerase defined in the first and/or in the third aspects of the present invention, or any of their embodiment,s
b) at least one ligase, preferably SplintR,
c) the primase/polymerase of *Thermus thermophilus* (*Tth*PrimPol), preferably as defined under the first aspect of the present invention,
d) dNTPs,
e) suitable buffer for the amplification of a target nucleic acid, preferably in isothermal conditions, preferably under alkaline conditions, as explained above.

Preferably, the kit does not comprise any primer and/or any RNase. Preferably, the buffer comprises Tris-HCL, and/or Mg²⁺, and/or Mn²⁺. Preferably, the suitable buffer is an alkaline buffer with a pH of between 7-8 , and comprises:
- 0.1, 0.25, preferably 0.5, 0.75, 1, 1.5, or 2 mM Mn²⁺, preferably MnCl₂,
- 15, 20, preferably 25, 30, or 35 mM Mg²⁺, preferably MgCl₂.

Preferably, the kit comprises the padlock probes defined in the first aspect or any of the embodiments, or in the sixth aspect or any of its embodiments. Preferably, the kit does not comprise any primer, nor RNAse.

### SEQUENCE LISTING

**SEQ ID NO: 1 Qualiphi pol.** 
Met Pro Arg Lys Met Tyr Ser Cys Asp Phe Glu Thr Thr Thr Lys Val
1               5                   10                  15

Glu Asp Cys Arg Val Trp Ala Tyr Gly Tyr Met Asn Ile Glu Asp His
            20                  25                  30

Ser Glu Tyr Lys Ile Gly Asn Ser Leu Asp Glu Phe Met Ala Trp Val
        35                  40                  45

Leu Lys Val Gln Ala Asp Leu Tyr Phe His Asn Leu Lys Phe Asp Gly
    50                  55                  60

Ala Phe Ile Ile Asn Trp Leu Glu Arg Asn Gly Phe Lys Trp Ser Ala
65                  70                  75                  80

Asp Gly Leu Pro Asn Thr Tyr Asn Thr Ile Ile Ser Arg Met Gly Gln
                85                  90                  95

Trp Tyr Met Ile Asp Ile Cys Leu Gly Tyr Lys Gly Lys Arg Lys Ile
            100                 105                 110

His Thr Val Ile Tyr Asp Ser Leu Lys Lys Leu Pro Phe Pro Val Lys
        115                 120                 125

Lys Ile Ala Lys Asp Phe Lys Leu Thr Val Leu Lys Gly Asp Ile Asp
    130                 135                 140

Tyr His Lys Glu Arg Pro Val Gly Tyr Lys Ile Thr Pro Glu Glu Tyr
145                 150                 155                 160

Ala Tyr Ile Lys Asn Asp Ile Gln Ile Ile Ala Glu Ala Leu Leu Ile
                165                 170                 175

Gln Phe Lys Gln Gly Leu Asp Arg Met Thr Ala Gly Ser Asp Ser Leu
            180                 185                 190

Lys Gly Phe Lys Asp Ile Ile Thr Thr Lys Lys Phe Lys Lys Val Phe
        195                 200                 205

Pro Thr Leu Ser Leu Gly Leu Asp Lys Glu Val Arg Tyr Ala Tyr Arg
    210                 215                 220

Gly Gly Phe Thr Trp Leu Asn Asp Arg Phe Lys Glu Lys Glu Ile Gly
225                 230                 235                 240

Glu Gly Met Val Phe Asp Val Asn Ser Leu Tyr Pro Ala Gln Met Tyr
                245                 250                 255

Ser Arg Leu Leu Pro Tyr Gly Glu Pro Ile Val Phe Glu Gly Lys Tyr
            260                 265                 270

Val Trp Asp Glu Asp Tyr Pro Leu His Ile Gln His Ile Arg Cys Glu
        275                 280                 285

Phe Glu Leu Lys Glu Gly Tyr Ile Pro Thr Ile Gln Ile Lys Arg Ser
    290                 295                 300
 
Arg Phe Tyr Lys Gly Asn Glu Tyr Leu Lys Ser Ser Gly Gly Glu Ile
305                 310                 315                 320

Ala Asp Leu Trp Leu Ser Asn Val Asp Leu Glu Leu Met Lys Glu His
                325                 330                 335

Tyr Asp Leu Tyr Asn Val Glu Tyr Ile Ser Gly Leu Lys Phe Lys Ala
            340                 345                 350

Thr Thr Gly Leu Phe Lys Asp Phe Ile Asp Lys Trp Thr Tyr Ile Lys
        355                 360                 365

Thr Thr Ser Glu Gly Ala Ile Lys Gln Leu Ala Lys Leu Met Leu Asn
    370                 375                 380

Ser Leu Tyr Gly Lys Phe Ala Ser Asn Pro Asp Val Thr Gly Lys Val
385                 390                 395                 400

Pro Tyr Leu Lys Glu Asn Gly Ala Leu Gly Phe Arg Leu Gly Glu Glu
                405                 410                 415

Glu Thr Lys Asp Pro Val Tyr Thr Pro Met Gly Val Phe Ile Thr Ala
            420                 425                 430

Trp Ala Arg Tyr Thr Thr Ile Thr Ala Ala Gln Ala Cys Tyr Asp Arg
        435                 440                 445

Ile Ile Tyr Cys Asp Thr Asp Ser Ile His Leu Thr Gly Thr Glu Ile
    450                 455                 460

Pro Asp Val Ile Lys Asp Ile Val Asp Pro Lys Lys Leu Gly Tyr Trp
465                 470                 475                 480

Ala His Glu Ser Thr Phe Lys Arg Ala Lys Tyr Leu Arg Gln Lys Thr
                485                 490                 495

Tyr Ile Gln Asp Ile Tyr Met Lys Glu Val Asp Gly Lys Leu Val Glu
            500                 505                 510

Gly Ser Pro Asp Asp Tyr Thr Asp Ile Lys Phe Ser Val Lys Cys Ala
        515                 520                 525

Gly Met Thr Asp Lys Ile Lys Lys Glu Val Thr Phe Glu Asn Phe Lys
    530                 535                 540

Val Gly Phe Ser Arg Lys Met Lys Pro Lys Pro Val Gln Val Pro Gly
545                 550                 555                 560

Gly Val Val Leu Val Asp Asp Thr Phe Thr Ile Lys Gly Thr Gly Ser
                565                 570                 575

Gly Ala Trp Lys Glu Trp Leu Glu Arg Lys Val Gly Glu Gly Arg Ala
            580                 585                 590

Arg Arg Leu Ile Glu Tyr Phe Gly Ser Ala Gly Glu Val Gly Lys Leu
 
595                 600                 605

Val Glu Asn Ala Glu Val Ser Lys Leu Leu Glu Val Pro Gly Ile Gly
    610                 615                 620

Asp Glu Ala Val Ala Arg Leu Val Pro Gly Tyr Lys Thr Leu Arg Asp
625                 630                 635                 640

Ala Gly Leu Thr Pro Ala Glu Ala Glu Arg Val Leu Lys Arg Tyr Gly
                645                 650                 655

Ser Val Ser Lys Val Gln Glu Gly Ala Thr Pro Asp Glu Leu Arg Glu
            660                 665                 670

Leu Gly Leu Gly Asp Ala Lys Ile Ala Arg Ile Leu Gly
        675                 680                 685
**SEQ ID NO: 2 *Thermus thermophilus* PrimPol** 
Met Arg Pro Ile Glu His Ala Leu Ser Tyr Ala Ala Gln Gly Tyr Gly
1               5                   10                  15

Val Leu Pro Leu Arg Pro Gly Gly Lys Glu Pro Leu Gly Lys Leu Val
            20                  25                  30

Pro His Gly Leu Lys Asn Ala Ser Arg Asp Pro Ala Thr Leu Glu Ala
        35                  40                  45

Trp Trp Arg Ser Cys Pro Arg Cys Gly Val Gly Ile Leu Pro Gly Pro
    50                  55                  60

Glu Val Leu Val Leu Asp Phe Asp Asp Pro Glu Ala Trp Glu Gly Leu
65                  70                  75                  80

Arg Gln Glu His Pro Ala Leu Glu Ala Ala Pro Arg Gln Arg Thr Pro
                85                  90                  95

Lys Gly Gly Arg His Val Phe Leu Arg Leu Pro Glu Gly Val Arg Leu
            100                 105                 110

Ser Ala Ser Val Arg Ala Ile Pro Gly Val Asp Leu Arg Gly Met Gly
        115                 120                 125

Arg Ala Tyr Val Val Ala Ala Pro Thr Arg Leu Lys Asp Gly Arg Thr
    130                 135                 140

Tyr Thr Trp Glu Ala Pro Leu Thr Pro Pro Glu Glu Leu Pro Pro Val
145                 150                 155                 160

Pro Gln Ala Leu Leu Leu Lys Leu Leu Pro Pro Pro Pro Pro Pro Arg
                165                 170                 175

Pro Ser Trp Gly Ala Val Gly Thr Ala Ser Pro Lys Arg Leu Gln Ala
            180                 185                 190

Leu Leu Gln Ala Tyr Ala Ala Gln Val Ala Arg Thr Pro Glu Gly Gln
 
195                 200                 205

Arg His Leu Thr Leu Ile Arg Tyr Ala Val Ala Ala Gly Gly Leu Ile
    210                 215                 220

Pro His Gly Leu Asp Pro Arg Glu Ala Glu Glu Val Leu Val Ala Ala
225                 230                 235                 240

Ala Met Ser Ala Gly Leu Pro Glu Trp Glu Ala Arg Asp Ala Val Arg
                245                 250                 255

Trp Gly Leu Gly Val Gly Ala Ser Arg Pro Leu Val Leu Glu Ser Ser
            260                 265                 270

Ser Lys Pro Pro Glu Pro Arg Thr Tyr Arg Ala Arg Val Tyr Ala Arg
        275                 280                 285

Met Arg Arg Trp Val
    290
**SEQ ID NO: 3 Qualiphi polymerase triple mutant Qx3** (the amino acid substitutions with respect to the parental polymerase Qualify of SEQ ID NO: 1 are highlighted in bold and underlined). 
Met Pro Arg Lys Met Tyr Ser Cys Asp Phe Glu Thr Thr Thr Lys Val
1               5                   10                  15

Glu Asp Cys Arg Val Trp Ala Tyr Gly Tyr Met Asn Ile Glu Asp His
            20                  25                  30

Ser Glu Tyr Lys Ile Gly Asn Ser Leu Asp Glu Phe Met Ala Trp Val
        35                  40                  45

Leu Lys Val Gln Ala Asp Leu Tyr Phe His Asn Leu Lys Phe Asp Gly
    50                  55                  60

Ala Phe Ile Ile Asn Trp Leu Glu Arg Asn Gly Phe Lys Trp Ser Ala
65                  70                  75                  80

Asp Gly Leu Pro Asn Thr Tyr Asn Thr Ile Ile Ser Arg Met Gly Gln
                85                  90                  95

Trp Tyr Met Ile Asp Ile Cys Leu Gly Tyr Lys Gly Lys Arg Lys Ile
            100                 105                 110

His Thr Val Ile Tyr Asp Ser Leu Lys Lys Leu Pro Phe Pro Val Lys
        115                 120                 125

Lys Ile Ala Lys Asp Phe Lys Leu Thr Val Leu Lys Gly Asp Ile Asp
    130                 135                 140

Tyr His Lys Glu Arg Pro Val Gly Tyr Lys Ile Thr Pro Glu Glu Tyr
145                 150                 155                 160
 
Ala Tyr Ile Lys Asn Asp Ile Gln Ile Ile Ala Glu Ala Leu Leu Ile
                165                 170                 175

Gln Phe Lys Gln Gly Leu Asp Arg Met Thr Ala Gly Ser Asp Ser Leu
            180                 185                 190

Lys Gly Phe Lys Asp Ile Ile Thr Thr Lys Lys Phe Lys Lys Val Phe
        195                 200                 205

Pro Thr Leu Ser Leu Gly Leu Asp Lys Glu Val Arg Tyr Ala Tyr Arg
    210                 215                 220

Gly Gly Phe Thr Trp Leu Asn Asp Arg Phe Lys Glu Lys Glu Ile Gly
225                 230                 235                 240

Glu Gly Met Val Phe Asp Val Asn Ser Leu Tyr Pro Ala Gln Met Tyr
                245                 250                 255

Ser Arg Leu Leu Pro Tyr Gly Glu Pro Ile Val Phe Glu Gly Lys Tyr
            260                 265                 270

Val Trp Asp Glu Asp Tyr Pro Leu His Ile Gln His Ile Arg Cys Glu
        275                 280                 285

Phe Glu Leu Lys Glu Gly Tyr Ile Pro Thr Ile Gln Ile Lys Arg Ser
    290                 295                 300

Arg Phe Tyr Lys Gly Asn Glu Tyr Leu Lys Ser Ser Gly Gly Glu Ile
305                 310                 315                 320

Ala Asp Leu Trp Leu Ser Asn Val Asp Leu Glu Leu Met Lys Glu His
                325                 330                 335

Tyr Asp Leu Tyr Asn Val Glu Tyr Ile Ser Gly Leu Lys Phe Lys Ala
            340                 345                 350

Thr Thr Gly Leu Phe Lys Asp Phe Ile Asp Lys Trp Thr Tyr Ile Lys
        355                 360                 365

Thr Thr Ser Glu Gly Ala Ile Lys Gln Leu Ala Lys Leu Met Leu Asn
    370                 375                 380

Ser Leu Tyr Gly Lys Phe Ala Ser Asn Pro Asp Val Thr Gly Lys Val
385                 390                 395                 400

Pro Tyr Leu Lys Glu Asn Gly Ala Leu Gly Phe Arg Leu Gly Lys Lys
                405                 410                 415

Lys Thr Lys Asp Pro Val Tyr Thr Pro Met Gly Val Phe Ile Thr Ala
            420                 425                 430

Trp Ala Arg Tyr Thr Thr Ile Thr Ala Ala Gln Ala Cys Tyr Asp Arg
        435                 440                 445

Ile Ile Tyr Cys Asp Thr Asp Ser Ile His Leu Thr Gly Thr Glu Ile
 
450                 455                 460

Pro Asp Val Ile Lys Asp Ile Val Asp Pro Lys Lys Leu Gly Tyr Trp
465                 470                 475                 480

Ala His Glu Ser Thr Phe Lys Arg Ala Lys Tyr Leu Arg Gln Lys Thr
                485                 490                 495

Tyr Ile Gln Asp Ile Tyr Met Lys Glu Val Asp Gly Lys Leu Val Glu
            500                 505                 510

Gly Ser Pro Asp Asp Tyr Thr Asp Ile Lys Phe Ser Val Lys Cys Ala
        515                 520                 525

Gly Met Thr Asp Lys Ile Lys Lys Glu Val Thr Phe Glu Asn Phe Lys
    530                 535                 540

Val Gly Phe Ser Arg Lys Met Lys Pro Lys Pro Val Gln Val Pro Gly
545                 550                 555                 560

Gly Val Val Leu Val Asp Asp Thr Phe Thr Ile Lys Gly Thr Gly Ser
                565                 570                 575

Gly Ala Trp Lys Glu Trp Leu Glu Arg Lys Val Gly Glu Gly Arg Ala
            580                 585                 590

Arg Arg Leu Ile Glu Tyr Phe Gly Ser Ala Gly Glu Val Gly Lys Leu
        595                 600                 605

Val Glu Asn Ala Glu Val Ser Lys Leu Leu Glu Val Pro Gly Ile Gly
    610                 615                 620

Asp Glu Ala Val Ala Arg Leu Val Pro Gly Tyr Lys Thr Leu Arg Asp
625                 630                 635                 640

Ala Gly Leu Thr Pro Ala Glu Ala Glu Arg Val Leu Lys Arg Tyr Gly
                645                 650                 655

Ser Val Ser Lys Val Gln Glu Gly Ala Thr Pro Asp Glu Leu Arg Glu
            660                 665                 670

Leu Gly Leu Gly Asp Ala Lys Ile Ala Arg Ile Leu Gly
        675                 680                 685
**SEQ ID NO: 4 Phi29 wildt-ype polymerase** 
Met Pro Arg Lys Met Tyr Ser Cys Asp Phe Glu Thr Thr Thr Lys Val
1               5                   10                  15

Glu Asp Cys Arg Val Trp Ala Tyr Gly Tyr Met Asn Ile Glu Asp His
            20                  25                  30

Ser Glu Tyr Lys Ile Gly Asn Ser Leu Asp Glu Phe Met Ala Trp Val
        35                  40                  45

Leu Lys Val Gln Ala Asp Leu Tyr Phe His Asn Leu Lys Phe Asp Gly
 
50                  55                  60

Ala Phe Ile Ile Asn Trp Leu Glu Arg Asn Gly Phe Lys Trp Ser Ala
65                  70                  75                  80

Asp Gly Leu Pro Asn Thr Tyr Asn Thr Ile Ile Ser Arg Met Gly Gln
                85                  90                  95

Trp Tyr Met Ile Asp Ile Cys Leu Gly Tyr Lys Gly Lys Arg Lys Ile
            100                 105                 110

His Thr Val Ile Tyr Asp Ser Leu Lys Lys Leu Pro Phe Pro Val Lys
        115                 120                 125

Lys Ile Ala Lys Asp Phe Lys Leu Thr Val Leu Lys Gly Asp Ile Asp
    130                 135                 140

Tyr His Lys Glu Arg Pro Val Gly Tyr Lys Ile Thr Pro Glu Glu Tyr
145                 150                 155                 160

Ala Tyr Ile Lys Asn Asp Ile Gln Ile Ile Ala Glu Ala Leu Leu Ile
                165                 170                 175

Gln Phe Lys Gln Gly Leu Asp Arg Met Thr Ala Gly Ser Asp Ser Leu
            180                 185                 190

Lys Gly Phe Lys Asp Ile Ile Thr Thr Lys Lys Phe Lys Lys Val Phe
        195                 200                 205

Pro Thr Leu Ser Leu Gly Leu Asp Lys Glu Val Arg Tyr Ala Tyr Arg
    210                 215                 220

Gly Gly Phe Thr Trp Leu Asn Asp Arg Phe Lys Glu Lys Glu Ile Gly
225                 230                 235                 240

Glu Gly Met Val Phe Asp Val Asn Ser Leu Tyr Pro Ala Gln Met Tyr
                245                 250                 255

Ser Arg Leu Leu Pro Tyr Gly Glu Pro Ile Val Phe Glu Gly Lys Tyr
            260                 265                 270

Val Trp Asp Glu Asp Tyr Pro Leu His Ile Gln His Ile Arg Cys Glu
        275                 280                 285

Phe Glu Leu Lys Glu Gly Tyr Ile Pro Thr Ile Gln Ile Lys Arg Ser
    290                 295                 300

Arg Phe Tyr Lys Gly Asn Glu Tyr Leu Lys Ser Ser Gly Gly Glu Ile
305                 310                 315                 320

Ala Asp Leu Trp Leu Ser Asn Val Asp Leu Glu Leu Met Lys Glu His
                325                 330                 335

Tyr Asp Leu Tyr Asn Val Glu Tyr Ile Ser Gly Leu Lys Phe Lys Ala
            340                 345                 350
 
Thr Thr Gly Leu Phe Lys Asp Phe Ile Asp Lys Trp Thr Tyr Ile Lys
        355                 360                 365

Thr Thr Ser Glu Gly Ala Ile Lys Gln Leu Ala Lys Leu Met Leu Asn
    370                 375                 380

Ser Leu Tyr Gly Lys Phe Ala Ser Asn Pro Asp Val Thr Gly Lys Val
385                 390                 395                 400

Pro Tyr Leu Lys Glu Asn Gly Ala Leu Gly Phe Arg Leu Gly Glu Glu
                405                 410                 415

Glu Thr Lys Asp Pro Val Tyr Thr Pro Met Gly Val Phe Ile Thr Ala
            420                 425                 430

Trp Ala Arg Tyr Thr Thr Ile Thr Ala Ala Gln Ala Cys Tyr Asp Arg
        435                 440                 445

Ile Ile Tyr Cys Asp Thr Asp Ser Ile His Leu Thr Gly Thr Glu Ile
    450                 455                 460

Pro Asp Val Ile Lys Asp Ile Val Asp Pro Lys Lys Leu Gly Tyr Trp
465                 470                 475                 480

Ala His Glu Ser Thr Phe Lys Arg Ala Lys Tyr Leu Arg Gln Lys Thr
                485                 490                 495

Tyr Ile Gln Asp Ile Tyr Met Lys Glu Val Asp Gly Lys Leu Val Glu
            500                 505                 510

Gly Ser Pro Asp Asp Tyr Thr Asp Ile Lys Phe Ser Val Lys Cys Ala
        515                 520                 525

Gly Met Thr Asp Lys Ile Lys Lys Glu Val Thr Phe Glu Asn Phe Lys
    530                 535                 540

Val Gly Phe Ser Arg Lys Met Lys Pro Lys Pro Val Gln Val Pro Gly
545                 550                 555                 560

Gly Val Val Leu Val Asp Asp Thr Phe Thr Ile Lys
                565                 570
**SEQ ID NO: 5 Qualiphi polymerase quintuple mutant Qx5** (the amino acid substitutions with respect to the parental polymerase Qualify of SEQ ID NO: 1 are highlighted in bold and underlined). 
Met Pro Arg Lys Met Tyr Ser Cys Asp Phe Glu Thr Thr Thr Lys Val
1               5                   10                  15

Glu Asp Cys Arg Val Trp Ala Tyr Gly Tyr Met Asn Ile Glu Asp His
            20                  25                  30

Ser Glu Tyr Lys Ile Gly Asn Ser Leu Asp Glu Phe Met Ala Trp Val
 
        35                  40                  45

Leu Lys Val Gln Ala Asp Leu Tyr Phe His Asn Leu Lys Phe Asp Gly
    50                  55                  60

Ala Phe Ile Ile Asn Trp Leu Glu Arg Asn Gly Phe Lys Trp Ser Ala
65                  70                  75                  80

Asp Gly Leu Pro Asn Thr Tyr Asn Thr Ile Ile Ser Arg Met Gly Gln
                85                  90                  95

Trp Tyr Met Ile Asp Ile Cys Leu Gly Tyr Lys Gly Lys Arg Lys Ile
            100                 105                 110

His Thr Val Ile Tyr Asp Ser Leu Lys Lys Leu Pro Phe Pro Val Lys
        115                 120                 125

Lys Ile Ala Lys Asp Phe Lys Leu Thr Val Leu Lys Gly Asp Ile Asp
    130                 135                 140

Tyr His Lys Glu Arg Pro Val Gly Tyr Lys Ile Thr Pro Glu Glu Tyr
145                 150                 155                 160

Ala Tyr Ile Lys Asn Asp Ile Gln Ile Ile Ala Glu Ala Leu Leu Ile
                165                 170                 175

Gln Phe Lys Gln Gly Leu Asp Arg Met Thr Ala Gly Ser Asp Ser Leu
            180                 185                 190

Lys Gly Phe Lys Asp Ile Ile Thr Thr Lys Lys Phe Lys Lys Val Phe
        195                 200                 205

Pro Thr Leu Ser Leu Gly Leu Asp Lys Met Val Arg Tyr Ala Tyr Arg
    210                 215                 220

Gly Gly Phe Thr Trp Leu Asn Asp Arg Phe Lys Glu Lys Glu Ile Gly
225                 230                 235                 240

Glu Gly Met Val Phe Asp Val Asn Ser Leu Tyr Pro Ala Gln Met Tyr
                245                 250                 255

Ser Arg Leu Leu Pro Tyr Gly Glu Pro Ile Leu Phe Glu Gly Lys Tyr
            260                 265                 270

Val Trp Asp Glu Asp Tyr Pro Leu His Ile Gln His Ile Arg Cys Glu
        275                 280                 285

Phe Glu Leu Lys Glu Gly Tyr Ile Pro Thr Ile Gln Ile Lys Arg Ser
    290                 295                 300

Arg Phe Tyr Lys Gly Asn Glu Tyr Leu Lys Ser Ser Gly Gly Glu Ile
305                 310                 315                 320

Ala Asp Leu Trp Leu Ser Asn Val Asp Leu Glu Leu Met Lys Glu His
                325                 330                 335
 
Tyr Asp Leu Tyr Asn Val Glu Tyr Ile Ser Gly Leu Lys Phe Lys Ala
            340                 345                 350

Thr Thr Gly Leu Phe Lys Asp Phe Ile Asp Lys Trp Thr Tyr Ile Lys
        355                 360                 365

Thr Thr Ser Glu Gly Ala Ile Lys Gln Leu Ala Lys Leu Met Leu Asn
    370                 375                 380

Ser Leu Tyr Gly Lys Phe Ala Ser Asn Pro Asp Val Thr Gly Lys Val
385                 390                 395                 400

Pro Tyr Leu Lys Glu Asn Gly Ala Leu Gly Phe Arg Leu Gly Lys Lys
                405                 410                 415

Lys Thr Lys Asp Pro Val Tyr Thr Pro Met Gly Val Phe Ile Thr Ala
            420                 425                 430

Trp Ala Arg Tyr Thr Thr Ile Thr Ala Ala Gln Ala Cys Tyr Asp Arg
        435                 440                 445

Ile Ile Tyr Cys Asp Thr Asp Ser Ile His Leu Thr Gly Thr Glu Ile
    450                 455                 460

Pro Asp Val Ile Lys Asp Ile Val Asp Pro Lys Lys Leu Gly Tyr Trp
465                 470                 475                 480

Ala His Glu Ser Thr Phe Lys Arg Ala Lys Tyr Leu Arg Gln Lys Thr
                485                 490                 495

Tyr Ile Gln Asp Ile Tyr Met Lys Glu Val Asp Gly Lys Leu Val Glu
            500                 505                 510

Gly Ser Pro Asp Asp Tyr Thr Asp Ile Lys Phe Ser Val Lys Cys Ala
        515                 520                 525

Gly Met Thr Asp Lys Ile Lys Lys Glu Val Thr Phe Glu Asn Phe Lys
    530                 535                 540

Val Gly Phe Ser Arg Lys Met Lys Pro Lys Pro Val Gln Val Pro Gly
545                 550                 555                 560

Gly Val Val Leu Val Asp Asp Thr Phe Thr Ile Lys Gly Thr Gly Ser
                565                 570                 575

Gly Ala Trp Lys Glu Trp Leu Glu Arg Lys Val Gly Glu Gly Arg Ala
            580                 585                 590

Arg Arg Leu Ile Glu Tyr Phe Gly Ser Ala Gly Glu Val Gly Lys Leu
        595                 600                 605

Val Glu Asn Ala Glu Val Ser Lys Leu Leu Glu Val Pro Gly Ile Gly
    610                 615                 620

Asp Glu Ala Val Ala Arg Leu Val Pro Gly Tyr Lys Thr Leu Arg Asp
625                 630                 635                 640
 
Ala Gly Leu Thr Pro Ala Glu Ala Glu Arg Val Leu Lys Arg Tyr Gly
                645                 650                 655

Ser Val Ser Lys Val Gln Glu Gly Ala Thr Pro Asp Glu Leu Arg Glu
            660                 665                 670

Leu Gly Leu Gly Asp Ala Lys Ile Ala Arg Ile Leu Gly
        675                 680                 685
**SEQ ID NO: 6 SplintR: DNA Ligase from *Paramecium bursaria* chlorella virus-1** 
Met Ala Ile Thr Lys Pro Leu Leu Ala Ala Thr Leu Glu Asn Ile Glu
1               5                   10                  15

Asp Val Gln Phe Pro Cys Leu Ala Thr Pro Lys Ile Asp Gly Ile Arg
            20                  25                  30

Ser Val Lys Gln Thr Gln Met Leu Ser Arg Thr Phe Lys Pro Ile Arg
        35                  40                  45

Asn Ser Val Met Asn Arg Leu Leu Thr Glu Leu Leu Pro Glu Gly Ser
    50                  55                  60

Asp Gly Glu Ile Ser Ile Glu Gly Ala Thr Phe Gln Asp Thr Thr Ser
65                  70                  75                  80

Ala Val Met Thr Gly His Lys Met Tyr Asn Ala Lys Phe Ser Tyr Tyr
                85                  90                  95

Trp Phe Asp Tyr Val Thr Asp Asp Pro Leu Lys Lys Tyr Ile Asp Arg
            100                 105                 110

Val Glu Asp Met Lys Asn Tyr Ile Thr Val His Pro His Ile Leu Glu
        115                 120                 125

His Ala Gln Val Lys Ile Ile Pro Leu Ile Pro Val Glu Ile Asn Asn
    130                 135                 140

Ile Thr Glu Leu Leu Gln Tyr Glu Arg Asp Val Leu Ser Lys Gly Phe
145                 150                 155                 160

Glu Gly Val Met Ile Arg Lys Pro Asp Gly Lys Tyr Lys Phe Gly Arg
                165                 170                 175

Ser Thr Leu Lys Glu Gly Ile Leu Leu Lys Met Lys Gln Phe Lys Asp
            180                 185                 190

Ala Glu Ala Thr Ile Ile Ser Met Thr Ala Leu Phe Lys Asn Thr Asn
        195                 200                 205

Thr Lys Thr Lys Asp Asn Phe Gly Tyr Ser Lys Arg Ser Thr His Lys
    210                 215                 220
 
Ser Gly Lys Val Glu Glu Asp Val Met Gly Ser Ile Glu Val Asp Tyr
225                 230                 235                 240

Asp Gly Val Val Phe Ser Ile Gly Thr Gly Phe Asp Ala Asp Gln Arg
                245                 250                 255

Arg Asp Phe Trp Gln Asn Lys Glu Ser Tyr Ile Gly Lys Met Val Lys
            260                 265                 270

Phe Lys Tyr Phe Glu Met Gly Ser Lys Asp Cys Pro Arg Phe Pro Val
        275                 280                 285

Phe Ile Gly Ile Arg His Glu Glu Asp Arg
    290                 295
**SEQ ID NO: 7 (DNA padlock specific for the human actine gene):** 
5'P-
TGCGGTGGACGATGGAAAAATGTAAAACGACGGCCGAATTCATAGCTGTTTCCTG
AAAAACCGCCTAGAAGCATT-3',
 wherein nucleotides in bold represent the 5' and 3' target-specific hybridization regions of the padlock, underlined nucleotides represent the backbone region of the padlock, and wherein the 5' end of the padlock is phosphorylated (indicated with "P") to facilitate the ligation of the 5' and 3' ends of the hybridized padlock.
**SEQ ID NO: 8 (DNA padlock specific for the human actine gene)**
   5'P-**TGCGGTGGACGATGG**N**CCGCCTAGAAGCATT**-3',
   wherein nucleotides in bold represent the 5' and 3' target-specific hybridization regions of the padlock, wherein "N" represents the nucleotide sequence of the backbone region of the padlock, and wherein the 5' end of the padlock is phosphorylated to facilitate the ligation of the 5' and 3' ends of the hybridized padlock.
**SEQ ID NO: 9 (DNA padlock specific for the SARS-CoV2 gene N)** 
5'P-
TTCTTGAACTGTTGCGAATTCGGTAAAACGACCATAGCTGTTGTAAAACGACCATA
GCTGTTGTAAAACGACCATAGCTGTTGTAAAACGACCTCAGCCGACCATAGCTGT
TGTAAAACGACCATAGCTGTTGTAAAACGACCATAGCTGTTCGATATCTGCCTGGA
GTTG*A*A*T-3'
 wherein nucleotides in bold represent the 5' and 3' target-specific hybridization regions of the padlock, underlined nucleotides represent the backbone region of the padlock, wherein the 5' end of the padlock is phosphorylated (indicated with "P") to facilitate the ligation of the 5' and 3' ends of the hybridized padlock, and wherein asterisks in the sequence are optional and indicate degradation resistant phosphorothioate bonds linking the last four 3' nucleotides.
**SEQ ID NO: 10 (DNA padlock specific for the SARS-CoV2 gene N):**
   **5'P-TTCTTGAACTGTTGC**N**TGCCTGGAGTTGAAT**-3'
   wherein nucleotides in bold represent the 5' and 3' target-specific hybridization regions of the padlock, "N" represents the nucleotide sequence of the backbone region of the padlock, wherein the 5' end of the padlock is phosphorylated (indicated with "P") to facilitate the ligation of the 5' and 3' ends of the hybridized padlock, and wherein, preferably, the last four nucleotides in the 3' end of the padlock are bound by phosphorothioate bonds.
**SEQ ID NO: 11 (DNA padlock specific for the 3' end of SARS-CoV2 RNA)** 
5'P-
AAGAAGCTATTAAAATCACCGCAACTGAACTACTTGTCGCCTCAGCGCATCTCCT
GATGAGGTTCCACCTGCCTCAGCGTCATTCTCCT-3'
 wherein nucleotides in bold represent the 5' and 3' target-specific hybridization regions of the padlock, wherein the 5' end of the padlock is phosphorylated (indicated with "P") to facilitate the ligation of the 5' and 3' ends of the hybridized padlock.
**SEQ ID NO: 12 (DNA padlock specific for the 3' end of SARS-CoV2 RNA)**
   **5'P-AAGAAGCTATTAAAATCACNGTCATTCTCCT-3'**
   wherein nucleotides in bold represent the 5' and 3' target-specific hybridization regions of the padlock, "N" represents the nucleotide sequence of the backbone region of the padlock probe, and wherein the 5' end of the padlock is phosphorylated (indicated with "P") to facilitate the ligation of the 5' and 3' ends of the hybridized padlock.
**SEQ ID NO: 13 (optimized DNA padlock, specific for the 3' end of SARS-CoV2 RNA)** 
5'P-
AAGAAGCTATTAAAATCACCGCAACTGAGGTACTTGTCGCCACAGAGCATCACCT
GATGAGGTTCCACCTGCCACAGAGTCATTCTCCT-3'
 wherein nucleotides in bold represent the 5' and 3' target-specific hybridization regions of the padlock, underlined nucleotides represent the backbone region of the padlock, and wherein the 5' end of the padlock is phosphorylated (indicated with "P") to facilitate the ligation of the 5' and 3' ends of the hybridized padlock.
**SEQ ID NO: 14 (optimized DNA padlock, specific for the 3' end of SARS-CoV2 RNA)**
   **5'P-AAGAAGCTATTAAAATCACNGTCATTCTCCT-3'**
   wherein nucleotides in bold represent the 5' and 3' target-specific hybridization regions of the padlock, "N" represents the nucleotide sequence of the backbone region of the padlock probe, and wherein the 5' end of the padlock is phosphorylated (indicated with "P") to facilitate the ligation of the 5' and 3' ends of the hybridized padlock.

The invention is described below by the following examples, which must be considered as merely illustrative and in no case limiting of the scope of the present invention.

### EXAMPLES

### Summary

The present invention relates to a method of primer-less isothermal amplification for detecting a target ribonucleic acid. In this method (a cartoon is shown in Figure 14), a specific DNA padlock probe, complementary to the 3' terminal region of a target ribonucleic acid, is circularized and amplified by rolling circle amplification (RCA), where the 3'-end of the target ribonucleic acid can act as an efficient primer by an optimized phi29 DNApol variant. The padlock-directed concatemeric ssDNA produced by RCA is further amplified by the combination of a modified phi29 DNApol (Qx3, Qx5,...) and a specific DNA primase (*Tth*PrimPol) which continuously synthesizes suitable DNA primers on the RCA product to trigger a hyperbranched rolling circle amplification (HRCA), that can be directly analyzed by any detection method. Variants of phi29 DNApol and their use in this and other amplification methods are also provided herein.

### 1. Starting alkaline conditions favour amplification detection by Qualiphi

Phi29 DNApol is one of the most widely used enzymes, mainly due to the extensive knowledge about its DNA synthetic activities, and its exceptional processivity, fidelity and strand-displacement ability (Blanco et. al., JBC, 1989; Esteban et al., JBC, 1993). Since its initial characterization (Blanco and Salas, PNAS 1984), neutral pH conditions have been systematically used for all applications of this enzyme, but the impact of alkaline pH conditions has not been explored in any detail to date.

As shown in Figure 1, the amplification capacity of Qualiphi increases at alkaline conditions (pH 8.8) on both a circular M13 ssDNA template (Figure 1A), and on a SARS CoV2-specific padlock probe (Figure 1B). As it can be observed when the reaction is started at pH7.5, and likely as a consequence of the acidification resulting from DNA synthesis (Tanner et al., 2015, BioTechniques), there is a significant reduction in the amplification capacity of the enzyme when the Tris-HCl concentration is reduced from 50 mM to 2 mM (lanes 4-6). Conversely, when the reaction was buffered with decreasing concentrations of the starting buffer Tris-HCl pH 8.8, DNA synthesis-induced acidification of the reaction did not reduce significantly the amount of DNA amplified by the enzyme, tolerating a very low (2 mM) concentration of buffer, but being 12 mM the optimal Tris-HCl pH 8.8 concentration. Under these optimal conditions, the amplification of a very low concentration (60 fM) of the input padlock probe could be detected by both SYBRGreen or by a pH-dependent colour change using Neutral Red (Figure 1C).

### 2. Thermo-stabilized versions of Qualiphi perform better in RNA target-driven padlock amplification

It has been reported that both, the efficiency and specificity of the ligation reaction by SplintR ligase is optimal at 37 °C (Jin et al., 2016, NAR). Therefore, a 1-step protocol, coupling padlock ligation to subsequent DNA amplification in the presence of saliva samples could be performed at 37 °C to minimize spurious (target-independent) amplification. However, our previous unpublished data showed that the chimeric Qualiphi version of Phi29 DNApol behaves very inefficiently at such a temperature. Therefore, we introduced in Qualiphi the changes previously reported to increase the thermostability of the wild-type Phi29 DNApol (patent WO 2017/109262A1), obtaining the Qx3 and Qx5 variants (see sequences in appendix "DNA polymerase sequences"). To test whether the changes introduced had the same thermostability effect in Qualiphi as in the wild-type enzyme, we tested the amplification efficiency of the variants in the 30 °C-39 °C temperature range. As shown in Figure 2, whereas the optimal reaction temperature for the chimeric Qualiphi version (Qphi; de Vega et al., PNAS 2010) of Phi29 DNApol was 32-34 °C (see lower panel/2 h in Figure 2), the amplification capacity of Qx3 and Qx5 remained constant over the range 30-37 °C and 30-39 °C, respectively, the latest showing the highest thermo-resistance.

On top of that, and even at the lowest reaction temperature assayed (30 °C), the thermo-stabilized versions Qx3 and Qx5 yielded a much higher amount of amplification product than the wild-type Qualiphi enzyme, resulting in large amplification of the padlock even at 1 h of reaction (upper panel).

### 3. The thermo-stabilized variant Qx5 performs better on DNA template/RNA primer hybrids

The superior activity of the thermo-stabilized variants of Qualiphi (Qx3 and Qx5 variants) in the RNA-targeted amplification of the circularized padlock could be due to a more efficient recognition of the DNA/RNA junction (produced after RNAse H nicking) required to start the initial RCA process. To explore that possibility, we analyzed the dynamic equilibrium between the 3'-5' exonuclease and 5'-3' polymerization activities of Qualiphi and its thermo-stabilized variant Qx5, by comparing their performance on a DNA template/DNA primer (left panel), *versus* a DNA template/RNA primer (right), as a function of dNTPs concentration. In the absence of dNTPs, the degradation pattern of the primer strand would fully reflect the 3'-5' exonuclease activity of each enzyme; as the dNTPs concentration increases, the exonuclease activity should be progressively competed out by DNA synthesis (polymerization activity) resulting in net primer extension once the dynamic equilibrium is displaced to polymerization.

As shown in Figure 3, both Qualiphi and its thermo-stabilized variant Qx5 behave the same on a DNA template/DNA primer substrate (left panel), showing a similar exonuclease activity in the absence of nucleotides, and requiring 0.1 µM dNTPs to get an efficient elongation of the primer. Unexpectedly, on the DNA template/RNA primer substrate (mimicking that generated during RNA-primed amplification of the DNA padlock), Qx5 showed a much higher exoribonucleolytic and polymerization activities than Qualiphi, being able to get an efficient synthesis at a dNTPs concentration lower than Qualiphi (0.1 µM and 1 µM, respectively), despite of its higher exonuclease activity. These results suggest that Qx5 has a better recognition of the DNA/RNA substrate, that would be beneficial as a starting point to trigger RNA(target)-primed RCA.

### 4. The thermo-stabilized variant Qx5 can efficiently start RNA-primed DNA synthesis after exonucleolysis of a 3'-terminal RNA protrusion

As shown before, Qx5 has a competent recognition of a DNA template/RNA primer substrate, that would be beneficial as a starting point to trigger RNA(target)-primed RCA after RNAse H digestion. However, to evaluate if the positioning of Qx5 adjacent to the padlock could be facilitated via recognition and previous exonucleolysis of a 3'-terminal single-stranded protrusion in the target RNA (i.e. the poly A tail present at the 3' end of SARS-CoV2 RNA), two additional template/primer substrates were generated.

As depicted in Figure 4, a template ssDNA oligonucleotide (5'TCCACCTGCCACAGA**GTCATTCTCCTAAGAAGCTATTAAAATCAC** 3') was hybridized (nucleotides in bold) to a ssRNA oligo corresponding to the SARS-CoV2 viral sequence proximal to the 3'-end polyA tail (*5*'-*GUGAUUUUAAUAGCUUCUUAGGAGAAUGAC;* left panel); or to an RNA oligo containing the same viral sequence and a portion (7 nt) of the polyA tail (*5*'-*GUGAUUUUAAUAGCUUCUUAGGAGAAUGACAAAAAAA*; right panel).

As shown in Figure 4, the exonuclease activity exhibited by Qx5 on both substrates was much more efficient than that by Qualiphi. Importantly, the more efficient removal of the 3'-end polyA tail (right panel) by Qx5 allows a much more proficient coupling of the exonuclease and polymerization activities, allowing the enzyme to get a net elongation of the 30mer RNA that requires the exhaustive exonucleolytic removal of the polyA tail. Therefore, these results will support the convenient use of Qx5 with padlocks probes designed to hybridize close to the 3'-end of the viral RNA, thus making unnecessary the use of RNAse H.

### 5. Experimental conditions required to optimize the RCA step when using a 3'-terminal padlock hybridized to its viral RNA target, in the presence of saliva

The above results support the convenience of hybridizing a padlock probe to the 3'-terminal viral sequence immediately preceding the polyA tail, that would make unnecessary the use of RNAse H. Thus, the resected RNA resulting from exonucleoytic trimming of the polyA tail by Qx5 would provide a valid and convenient primer to drive the RCA reaction. To test this hypothesis, a 3'-terminal padlock (padlock 3'end) was designed and tested for RCA in the presence of a synthetic single-stranded RNA target corresponding to the 3' end of SARS-CoV2 RNA. This RCA assay was performed with Qx5, in the presence of saliva, and in the absence of additional primers that could start the amplification reaction. Figure 5 summarizes the optimized conditions regarding temperature and pH, and the strict need of the SplintR ligase to circularize the padlock probe. Qx5 amplified efficiently and specifically only the ligated (circularized) probe, indicating that the RCA reaction can be primed by the RNA target once its polyA tail has been exonucleolytically trimmed. This primer-less RCA step, yields a 10⁴ amplification factor. Both, specificity and efficiency of the reaction was dependent on: 1) presence of target RNA (its absence results in small amounts of non-specific amplification products); 2) Specific (target-assisted) ligation of the padlock ends by SplintR at 37 °C (its absence only produces target-independent minimal amounts of amplified DNA); 3) Amplification at 37 °C (decreasing the reaction temperature to 30 °C reduces 4-fold the yield of amplified DNA; 4) alkaline pH 8.8 (decreasing the reaction pH to 7.5 reduces more than 8-fold the amplified DNA).

### 6. Addition of TthPrimPol (after the RCA step) largely improves padlock amplification when testing synthetic SARS-CoV2 RNA

To improve the sensitivity one step further, the primer-less RCA step was coupled to a second isothermal amplification step which also does not require adding primers (see scheme in Figure 6). This second amplification step is triggered by *Thermus thermophilus* PrimPol (*Tth*PrimPol; Picher et al. 2016, Nat. Comm.), a unique DNA primase that needs a ssDNA template, but it does not work on ssRNA. Thus, in targetpositive samples, once the padlock is circularized and the RCA reaction starts to produce the ssDNA concatemer, multiple DNA primers can be now generated by *Tth*PrimPol and further elongated by Qx5, resulting in an exponential amplification (MDA) of the whole padlock sequence. The experiment shown in Figure 6 tests the efficiency and specificity of padlock amplification in the presence of saliva, either in the absence or presence of the specific viral RNA target; after ligation of the 3'-end padlock probe, and further RCA with Qx5, the additional incubation step in the presence of 25 or 300 nM of *Tth*PrimPol increased the ssDNA amplification product 3-fold and 10-fold, respectively, improving the sensitivity of the detection procedure. However, addition of *Tth*PrimPol to the control (off-target) samples also increases background amplification.

### 7. The use of 25 mM Mg²⁺ concentration significantly reduces the background when using TthPrimPol for padlock amplification.

In an attempt to improve the specificity of the amplification reaction when using the additional amplification step with *Tth*PrimPol, we explored the effect of increasing the concentration of Mg²⁺ ions on the amplification outcome. For that, similar 2-steps padlock amplification experiments were carried out, hybridizing the padlock 3' to a synthetic single-stranded RNA target containing the 3'-terminal SARS-CoV2 sequence, in the presence of increasing concentrations of MgCl₂. As shown in Figure 7, whereas the amount of amplified DNA in the presence of target remained constant in the range of the Mg²⁺ concentrations assayed (5, 10 and 25 mM), the quantity of non-specific (off-target) amplification product gradually decreased when using increasing MgCl₂ concentrations, being undetectable at 25 mM. In the presence of higher Mg²⁺ concentrations (50 mM), amplification products were detected neither in both targetpositive nor in target-negative samples.

Therefore, using 25 mM MgCl₂ in combination with *Tth*PrimPol extremely improved both, the sensitivity and the specificity of the detection procedure.

### 8. The TthPrimPol-assisted padlock amplification method is valid to detect the viral RNA in SARS-CoV2 infected patients

To determine whether the use of *Tth*PrimPol also had a positive effect on the detection of the virus in patient samples, a similar experiment was performed with the same padlock probe (3'-end) that hybridizes specifically in the region immediately preceding the 3'-terminal polyA of the viral SARS-CoV2 RNA. After hybridization of the padlock 3'-end with total RNA extracted from human nasopharyngeal swabs of positive and negative patients, the 3'-terminal polyA flap has to be exonucleolytically removed to allow subsequent amplification of the padlock probe by RCA, as described above.

As it can be observed in Figure 8, although the RCA reaction with Qx5 discriminated between the positive and negative samples, the efficiency of the reaction was very low, yielding a tiny amount of amplified DNA. Conversely, the additional 1-hour incubation of the RCA products with *Tth*PrimPol increased more than 40-fold the sensitivity of the procedure. However, under those conditions not only the positive, but also the negative control (not containing viral RNA) gave rise to non-specific amplification products.

### 9. 25 mM MgCl₂ improves the specificity of the TthPrimPol-assisted padlock amplification method, also to detect SARS-CoV2 infected patients

To determine whether the use of high MgCl₂ concentrations also had a positive effect on the specificity of detection of the virus in patient samples, a similar experiment was performed with the same padlock probe (3'-end) that hybridizes specifically in the region of the viral RNA immediately preceding the 3'-terminal polyA of the viral RNA. After hybridization of the padlock 3'-end with total RNA extracted from human nasopharyngeal swabs (combined in two separated pools of positive and negative patients), *Tth*PrimPol-assisted padlock amplification was performed at the conventional (5 mM) or at an elevated (25 mM) MgCl₂ concentration, the latter previously shown (Fig. 7) to reduce the background (off-target) amplification when using synthetic single-stranded RNA targets.

As shown in Figure 9, the use of 25 mM MgCl₂ reduced the background (off-target) amplification in the pool of negative samples (both in the presence or absence of saliva, and after incubation for either 30 min or 1 h with *Tth*PrimPol), when compared to the use of 5 mM MgCl₂. Conversely, rising the MgCl₂ concentration from 5 mM to 25 mM increased the specific amplification obtained with the pool of positive samples, both in the presence/absence of saliva.

Altogether, these results indicate that using 25 mM MgCl₂ in combination with *Tth*PrimPol extremely improved both, the sensitivity and the specificity of detection of the viral RNA extracted from patient samples, and even in the presence of saliva.

### 10. Improvement of the padlock probe sequence for optimal TthPrimPol priming increases both the sensitivity and specificity of the detection procedure.

As shown in Figure 10a, inspection of the original 3'-end padlock sequence used in the above experiments allowed us to identify the presence of several preferential priming sites for *Tth*PrimPol (3'CTCC; 3'CTC), that could generate a non-specific (off-target) amplification of the linear (non-ligated) padlock, perhaps explaining the background observed in previous assays, especially when using 5 mM MgCl₂, or when *Tth*PrimPol was already present at the beginning of the RCA reaction (1 step amplification). Therefore, to decrease the off-target amplification of the linear padlock, but to improve *Tth*PrimPol priming on the target-specific RCA product, the sequence of the original 3'-end padlock was optimized to: 1) eliminate the potential priming sites for *Tth*PrimPol on the linear padlock; 2) increase the number of optimal *Tth*PrimPol priming sites on the ssDNA product generated by RCA (see Figure 10a).

To test our hypothesis, both the original and optimized padlocks were comparatively used in the *Tth*PrimPol-assisted detection of a synthetic single-stranded RNA target containing the sequence of the 3'-end of the SARS-CoV2 RNA, and even using 25 mM MgCl₂ to improve the specificity (as previously shown in Figs 7 and 9). As indicated in Figure 10b (lower panel), two amplification protocols were set up: first, the conventional 2-step amplification protocol, where the ligation products are subjected first to RCA with Qx5 for 1 h, and the resulting ssDNA concatemer is further amplified with *Tth*PrimPol for 1 h (part B, left panels); a new 1-step amplification protocol, where the ligated products were amplified during 2 h in the presence of both, Qx5 and *Tth*PrimPol (part B, right panels). As shown in Figure 10b (lower panel), under both experimental conditions, the amount of target-dependent amplification obtained with the optimized padlock was similar, and higher than that produced with the non-optimized padlock. Importantly, in the 2 step protocol, the off-target amplification background remained quite low with both padlocks likely due to the presence of 25 mM MgCl₂. However, the presence of 25 mM MgCl₂ could not avoid the background when the reaction occurs in a single step with the original padlock, suggesting that the early presence of *Tth*PrimPol before the RCA product is accumulated, facilitates the spurious priming and amplification of the linear padlock. Strikingly, the optimized padlock did not produce a significant background when using the single step protocol, which is very convenient to simplify the procedure. Altogether, these results indicate that using 25 mM MgCl₂ in combination with *Tth*PrimPol and the optimized padlock probe extremely improved both, the sensitivity and the specificity of the detection procedure.

### 11. Mn²⁺ ions used to boost TthPrimPol synthesis of primers also improve RCA by Qx5

The priming activity of several PrimPols, as human PrimPol shows a strong dependence on Mn²⁺ ions to activate its priming function (García-Gómez et al., 2013, Mol Cell). Although *Tth*PrimPol can use Mg²⁺ as metal activator, the priming activity with Mn²⁺ is higher (Picher et al., 2016, Nat. Comm.). To explore the effect of Mn²⁺ ions on the *Tth*PrimPol-assisted amplification of the 3' end padlock in the presence of a synthetic single-stranded RNA target containing the sequence of the 3'-end of the SARS-CoV2 RNA, we compared two experimental conditions: one in which the target-driven ligation products were amplified by RCA only with Qx5 during 1 h, *versus* the 2-steps amplification protocol (RCA + *Tth*PrimPol-driven HRCA) described above. As shown in Figure 11a, under both experimental conditions, the extra addition of 0.5 mM MnCl₂, on the one hand allowed for a 25% increase in the yield of the amplification product, increasing the sensitivity of the detection, and on the other hand reduced the off-target amplification, increasing the specificity of the detection method. The higher production of amplified DNA observed during the RCA reaction indicates the stimulation of the amplification capacity of Qx5 by Mn²⁺ ions.

To ascertain whether such an implementation could be used to detect the SARS-CoV2 RNA, similar assays to those described before were carried out, in the presence of a synthetic RNA target corresponding to the viral 3'-terminal sequence, or SARS-CoV2 RNA extracted from infected cells. As shown in Figure 11b, the sequential use of an RCA step by Qx5, followed by a *Tth*PrimPol-assisted HRCA step, using 25 mM MgCl₂, 0.5 mM MnCl₂, pH 8.8 and 37 °C allowed the sensitive and specific detection of a low concentrations (5 pM) of the synthetic RNA oligo, and SARS-CoV2 RNA obtained from infected cells (provided by Antonio Alcami, CBMSO, Madrid).

### 12. Phi29 DNA polymerase variant Qx5 shows an unexpected improved ability to use also DNA primers.

Having determined the improved ability to degrade the 3' end of an RNA partially hybridized to DNA, that makes Qx5 the enzyme of choice for carrying out the RNA-primed RCA of the padlock probe, we wanted to ascertain whether the mutations introduced in Qualiphi (Qx5) also provided an advantage to extend the PrimPolgenerated DNA primers, when amplifying the ssDNA generated during the RCA. To mimic this second step, we set up an MDA assay using as template the circular ssDNA of bacteriophage M13 and providing convenient primers at multiple sites along the molecule, that will be further elongated by the polymerase. Two types of DNA primers were considered: 1) synthetic random DNA hexamers that will hybridize at multiple sites of the M13 ssDNA; 2) DNA primers generated by *Tth*PrimPol preferentially at the 3'-CTCC and 3'-CTC sequences present along the circular M13 ssDNA molecule. In both cases, the DNA primers should be further elongated by the Phi29 DNA polymerase to give rise to an exponential amplification of the template DNA. As it can be observed in Figure 12 (Phi29 DNApol variant + *Tth*PrimPol) and Figure 13 (Phi29 DNA pol variant + random hexamers), when the reaction temperature was 37 °C, the variant Qx5 was clearly better (high DNA yield even at short reaction times) than both the wild-type enzyme and Qualiphi. Therefore, the mutations introduced make Qx5 a more robust enzyme to elongate both, an RNA primer (as needed *ab initio* of the first RCA step of the method) and DNA primers (produced during the second PrimPolmediated MDA step of the method).

Unexpectedly, the superior amplification by Qx5 was maintained when the reaction temperature was 30 °C (see Figures 12 and 13), supporting the benefit of using Qx5 in other DNA amplification methods performed at low DNA temperature.

## Claims

1. A method for detecting the presence of a single-stranded target nucleic acid by hyperbranched rolling circle amplification (HRCA), the method comprising the steps of:
a) contacting the target nucleic acid with at least one padlock probe, wherein the at least one padlock probe comprises a 5' target-specific hybridization region that is complementary to a first region of the target nucleic acid, and a 3' target-specific hybridization region that is complementary to a second region of the target nucleic acid, wherein the first and the second regions are located adjacent to each other and located in the 3' region of the target nucleic acid,
b) circularising the padlock probe whose 5' and 3' end sequences have hybridized to the target nucleic acid by ligating the ends of said padlock probe,
c) subjecting the circularized padlock probe to rolling circle amplification (RCA), wherein the RCA is carried out by a bacteriophage phi29 DNA polymerase (phi29 DNApol-type enzyme), wherein the 3' end of the target nucleic acid is used as a primer for the RCA,
d) during and/or after step c) amplifying the product of c) using the DNA primers made by the primase/polymerase of *Thermus thermophilus* (*Tth*PrimPol), and
e) detecting the amplified product.

2. The method according to claim 1 wherein the phi29 DNApol-type enzyme comprises an amino acid sequence that is at least 70% identical to SEQ ID NO: 1, wherein the DNA polymerase comprises 3'-5' exonuclease and processive strand-displacement DNA synthesis.

3. The method according to claim 2, wherein the phi29 DNApol-type enzyme comprises at least the amino acid substitutions E415X, E416X and E417X, as defined using the amino acid numbering of SEQ ID NO: 1, wherein X is a positively charged amino acid, preferably lysine (K).

4. The method according to claim 3, wherein the phi29 DNApol-type further comprises the amino acid substitutions E218M and V267L, as defined using the amino acid numbering of SEQ ID NO: 1.

5. The method according to any one of claims 1 to 4, wherein the primase/polymerase of *Thermus thermophilus* (*Tth*PrimPol) is at least 70% identical to SEQ ID NO: 2.

6. The method according to claim 5, wherein the thermostable primase/polymerase of *Thermus thermophilus* (*Tth*PrimPol) is at least 90% identical to SEQ ID NO: 2, and wherein the at least one padlock probe is **characterized by**:
i. not having in its nucleotide sequence the sequences "5'-CCTC-3"' and/or "5'-CTC-3"', and/or
ii. having in its nucleotide sequence one or more sequences "5'-GAGG-3"' and/or "5'-GAG-3"'.

7. The method according to claim 6, wherein a specific DNA primer for the stranddisplaced complementary copy of the RCP is added.

8. The method according to any one of the previous claims, wherein the padlock comprises SEQ ID NO: 7-14, or a sequence with at least 90% sequence identity to SEQ ID Nos: 7-14.

9. The method according to any one of the previous claims, wherein the single-stranded target is a ribonucleic acid.

10. The method according to claim 9, wherein the ribonucleic acid comprises a polyA tail, and wherein the first and second regions of the target ribonucleic acid are not located in said polyA tail.

11. The method according to any one of claims 1 to 10, wherein the method is performed at alkaline pH, preferably at pH ranging from 8.5-9.

12. The method according to any one of claims 1 to 11, wherein this isothermal method is performed in the 30-37°C range.

13. A padlock probe comprising SEQ ID NO: 7-14, or a sequence with at least 90% sequence identity to SEQ ID NOs: 7-14.

14. A bacteriophage phi29 DNA polymerase comprising an amino acid sequence that is at least 90% identical to SEQ ID NO: 1, wherein the bacteriophage phi29 DNA polymerase comprises at least the amino acid substitutions E415K, E416K and E417K, as defined using the amino acid numbering of SEQ ID NO: 1.

15. The bacteriophage phi29 DNA polymerase according to claim 18, wherein the bacteriophage phi29 DNA polymerase further comprises the amino acid substitutions E218M and V267L, as defined using the amino acid numbering of SEQ ID NO: 1.
